# EUROPEAN PATENT SPECIFICATION

(11) **EP 3 893 902 B1**
(45) Date of publication and mention of the grant of the patent: **23.10.2024**
(21) Application number: 19895401.8
(22) Date of filing: 10.12.2019
(51) Int. Cl.: A61K 35/17, A61K 38/00, A61K 38/08, A61K 38/10, A61P 35/00

(54) **INHIBITORS OF PD-L1 ACTIVITY OR EXPRESSION FOR USE IN THE TREATMENT OF SERRATED COLORECTAL CANCER**
INHIBITOREN DER PD-L1-AKTIVITÄT ODER -EXPRESSION ZUR VERWENDUNG IN DER BEHANDLUNG VON SERRATIERTEM KOLOREKTALEM KREBS
INHIBITEURS DE L'ACTIVITÉ OU DE L'EXPRESSION DE PD-L1 POUR L'UTILISATION DANS LE TRAITEMENT DU CANCER COLORECTAL D'ASPECT CRÉNELÉ

(30) Priority: 11.12.2018 US 201862778124 P
(43) Date of publication of application: 20.10.2021
(73) Proprietor: Sanford Burnham Prebys Medical Discovery Institute, La Jolla, CA 92037 (US)
(72) Inventor: MOSCAT, Jorge, La Jolla, California 92037 (US); DIAZ-MECO, Maria T., La Jolla, California 92037 (US); DURAN, Angeles, La Jolla, California 92037 (US); NAKANISHI, Yuki, La Jolla, California 92037 (US)
(74) Representative: Mewburn Ellis LLP
(86) International application number: PCT/US2019/065529
(87) International publication number: WO 2020/123543

(56) References cited:
- WO-A1-2014/062845
- WO-A1-2016/033555
- WO-A1-2017/127282
- WO-A1-2017/210119
- US-A1- 2001 044 113
- US-A1- 2009 170 133
- US-A1- 2016 289 768
- US-A1- 2017 051 360
- US-A9- 2018 044 419
- DUNG T. LE ET AL: "PD-1 Blockade in Tumors with Mismatch-Repair Deficiency", THE NEW ENGLAND JOURNAL OF MEDICINE, vol. 372, no. 26, 25 June 2015 (2015-06-25), US, pages 2509 - 2520, XP055390373, ISSN: 0028-4793, DOI: 10.1056/NEJMoa1500596
- HAILONG ZHU ET AL: "Original Article Clinical significance of programmed death ligand-1 (PD-L1) in colorectal serrated adenocarcinoma", INT J CLIN EXP PATHOL, 1 January 2015 (2015-01-01), XP055725866, Retrieved from the Internet <URL:https://www.ncbi.nlm.nih.gov/pmc/articles/PMC4583920/pdf/ijcep0008-9351.pdf>
- FESSLER EVELYN ET AL: "TGF[beta] signaling directs serrated adenomas to the mesenchymal colorectal cancer subtype", EMBO MOLECULAR MEDICINE, vol. 8, no. 7, 24 July 2016 (2016-07-24), US, pages 745 - 760, XP055961510, ISSN: 1757-4676, DOI: 10.15252/emmm.201606184
- NAKANISHI YUKI ET AL: "Control of Paneth Cell Fate, Intestinal Inflammation, and Tumorigenesis by PKC[lambda]/[iota]", CELL REPORTS, vol. 16, no. 12, 1 September 2016 (2016-09-01), US, pages 3297 - 3310, XP055961471, ISSN: 2211-1247, Retrieved from the Internet <URL:https://www.ncbi.nlm.nih.gov/pmc/articles/PMC5043519/pdf/nihms812439.pdf> DOI: 10.1016/j.celrep.2016.08.054
- NAKANISHI ET AL.: "Simultaneous Loss of Both Atypical Protein Kinase C Genes in the Intestinal Epithelium Drives Serrated Intestinal Cancer by Impairing Immunosurveillance", IMMUNITY, vol. 49, no. 6, 11 December 2018 (2018-12-11), pages 1132 - 1147, XP055725825
- LI ET AL.: "Identification of Gene Expression Changes from Colitis to CRC in the Mouse CAC Model", PLOS ONE, vol. 9, no. 5, 17 April 2014 (2014-04-17), pages 1 - 10, XP055725828
- LLADO ET AL.: "Repression of intestinal stem cell function and tumorigenesis through direct phosphorylation of beta-catenin and Yap by PKCzeta", CELL REPORTS, vol. 10, no. 5, 5 February 2015 (2015-02-05), pages 740 - 754, XP055725861
- YEO ET AL.: "Phosphorylated Protein Kinase C (Zeta/Lambda) Expression in Colorectal Adenocarcinoma and Its Correlation with Clinicopathologic Characteristics and Prognosis", JOURNAL OF CANCER, vol. 8, no. 16, 20 September 2017 (2017-09-20), pages 3371 - 3377, XP055725863
- YOSHIHAMA ET AL.: "High expression of KIBRA in low atypical protein kinase C-expressing gastric cancer correlates with lymphatic invasion and poor prognosis", CANCER SCIENCE, vol. 104, no. 2, 19 November 2012 (2012-11-19), pages 259 - 265, XP055725864
- ZHU ET AL.: "Clinical significance of programmed death ligand-1 (PD-L1) in colorectal serrated adenocarcinoma", INT. J. CLIN. EXP. PATHOL., vol. 8, no. 8, 1 August 2015 (2015-08-01), pages 9351 - 9359, XP055725866

## Description

### BACKGROUND

The serrated adenocarcinoma subtype of colorectal cancers (CRCs) accounts for 15-30% of all CRCs and is aggressive and treatment-resistant. Serrated CRC develops through precursor lesions most commonly found in the proximal colon. Serrated CRC is customarily diagnosed endoscopically by detection of precursor lesions. However, precursor lesions are difficult to identify in a patient due to limitations in technology or the endoscopist. In a non-limiting example, flat lesions in the right colon covered by mucus caps are particularly difficult to identify and are reported in more than half of serrated polyps or adenomas. Lastly, high-risk serrated adenomas are commonly misclassified as low risk hyperplastic polyps. Thus, there is a need for more accurate methods of diagnosing serrated CRC in patients to ensure proper remedial and preventative treatment regimens.

US2009/0170133 describes intact IGFBP-3 as a colon cancer risk factor in patients with IBD. Nakanishi et al. 2018 Immunity 18 49(6*)* report the simultaneous loss of both atypical protein kinase C genes in the intestinal epithelium as a driver of serrated intestinal cancer by impairing immunosurveillance. US2017/0051360 describes diagnostic methods and compositions for treatment of glioblastoma. WO2017/210119 describes the combination of ramucirumab and pembrolizumab for the treatment of certain cancers. Li et al. 2014 PLoS One 9(5*)* report the identification of gene expression changes from colitis to CRC in the mouse CAC model Llado *et al.* 2015 *10(5)* report the repression of intestinal stem cell function and tumorigenesis through direct phosphorylation of β-catenin and Yap by PKCζ. Yeo et al. 2017 J. Cancer 8(16*)* report phosphorylated protein kinase C expression in colorectal adenocarcinoma and its correlation with clinicopathologic characteristics and prognosis. Yoshihama et al. 2012 Cancer Sci. 104(2*)* report that high expression of KIBRA in low atypical protein kinase C-expressing gastric cancer correlates with lymphatic invasion and poor prognosis. Zhu et al. 2015 J. Clin. Exp. Pathol. 8(8*)* report the clinical significance of PD-L1 in colorectal serrated adenocarcinoma. Dung etal. 2015 NEJM 372(6*)* report PD-1 blockade in tumors with mismatch-repair deficiency. WO 2016/033555 describes combination therapy with hyaluronan-degrading enzyme and immune checkpoint inhibitor. Fessler et al. 2016 EMBO Mol. Med. 8(7*)* report that TGFβ signaling directs serrated adenomas to the mesenchymal colorectal cancer subtype.

### SUMMARY

Little is known about the role of the tumor microenvironment, namely the stromal, inflammatory and immune responses, in the development of serrated CRC. CRC is thought to develop through precursor lesions that originate from either conventional or alternative pathways. Serrated CRC originates from endometrial intraepithelial carcinoma (EIC) precursor lesions though the alternative CRC pathway in the absence of changes in conventional biomarkers such as APC or β-catenin. The alternative pathway is initiated by the formation of serrated adenoma, which gradually progress to malignant EIC. EIC is associated with activation of the MAPK cascade, occasionally as a result of activating mutations in *KRAS* or *BRAF.* However, these mutations are not present in all serrated adenomas, so early detection of mutations in *KRAS or BRAF have* limited use in early stage detection of serrated CRC.

Serrated precursor lesions are associated with a transcriptional subtype of CRC that has very poor prognosis. There is no clear therapeutic consensus on how, or whether, CRC originating from the serrated pathway should be treated differently in the clinic than conventional CRC, despite the clear mechanistic differences between the two. Immunotherapy may be a viable option for patients with serrated CRC. In a non-limiting example, Programmed cell death 1 (PD-1) immune checkpoint inhibitors are promising therapeutic solution to certain subclinical phenotypes of serrated CRC, but not all of them. Consequently, understanding the immunological environment of serrated tumors is critically important for identifying more efficacious therapies.

Chronic inflammatory diseases of the intestine are associated with an elevated risk for CRC. Atypical protein kinase C (aPKC) 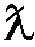 / 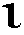 (PKC 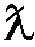 / 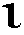 ; encoded by the *PRKCI* gene) is a regulator of intestinal inflammation, particularly in the context of inflammatory bowel diseases (IBD), such as Crohn's disease (CD) and ulcerative colitis (UC). PKC 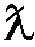 / 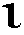 is required for Paneth cell differentiation and is a negative regulator of cell death in the intestinal epithelium. PKC 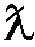 / 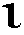 levels decrease in Paneth cells of CD patients in a manner that correlates with disease progression. Furthermore, a Kaplan-Meier survival analysis of CRC patients demonstrated that low PKC 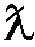 / 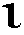 levels correlate with significantly worse patient survival rates. Without wishing to be bound by any particular theory, this proinflammatory environment of the PKC 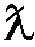 / 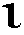 -deficient intestinal epithelium may be linked to a defective barrier due to impaired Paneth cell differentiation and increased intestinal epithelial cell (IEC) death. Interestingly, even in the presence of chronic inflammation, the selective genetic inactivation of PKC 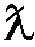 / 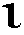 in the intestinal epithelium does not lead to tumorigenesis unless combined with *Apc* deficiency, in which case the number of intestinal adenomas increases significantly through a mechanism that depends on inflammation and the microbiome; however, these adenomas never progress to aggressive and invasive stages. It is thought that aPKC, PKCζ (encoded by the gene PRKCZ), might compensate for the loss of PKC 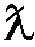 / 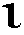 by maintaining pathways that can prevent cancer initiation and progression in PKC 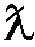 / 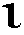 -deficient intestines.

The present disclosure provides that genetic inactivation of both aPKCs results in spontaneous tumorigenesis through the serrated pathway that progress to advanced CRC. Epithelial PKC 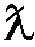 / 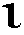 deficiency leads to immunogenic cell death that repressed tumor initiation. While PKCζ loss leads to impaired interferon and CD8+ T cell responses concomitant with stromal activation and immunosuppression. Thus, PKCζ and PKC 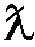 / 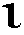 cooperatively suppress serrated tumorigenesis. As such, low levels of PKCζ and PKC 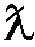 / 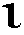 detected in a sample obtained from a subject may be indicative that the subject has, or will develop serrated CRC.

The present disclosure further provides that serrated tumors in mice from mouse cell lines with deletion of PKCζ and PKC 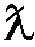 / 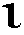 show increased expression of hyaluronan (HA), and CD44 which is a receptor for HA and a cancer stem cell marker. This increase in hyaluronan is concomitant with a significant decrease in other stem cell markers. These findings suggest that detection of either HA or CD44, or the combination of the HA and CD44, in a sample obtained from a subject may be indicative that the subject has, or will, develop serrated CRC.

The invention provides an inhibitor of programmed death-ligand 1 (PD-L1) activity or expression for use in a method for treating a subtype of a disease or condition in a subject, the method comprising: determining a decreased expression level of PKCζ and PKC 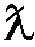 / 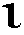 in a biological sample obtained from the subject compared to a level of expression of PKCζ and PKC 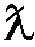 / 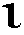 in an individual who does not have the disease or condition; and administering to the subject a therapeutically effective amount of the programmed death-ligand 1 (PD-L1) inhibitor, wherein the subtype of the disease or condition comprises a disease or condition characterized by serrated polyps in an intestine or by serrated adenocarcinoma. The invention is defined by the claims.

Aspects of the disclosure further provide methods of treating a subtype of a disease or condition in a subject by administering to the subject a therapeutically effective amount of an inhibitor of programmed death-ligand 1 (PD-L1) activity or expression, provided low levels of expression of PKCζ and PKC 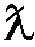 / 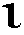 are detected in a biological sample obtained from the subject, as compared to levels of expression of PKCζ and PKC 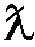 / 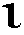 in an individual who does not have the disease or condition. In some aspects, a high level of expression of hyaluronan is detected in a biological sample obtained from the subject, as compared to a level of expression of hyaluronan in an individual who does not have the subtype of the disease or condition. In some aspects, the high level of hyaluronan is detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the high level of expression of hyaluronan comprises ribonucleic acid (RNA), deoxyribonucleic acid (DNA), or protein. In some aspects, the expression of PKCζ and PKC 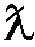 / 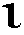 that is detected in the biological sample obtained from the subject is indicative of an increase in expression of hyaluronan in the biological sample, as compared to an individual who does not have the subtype of the disease or condition. In some aspects, the disease or condition comprises colorectal cancer, pancreatic cancer, inflammatory bowel disease, ulcerative colitis, Crohn's disease, fibrosis, fibrostenosis, or a combination thereof. In some aspects, the subtype of the disease or condition comprises a disease or condition characterized by an increase in hyaluronan in the biological sample obtained from the subject, as compared to the individual who does not have the subtype of the disease or condition. In some aspects, the subtype of the disease or condition comprises a disease or condition characterized by serrated polyps in an intestine of the subject. In some aspects, the serrated polyps comprise sessile serrated adenomas (SSAs). In some aspects, the subtype of the disease or condition comprises a disease or condition characterized by serrated adenocarcinoma. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, the methods further comprise administering to the subject a therapeutically effective amount of an inhibitor of hyaluronan activity or expression. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF- β1 activity or expression comprises an antibody, or antigen-binding fragment. In some aspects, the inhibitor of TGF-β1 activity or expression comprises a small molecule. In some aspects, the inhibitor of TGF-β1 activity or expression comprises a small molecule inhibitor of a receptor of TGF- β1. In some aspects, the inhibitor of TGF- β1 activity or expression comprises galunisertib. In some aspects, the biological sample comprises blood, blood plasma, sera, or tissue biopsy. In some aspects, the subject is a human. In some aspects, the subject is a mammal. In some aspects, the low levels of expression of PKCζ and PKC 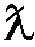 / 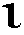 are detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 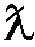 / 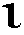 comprise ribonucleic acid (RNA), deoxyribonucleic acid (DNA), or protein.

Aspects disclosed herein provide methods of inhibiting or reducing programmed death-ligand 1 (PD-L1) activity or expression in a subject suffering from a disease or condition, comprising: a) identifying the subject as having low levels of expression of PKCζ and PKCk/t , as compared to the levels of expression of PKCζ and PKC 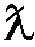 / 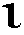 in an individual who does not have the disease or condition; and b) administering to the subject a therapeutically effective amount of an inhibitor of PD-L1 activity or expression. In some aspects, the methods further comprise: identifying the subject as having a high level of expression of hyaluronan, as compared to the level of expression of hyaluronan in an individual who does not have the disease or condition. In some aspects, n the level of expression of hyaluronan is identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the level of expression of hyaluronan comprises ribonucleic acid (RNA), deoxyribonucleic acid (DNA), or protein. In some aspects, the low levels of expression of PKCζ and PKC 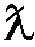 / 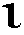 are indicative of an increase in expression of hyaluronan in the subject, as compared to an individual who does not have the disease or condition. In some aspects, the disease or condition comprises colorectal cancer (CRC), serrated CRC, pancreatic cancer, inflammatory bowel disease, ulcerative colitis, Crohn's disease, fibrosis, fibrostenosis, or a combination thereof. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, the methods further comprise administering to the subject a therapeutically effective amount of an inhibitor of hyaluronan activity or expression. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF- β1 activity or expression comprises galunisertib.

In some aspects, the biological sample comprises blood, blood plasma, sera, or tissue biopsy. In some aspects, the subject is a human. In some aspects, the subject is a mammal. In some aspects, the levels of expression of PKCζ and PKC 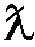 / 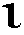 are identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 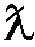 / 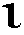 comprise ribonucleic acid (RNA), deoxyribonucleic acid (DNA), or protein.

Aspects disclosed herein provide methods of treating a subtype of a disease or condition in a subject by administering to the subject a therapeutically effective amount of an inhibitor of hyaluronan activity or expression, provided low levels of expression of PKCζ and PKC 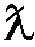 / 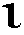 are detected in a biological sample obtained from the subject, as compared to levels of expression of PKCζ and PKC 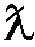 / 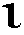 in an individual who does not have the disease or condition. In some aspects, a high level of hyaluronan is detected in a biological sample obtained from the subject, as compared to the level of expression of hyaluronan in an individual who does not have the subtype of the disease or condition. In some aspects, the high level of hyaluronan is detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 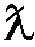 / 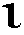 are detected comprise ribonucleic acid (RNA), deoxyribonucleic acid (DNA), or protein. In some aspects, the expression of PKCζ and PCK 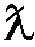 / 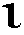 that are detected in the biological sample obtained from the subject are indicative of an increase in expression of hyaluronan in the biological sample, as compared to an individual who does not have the subtype of the disease or condition. In some aspects, the disease or condition comprises colorectal cancer, pancreatic cancer, inflammatory bowel disease, ulcerative colitis, Crohn's disease, fibrosis, fibrostenosis, or a combination thereof. In some aspects, the subtype of the disease or condition comprises a disease or condition characterized by an increase in hyaluronan in the biological sample obtained from the subject, as compared to the individual who does not have the subtype of the disease or condition. In some aspects, the subtype of the disease or condition comprises a disease or condition characterized by serrated polyps in an intestine of the subject. In some aspects, the serrated polyps comprise sessile serrated adenomas (SSAs). In some aspects, the subtype of the disease or condition comprises a disease or condition characterized by serrated adenocarcinoma. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF- β1 activity or expression comprises galunisertib. In some aspects, the methods further comprise administering to the subject a therapeutically effective amount of an inhibitor of programmed death-ligand 1 (PD-L1) activity or expression. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, the biological sample comprises blood, blood plasma, sera, or tissue biopsy. In some aspects, the subject is a human. In some aspects, the subject is a mammal. In some aspects, the levels of expression of PKCζ and PKC 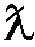 / 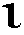 are detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 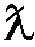 / 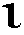 comprise ribonucleic acid (RNA), deoxyribonucleic acid (DNA), or protein.

Aspects disclosed herein provide methods of inhibiting or reducing hyaluronan activity or expression in a subject suffering from a disease or condition, comprising: a) identifying the subject as having low levels of expression of PKCζ and PKC 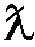 / 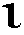 , as compared to the levels of expression of PKCζ and PKC 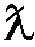 / 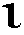 in an individual who does not have the disease or condition; and b) administering to the subject a therapeutically effective amount of an inhibitor of hyaluronan activity or expression. In some aspects, the methods further comprise: identifying the subject as having a high level of expression of hyaluronan, as compared to the level of expression of hyaluronan in an individual who does not have the disease or condition. In some aspects, the level of expression of hyaluronan is identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the high level of expression of hyaluronan comprises ribonucleic acid (RNA), deoxyribonucleic acid (DNA), or protein. In some aspects, the low levels of expression of PKCζ and PKC 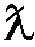 / 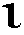 are indicative of an increase in expression of hyaluronan in the subject, as compared to an individual who does not have the disease or condition. In some aspects, the disease or condition comprises colorectal cancer (CRC), serrated CRC, pancreatic cancer, inflammatory bowel disease, ulcerative colitis, Crohn's disease, fibrosis, fibrostenosis, or a combination thereof. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF-β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF- β1 activity or expression comprises galunisertib. In some aspects, the methods further comprise administering to the subject a therapeutically effective amount of an inhibitor of programmed death-ligand 1 (PD-L1) activity or expression. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, the biological sample comprises blood, blood plasma, sera, or tissue biopsy. In some aspects, the subject is a human. In some aspects, the subject is a mammal. In some aspects, the levels of expression of PKCζ and PKC 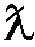 / 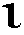 are identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 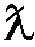 / 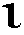 comprise ribonucleic acid (RNA), deoxyribonucleic acid (DNA), or protein. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase.

Aspects disclosed herein provide methods of ameliorating or preventing tumorigenesis in a subject with serrated colorectal cancer (CRC) by administering to the subject a therapeutically effective amount of an inhibitor of hyaluronan activity or expression, provided low levels of expression of PKCζ and PKC 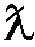 / 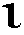 are detected in a biological sample obtained from the subject, as compared to levels of expression of PKCζ and PKC 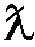 / 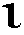 in an individual who does not have serrated CRC. In some aspects, a high level of expression of hyaluronan is detected in a biological sample obtained from the subject, as compared to a level of expression of hyaluronan in an individual who does not have the CRC. In some aspects, the high level of expression of hyaluronan is detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the high level of expression of hyaluronan comprises ribonucleic acid (RNA), deoxyribonucleic acid (DNA), or protein. In some aspects, the low levels of expression of PKCζ and PCK 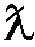 / 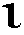 that are detected in the biological sample obtained from the subject are indicative of an increase in expression of hyaluronan in the biological sample, as compared to an individual who does not have the serrated CRC. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF-β1 activity or expression comprises galunisertib. In some aspects, the methods further comprise administering to the subject a therapeutically effective amount of an inhibitor of programmed death-ligand 1 (PD-L1) activity or expression. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, the biological sample comprises blood, blood plasma, sera, or tissue biopsy. In some aspects, the subject is a human. In some aspects, the subject is a mammal. In some aspects, the low levels of expression of PKCζ and PKC 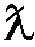 / 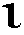 are detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 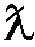 / 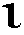 comprise ribonucleic acid (RNA), deoxyribonucleic acid (DNA), or protein.

Aspects disclosed herein provide methods of ameliorating or preventing tumorigenesis in a subject with serrated colorectal cancer (CRC) by, the method comprising: a) identifying the subject as having low levels of expression of PKCζ and PKC 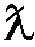 / 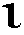 , as compared to the levels of expression of PKCζ and PKC 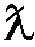 / 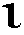 in an individual who does not have the serrated CRC; and b) administering to the subject a therapeutically effective amount of an inhibitor of hyaluronan activity or expression. In some aspects, the methods further comprise: identifying the subject as having a high level of expression of hyaluronan, as compared to the level of expression of hyaluronan in an individual who does not have the CRC. In some aspects, the high level of expression of hyaluronan is identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the level of expression of hyaluronan comprises ribonucleic acid (RNA), deoxyribonucleic acid (DNA), or protein. In some aspects, the low levels of expression of PKCζ and PKC 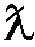 / 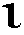 are indicative of an increase in expression of hyaluronan in the subject, as compared to an individual who does not have the disease or condition. In some aspects, the biological sample comprises blood, blood plasma, sera, or tissue biopsy. In some aspects, the subject is a human. In some aspects, the subject is a mammal. In some aspects, the levels of expression of PKCζ and PKC 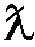 / 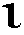 are identified by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 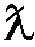 / 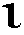 comprise ribonucleic acid (RNA), deoxyribonucleic acid (DNA), or protein. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise administering to the subject a therapeutically effective amount of an inhibitor of transforming growth factor beta 1 (TGF-β1) activity or expression. In some aspects, the inhibitor of TGF- β1 activity or expression comprises an antibody, or antigen-binding fragment. In some aspects, the inhibitor of TGF-β1 activity or expression comprises a small molecule. In some aspects, the inhibitor of TGF-β1 activity or expression comprises a small molecule inhibitor of a receptor of TGF- β1. In some aspects, the inhibitor of TGF- β1 activity or expression comprises galunisertib. In some aspects, the methods further comprise administering to the subject a therapeutically effective amount of an inhibitor of programmed death-ligand 1 (PD-L1) activity or expression. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody.

Aspects disclosed herein provide methods of diagnosing a subtype of colorectal cancer (CRC) in a subject in need thereof, the method comprising: a)obtaining a biological sample from a subject in need thereof; b) subjecting the biological sample to an assay suitable to detect levels of expression of PKCζ and PKC 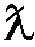 / 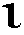 ; and c) diagnosing the subject with serrated colorectal cancer (CRC), provided the levels of expression of PKCζ and PKC 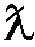 / 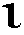 detected in the biological sample obtained from the subject are low, as compared to levels of expression of PKCζ and PKC 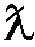 / 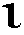 in an individual that does not have the CRC. In some aspects, the methods further comprise: a) subjecting the biological sample to an assay suitable to detect a colon cancer subtype (CCS), the CCS comprising chromosomal unstable phenotype (CCS1), microsatellite instability-high and CpG island methylator phenotypes (MSI/CIMP+), or microsatellite stable phenotype (MSS); and b) diagnosing the subject with serrated CRC, provided the CCS detected comprises the MSS phenotype. In some aspects, the low levels of expression of PKCζ and PKC 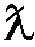 / 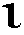 are indicative of an increase in expression of hyaluronan in the subject, as compared to an individual who does not have the disease or condition. In some aspects, the biological sample comprises blood, blood plasma, sera, or tissue biopsy. In some aspects, the subject is a human. In some aspects, the subject is a mammal. In some aspects, the assay suitable to detect the levels of expression of PKCζ and PKC 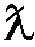 / 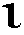 comprises polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 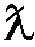 / 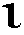 comprise ribonucleic acid (RNA), deoxyribonucleic acid (DNA), or protein. In some aspects, the methods further comprise treating the subject by administering a therapeutically effective amount of an inhibitor of transforming growth factor beta 1 (TGF-β1) activity or expression, provided the subject is diagnosed with serrated CRC. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF-β1 activity or expression comprises galunisertib. In some aspects, the methods further comprise treating the subject by administering a therapeutically effective amount of an inhibitor of hyaluronan activity or expression, provided the subject is diagnosed with serrated CRC. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise treating the subject by administering a therapeutically effective amount of an inhibitor of programmed death-ligand 1 (PD-L1) activity or expression, provided the subject is diagnosed with serrated CRC. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody.

Aspects disclosed herein provide methods of characterizing a subtype of colorectal cancer (CRC) in a subject, the method comprising: a) obtaining a biological sample from a subject with colorectal cancer (CRC); b) subjecting the biological sample to an assay suitable to detect levels of expression of PKCζ and PKC 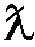 / 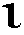 ; and c) characterizing the CRC as serrated CRC, provided the levels of expression of PKCζ and PKC 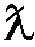 / 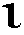 detected in the biological sample obtained from the subject are low, as compared to the levels of expression of PKCζ and PKC 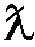 / 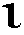 in an individual that does not have the CRC. In some aspects, the methods further comprise: a) subjecting the biological sample to an assay suitable to detect a colon cancer subtype (CCS), the CCS comprising chromosomal unstable phenotype (CCS1), microsatellite instability-high and CpG island methylator phenotypes (MSI/CIMP+), or microsatellite stable phenotype (MSS); and b) characterizing the subtype of CRC as serrated CRC, provided the CCS detected comprises the MSS phenotype. In some aspects, the low levels of expression of PKCζ and PKC 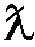 / 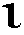 are indicative of an increase in expression of hyaluronan in the subject, as compared to an individual who does not have the disease or condition. In some aspects, the biological sample comprises blood, blood plasma, sera, or tissue biopsy. In some aspects, the subject is a human. In some aspects, the subject is a mammal. In some aspects, the assay suitable to detect the levels of expression of PKCζ and PKC 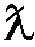 / 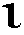 comprises polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 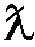 / 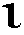 comprise ribonucleic acid (RNA), deoxyribonucleic acid (DNA), or protein. In some aspects, the methods further comprise treating the subject by administering a therapeutically effective amount of an inhibitor of transforming growth factor beta 1 (TGF-β1) activity or expression, provided the CRC is characterized as serrated CRC. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF-β1 activity or expression comprises galunisertib. In some aspects, the methods further comprise treating the subject by administering a therapeutically effective amount of an inhibitor of hyaluronan activity or expression, provided the CRC is characterized as serrated CRC. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise treating the subject by administering a therapeutically effective amount of an inhibitor of programmed death-ligand 1 (PD-L1) activity or expression, provided the CRC is characterized as being serrated CRC. In some aspects, the inhibitor of PD-L1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody.

Aspects disclosed herein provide methods of determining whether a subject suffering from an inflammatory bowel disease (IBD) has, or will, develop serrated colorectal cancer (CRC), the method comprising: a) obtaining a biological sample from the subject; b) subjecting the biological sample to an assay suitable to detect levels of expression of PKCζ and PKC 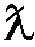 / 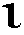 ; and c) determining that the subject has, or will develop, serrated CRC, provided the levels of expression of PKCζ and PKC 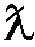 / 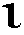 detected in the biological sample obtained from the subject are low, as compared to levels of expression of PKCζ and PKC 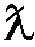 / 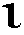 in an individual that does not have CRC. In some aspects, the IBD comprises Crohn's disease, ulcerative colitis, medically refractory ulcerative colitis, medically refractory or complicated form of Crohn's disease, colitis, fibrosis, fibrostenosis, or a combination thereof. In some aspects, the low levels of expression of PKCζ and PKC 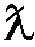 / 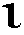 are indicative of an increase in expression of hyaluronan in the subject, as compared to an individual who does not have the disease or condition. In some aspects, the biological sample comprises blood, blood plasma, sera, or tissue biopsy. In some aspects, the subject is a human. In some aspects, the subject is a mammal. In some aspects, the assay suitable to detect levels of expression of PKCζ and PKC 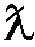 / 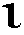 comprises polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof. In some aspects, the levels of expression of PKCζ and PKC 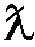 / 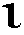 comprise ribonucleic acid (RNA), deoxyribonucleic acid (DNA), or protein. In some aspects, the methods further comprise treating the subject by administering a therapeutically effective amount of an inhibitor of transforming growth factor beta 1 (TGF-β1) activity or expression, provided the subject is determined to have, or will develop, serrated CRC. In some aspects, in the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF- β1. In some aspects, the inhibitor of TGF- β1 activity or expression comprises galunisertib. In some aspects, the methods further comprise treating the subject by administering a therapeutically effective amount of an inhibitor of hyaluronan activity or expression, provided the subject is determined to have, or will develop, serrated CRC. In some aspects, the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the methods further comprise treating the subject by administering a therapeutically effective amount of an inhibitor of programmed death-ligand 1 (PD-L1) activity or expression, provided the subject is determined to have, or will develop, serrated CRC. In some aspects, the inhibitor of PD-L 1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody.

Aspects disclosed herein provide models of serrated colorectal cancer comprising: a) a quantity of cells that do not express, or underexpress, PKCζ and PKC 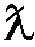 / 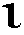 ; b) an animal that has been injected with the quantity of the cells that do not express, or underexpress, PKCζ and PKC 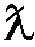 / 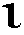 ; or c) a transgenic animal with a transgene comprising a knockout or knockdown of Prkci and Prkcz. In some aspects, the quantity of cells and the transgenic animal are further characterized by a microsatellite stable phenotype. In some aspects, the quantity of cells and the transgenic animal are enriched for an expression of one or more genes in the Extracellular Signal-Regulated Kinase (ERK) pathway and/or Yes Associated Protein (YAP) pathway. In some aspects, the quantity of cells exhibit lower cell death, as compared to a quantity of cells that do not express, or underexpress, PKCζ or PKC 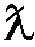 / 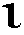 . In some aspects, the transgenic animal exhibits lower cell death, as compared to a transgenic animal with a transgene comprising a knockout or knockdown of Prkci or Prkcz. In some aspects, the quantity of cells overexpress hyaluronan. In some aspects, the transgenic animal overexpresses hyaluronan in stroma of serrated tumors of the transgenic animal. In some aspects, the quantity of cells overexpress CD44. In some aspects, the transgenic animal overexpresses CD44. In some aspects, the quantity of cells underexpress stem cell biomarkers comprising Olfm4, Ascl2, or Lgr5. In some aspects, the transgenic animal underexpresses a stem cell biomarker comprising Olfm4, Ascl2, or Lgr5. In some aspects, the transgenic animal underexpresses an interferon-responsive gene comprising Irf7, Oasia, Isg15, or Cxcl10. In some aspects, the quantity of cells underexpresses an interferon-responsive gene comprising Irf7, Oasia, Isg15, or Cxcl10. In some aspects, the transgenic animal comprises a mammal. In some aspects, the transgenic animal comprises a mouse.In some aspects, the transgenic animal comprises a rat. In some aspects, the transgenic animal comprises a monkey. In some aspects, the quantity of cells comprises an organoid. In some aspects, the quantity of cells comprises a 3D culture. In some aspects, the transgenic animal is produced by breeding a first animal comprising a transgene encoding a recombinase operably linked to a ubiquitous promoter in villus and crypt epithelial cells of a small and a large intestine, and a second animal comprising a loxP-flanked genes comprising Prkci and Prkcz, to produce a transgenic animal with a knockout or knockdown of Prkci and Prkcz, respectively, the villus and the crypt epithelial cells of the small and the large intestine. In some aspects, the ubiquitous promoter comprises a vasa promoter, c-kit promoter, Dnd1 promoter, Dppa3 promoter, Fkbp6 promoter, Fragilis promoter, Fragilis-2 promoter, GDF-3 promoter, Mov10l1 promoter, Nanog promoter, Nanos2 promoter, Nanos3 promoter, oct3/4 promoter, Prdm1 promoter, Prdm14 promoter, Tex13 promoter, Tiar promoter or TNAP promoter.

Aspects disclosed herein provide methods of screening for modulators of serrated tumorigenesis, the method comprising: a) providing the model of serrated colorectal cancer disclosed herein b) contacting the model with a putative modulator of tumorigenesis; and c) detecting one or more changes in one or more tumor-associated parameters in the model. In some aspects, the tumor-associated parameter comprises recruitment of CD8+ T cells to a serrated tumor, progression of a sessile serrated adenoma (SSA)to adenocarcinoma, a number of serrated tumors, a size of serrated tumors, a total load of serrated tumors, an increase in an expression of alpha-smooth muscle actin (αSMA), or collagen deposition, or a combination thereof. In some aspects, the putative modulator negatively modulates tumorigenesis, provided an increase in CD8+ T cells to a serrated tumor is observed. In some aspects, the putative modulator negatively modulates tumorigenesis, provided the progression of the SSA to adenocarcinoma is not observed. In some aspects, the putative modulator negatively modulates tumorigenesis, provided that a decrease in a number of serrated tumors, as compared to the number of serrated tumors in the model before contacting the model with the putative modulator, is observed. In some aspects, the putative modulator negatively modulates tumorigenesis, provided that a reduction in a size of serrated tumor, as compared to the size of serrated tumors in the model before contacting the model with the putative modulator, is observed. In some aspects, the putative modulator negatively modulates tumorigenesis, provided that a decrease in a total load of serrated tumors, as compared to the total load of serrated tumors in the model before contacting the model with the putative modulator, is observed. In some aspects, the putative modulator negatively modulates tumorigenesis, provided that an increase in expression of αSMA, as compared to the expression of αSMA in the model before contacting the model with the putative modulator, is not observed. In some aspects, the putative modulator negatively modulates tumorigenesis, provided than an increase in collagen deposition in the model, as compared to collagen deposition in the model before contacting the model with the putative modulator, is not observed. In some aspects, the putative modulator is selected from the group consisting of an inhibitor of programmed death-ligand 1 (PD-L1) activity or expression, an inhibitor of transforming growth factor beta 1 (TGF-β1) activity or expression, and an inhibitor of hyaluronan activity or expression. In some aspects, the inhibitor of PD-L 1 activity or expression comprises an antibody, or antigen-binding fragment. In some aspects, the inhibitor of PD-L1 activity or expression comprises a small molecule. In some aspects, the inhibitor of PD-L1 activity or expression comprises an antagonist of PD-L1. In some aspects, the inhibitor of PD-L1 activity or expression comprises an antagonist of programmed cell death protein 1 (PD-1). In some aspects, the inhibitor of PD-L1 activity or expression comprises an anti-PD-L1 antibody. In some aspects, the inhibitor of PD-L1 activity or expression comprises an anti-PD-1 antibody. In some aspects, in the inhibitor of hyaluronan activity or expression comprises an antibody, or antigen-binding fragment. In some aspects, the inhibitor of hyaluronan activity or expression comprises a small molecule. In some aspects, the inhibitor of hyaluronan activity or expression comprises an inhibitor of hyaluronan-CD44 binding or agonism. In some aspects, the inhibitor of hyaluronan activity or expression comprises an antagonist of CD44. In some aspects, the inhibitor of hyaluronan activity or expression comprises an antibody inhibitor of CD44 activity or expression. In some aspects, the inhibitor of hyaluronan activity or expression comprises a small molecule inhibitor of CD44 activity or expression. In some aspects, the inhibitor of hyaluronan activity or expression comprises an agonist of hyaluronidase activity or expression. In some aspects, n the inhibitor of hyaluronan activity or expression comprises Pegvorhyaluronidase alfa (PVHA). In some aspects, the inhibitor of hyaluronan activity or expression comprises exogenous hyaluronidase. In some aspects, the inhibitor of hyaluronan activity or expression comprises recombinant hyaluronidase. In some aspects, the inhibitor of TGF- β1 activity or expression comprises an antibody, or antigen-binding fragment. In some aspects, the inhibitor of TGF- β1 activity or expression comprises a small molecule. In some aspects, the inhibitor of TGF- β1 activity or expression comprises a small molecule inhibitor of a receptor of TGF-β1. In some aspects, the inhibitor of TGF- β1 activity or expression comprises galunisertib.

Aspects disclosed herein provide methods of producing an animal model of serrated colorectal cancer comprising: a) providing an animal that is Prkci fl/fl and Prkcz fl/fl, wherein the animal has loxP sites flanking genes comprising Prkci and Prkcz; and b) crossing the animal that is Prkci fl/fl and Prkcz fl/fl with an animal expressing a Cre recombinase operably linked to an ubiquitous promoter in villus and crypt epithelial cells of a small and a large intestine, thereby generating a transgenic animal with deletions in Pkcζ and PKC 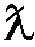 / 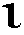 in the villus and the crypt epithelial cells of the small and the large intestine. In some aspects, the animal model comprises a mammal. In some aspects, the animal model comprises a mouse. In some aspects, the animal model comprises a rat. In some aspects, the animal model comprises a monkey. In some aspects, the animal model is characterized by a microsatellite stable phenotype. In some aspects, the animal model is enriched for an expression of one or more genes in the Extracellular Signal-Regulated Kinase (ERK) pathway and/or Yes Associated Protein (YAP) pathway. In some aspects, the animal model exhibits lower cell death, as compared to an animal that does not express, or underexpress, PKCζ or PKC 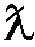 / 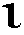 . In some aspects, the animal model exhibits lower cell death, as compared to an animal with a transgene comprising a knockout or knockdown of Prkci or Prkcz. In some aspects, the animal model overexpresses hyaluronan in stroma of serrated tumors of the animal. In some aspects, the animal model overexpresses CD44, as compared to a normal animal. In some aspects, the animal model underexpresses a stem cell biomarker comprising Olfm4, Ascl2, or Lgr5, as compared to a normal animal. In some aspects, the animal model underexpresses an interferon-responsive gene comprising Irf7, Oas1a, Isg15, or Cxcl10, as compared to a normal animal.

### BRIEF DESCRIPTION OF THE DRAWINGS

The invention is defined by the claims. A better understanding of the features and advantages of the present invention will be obtained by reference to the following detailed description, in which the principles of the disclosure are utilized, and the accompanying drawings of which:

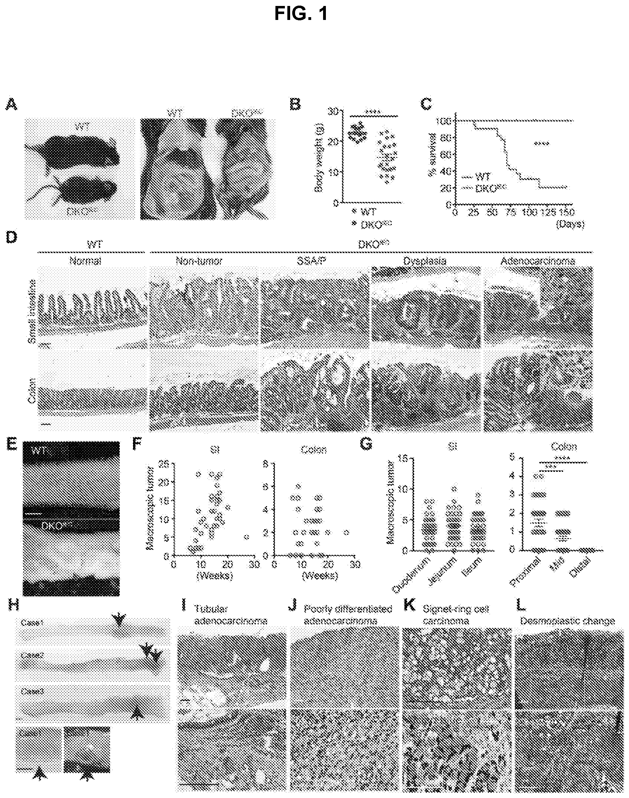

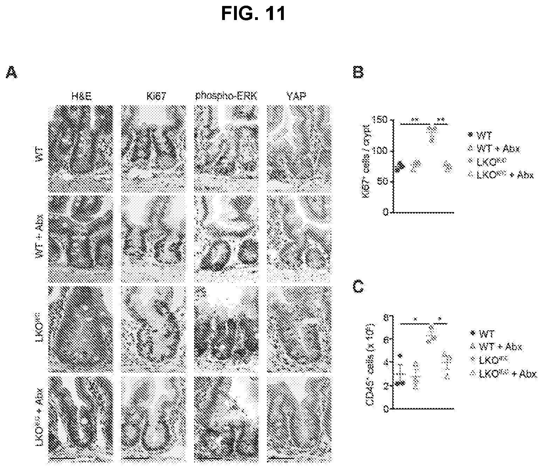

**FIG. 1** exemplifies simultaneous inactivation of PKC 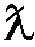 / 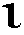 and PKCζ drives serrated tumorigenesis in small intestine and colon. **FIG. 1A** exemplifies cross images of wild-type (WT) and Pkci and Pkcz knockout (DKO^{IEC}). Arrows denote dilated intestine. **FIG.1B** exemplifies body weight of WT (n=15) and DKO^{IEC} mice (n-23). **FIG. 1C** exemplifies Kaplan-Meier survival curve of WT (n=20) and DKO^{IEC} (n=22) mice. **FIG. 1D** exemplifies hematoxylin and eosin (H&E) staining of small intestine and colon sections from WT and DKO^{IEC mice} (n=20). Scale bars, 100µm. **FIG. 1E** exemplifies macroscopic images of small intestines from WT and DKO^{IEC} mice. Arrows denote tumors. Scale bar, 2mm. **FIG. 1F** exemplify the number of macroscopic tumors at different ages in small intestine and colon of DKO^{IEC} mice. **FIG. 1G** exemplifies the number of macroscopic tumors with different tumor size distribution in small intestine and colon of DKO^{IEC} mice. Each circle represents one mouse (n-39). **FIG. 1H** exemplifies gross images of colons from of DKO^{IEC} mice. Arrows denote tumors in ascending colons. Dotted circle denotes mucin in tumor. Scale bars, 2mm. **FIG. 11** exemplifies images of tubular adenocarcinoma observed in DKO^{IEC} mice. **FIG. 1J** exemplifies poorly differentiated adenocarcinoma observed in DKO^{IEC} mice. **FIG. 1K** exemplifies signet-ring cell carcinoma observed in DKO^{IEC} mice. **FIG. 1L** exemplifies desmoplastic change observed in DKO^{IEC} mice. Arrows denote invasive tumor front. Scale bars, 100µm. Results are shows as mean +/- standard error of the mean (SEM).

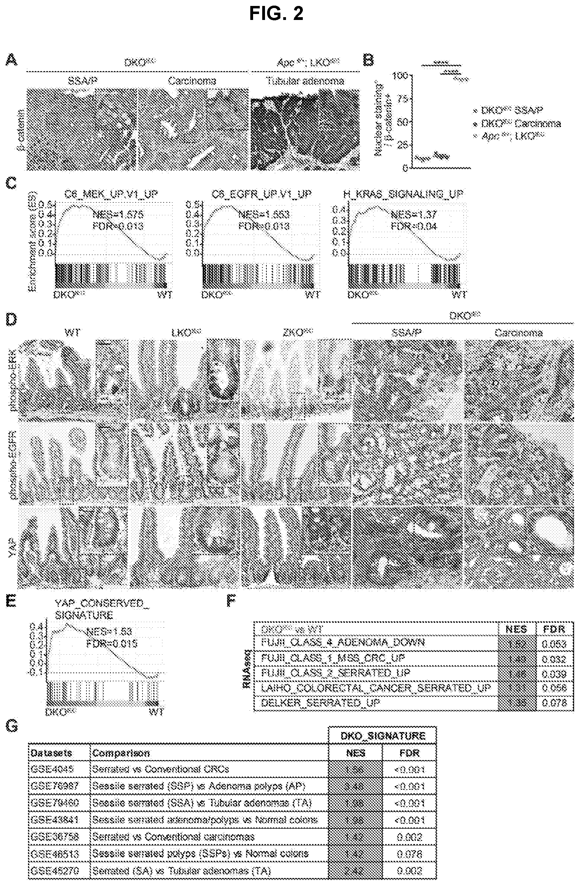

**FIG. 2** exemplifies DKO^{IEC} mice tumors carry a distinct molecular signature which resembles human serrated tumors. **FIG. 2A** and **FIG. 2B** exemplify catenin staining and quantification in the indicated types of intestinal tumors from DKO^{IEC} or Apc^{fl/+}; LKO^{IEC} mice (n = 3). Scale bar, 50 µm. **FIG. 2C** exemplifies gene set enrichment analysis (GSEA) plots of enrichment of the indicated gene signatures in DKO^{IEC} tumor vs. wild-type (WT) intestine. The term, "NES," refers to the normalized enrichment score.The term, "FDR," refers to the false discovery rate. **FIG. 2D** exemplifies phospho-ERK, phospho-EGFR, and YAP staining in small intestines from mice of the indicated genotype (n = 3). Scale bars, 25 µm. **FIG. 2E** exemplifies a GSEA plot of DKO^{IEC} tumor vs. WT intestine. **FIG. 2F** exemplifies a GSEA of transcriptomic data from RNA-seq on DKO^{IEC} tumors vs. WT intestine. **FIG. 2G** exemplifies a GSEA of enrichment of the DKO^{IEC} mouse gene set (DKO_SIGNATURE) in human serrated datasets. Results are shown as mean +/- SEM.

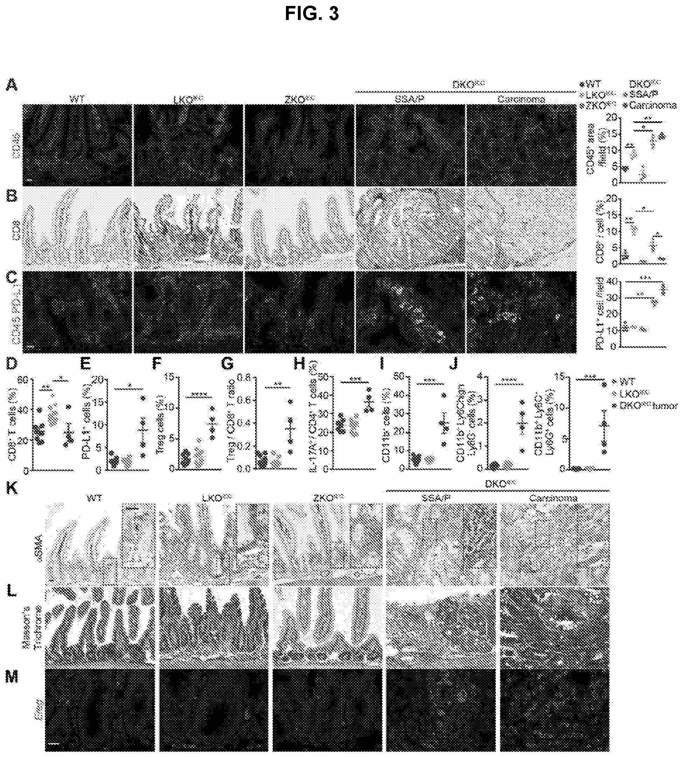

**FIG. 3** exemplifies intestinal immunosuppression in DKO^{IEC} tumors. **FIG. 3A****-****FIG. 3C** exemplify staining for CD45, CD8, and CD45 and PD-L1 in small intestines from mice of the indicated genotypes and lesions, and quantification (n = 3). Lines mark the tumor (T) border. **FIG. 3D****-****FIG. 3J** exemplifies flow cytometry analyses of immune cell populations from mice of the indicated genotypes. Percentage of CD8+ T cells (FIG. 3D), PD-L1+ **(****FIG. 3E****),** Treg cells (Foxp3+CD4+TCR-β+; **FIG. 3F****)** in CD45+ cells; ratio of Treg cells to CD8+ T cells **(****FIG. 3G****);** percentage of IL-17A-producing cells in CD4+ T cells **(****FIG. 3H****);** percentage of CD11b+ **(****FIG. 3I****)** and CD11b+Ly6ChighLy6G- and CD11b+Ly6C+Ly6G+ (J) cells in CD45+ cells. (n: WT = 7, LKO^{IEC} = 8, and DKO^{IEC} tumor = 4). **FIG. 3K****-****FIG. 3M****)** αSMA **(****FIG. 3K****)** and Masson's trichrome **(****FIG. 3L****)** staining, and Ereg in situ hybridization **(****FIG. 3M****)** in small intestines from mice of the indicated genotypes and lesions (n = 3). Results are shown as mean +/- SEM. Scale bars, 25µm.

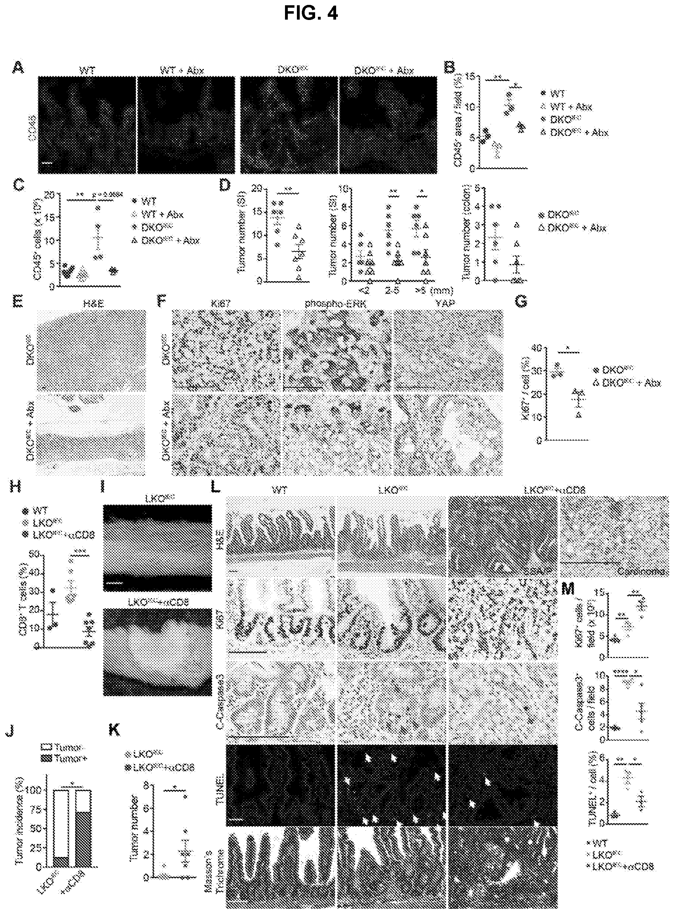

**FIG. 4** exemplifies the role of CD8+ T cells in serrated tumorigenesis in DKO^{IEC} mice. **FIG. 4A** and **FIG. 4B** exemplify CD45+ staining (FIG. 4A) and quantification **(****FIG. 4B****)** in small intestines from WT and DKO^{IEC} mice with or without antibiotic (Abx) treatment (n = 3). **FIG. 4C** exemplifies flow cytometry analyses of CD45+ cell populations from WT and DKO^{IEC} mice in the Abx experiment (n: WT Ctrl = 8, WT Abx-treated = 5, DKO^{IEC} Ctrl = 4, and DKO^{IEC} Abx-treated = 3). **FIG. 4D** exemplifies quantification of the total numbers of tumors in small intestine and colon, and stratification of small-intestinal tumor numbers according to size in DKO^{IEC} mice in the Abx experiment. (n: Ctrl =6 and Abx-treated = 7). **FIG. 4E****-****FIG. 4G** exemplify haemotoxylin and eosin (H&E) staining (FIG. 4E) and Ki67, phospho-ERK, and YAP staining **(****FIG. 4F****)** of small-intestinal tumors from DKO^{IEC} mice in the Abx experiment, and quantification of Ki67 staining **(****FIG. 4G****).** Scale bars, 100 µm. **FIG. 4H** exemplifies flow cytometry analyses of CD8+ T cells from WT and LKO^{IEC} mice treated with or without αCD8 antibody (n: WT = 3, LKO^{IEC} = 6, and LKO^{IEC} αCD8 antibody-treated = 7). **FIG. 4I** exemplifies macroscopic images of LKO^{IEC} small intestines treated in the CD8-depletion experiment. The arrow denotes tumor. Scale bar, 2 mm. **FIG. 4J** and **FIG. 4K** exemplify quantification of incidence **(****FIG. 4J****)** and total number **(****FIG. 4K****)** of small-intestinal tumors in the CD8-depletion experiment (n = 7). **FIG. 4L** and **FIG. 4M** exemplify H&E, Ki67, cleaved caspase 3 (C-Caspase3), TUNEL, and Masson's trichrome staining of small-intestinal samples from mice of the indicated genotypes and treatment **(****FIG. 4L****),** and quantification **(****FIG. 4M****)** (n = 3-5). Scale bars, 100 µm. Results are shown as mean +/- SEM.

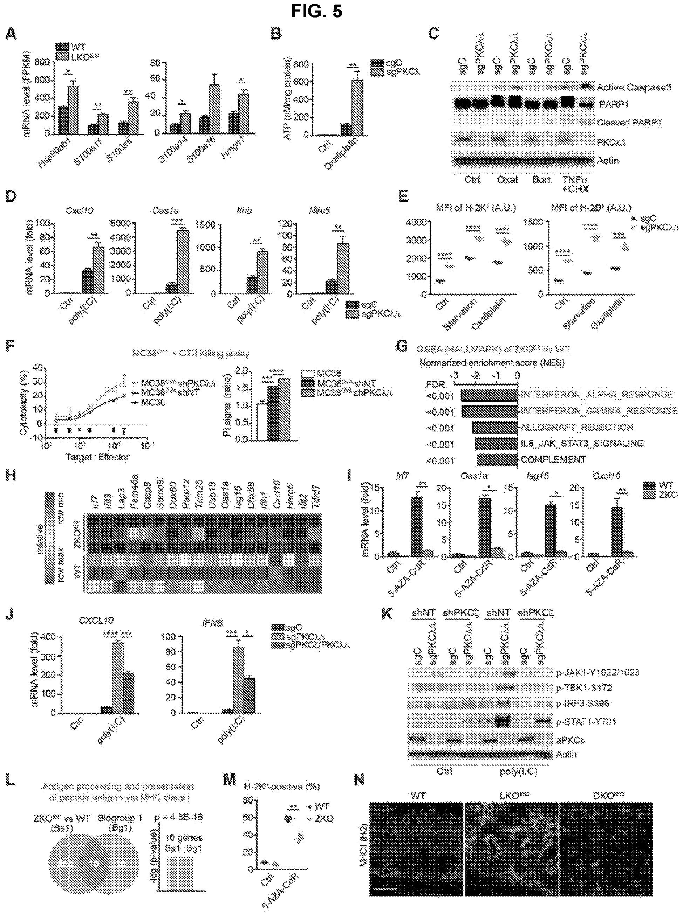

**FIG. 5** exemplifies suppression of the interferon response by PKCζ loss impairs CD8+-dependent immunosurveillance. **FIG. 5A** exemplifies mRNA expression for the indicated genes in LKO^{IEC} vs. WT IECs from RNA-seq data. **FIG. 5B** exemplifies extracellular ATP levels in sgC and sgPKC 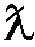 / 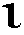 MODE-K cells treated with Oxaliplatin for 24 hr (20 µM) (n = 3). **FIG. 5** **C** exemplifies a western blot of indicated proteins in sgC and sgPKC 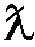 / 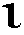 / 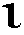 MODE-K cells treated for 24 hr with Oxaliplatin (20 µM), Bortezomib (0.1 µM) or TNFα (10 ng/ml) plus cycloheximide (2.5 µg/ml). **FIG. 5D** exemplifies qRT-PCR analyses of Cxcl 10, Oas1a, Ifnb, and Nlrc5 mRNA levels in MODE-K of the indicated genotypes transfected with 0.5 µg/ml of poly(I:C) or mock transfected for 6 hr (n = 3). **FIG. 5E** exemplifies surface expression (mean fluorescence intensity, MFI) of H-2Kb and H-2Dk in MODE-K cells of the indicated genotypes either HBSS-starved or treated with Oxaliplatin (20 µM) for 24 hr (n =3). **FIG. 5F** exemplifies OT-I cells-MC38OVA killing assay (24 hr) at the indicated T:E ratios (left). PI staining in shNT and sgPKC 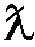 / 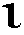 MC38^{OVA} cells after coculture with OT-I cells for 24 hr (right) (n = 3). **FIG. 5G** exemplifies GSEA of transcriptomic data from RNA-seq on ZKO^{IEC} vs. WT IECs. The indicated gene signatures were applied to the analyses. FDR, false discovery rate. **FIG. 5H** exemplifies a heatmap of RNA-seq data of WT and ZKO^{IEC} IECs representing the genes related to interferon response differentially expressed between genotypes. **FIG. 5I** exemplifies qRT-PCR analyses of Irf7, Oasia, Isg15, and Cxcl10 mRNA levels in organoids of the indicated genotypes with or without 5AZA-CdR treatment. **FIG. 5J** exemplifies qRT-PCR analyses of CXCL10 and IFNB mRNA levels in 293T cells of the indicated genotypes transfected with 0.5 µg/ml of poly(I:C) or mock transfected for 6 hr (n = 3). **FIG. 5K** exemplifies a western blot of indicated proteins in 293T cells of the indicated genotypes transfected with 0.5 µg/ml of poly(I:C) or mock transfected for 6 hr (n = 3). **FIG. 5L** exemplifies a NextBio analysis of genes differentially expressed in ZKO^{IEC} vs. WT IECs. Venn diagram show the number of common and unique genes in both sets. Gene signatures corresponding to GO terms "antigen processing and presentation of peptide antigen via MHC class l". **FIG. 5M** exemplifies a flow cytometry analysis of H-2Kb-positive cell population in organoids of the indicated genotypes with or without 5-AZA-CdR treatment (n = 3). **FIG. 5N** exemplifies MHC I (H-2) staining in small intestines from mice of the indicated genotypes (n = 3). Scale bar, 50 µm. Results are shown as mean +/- SEM.

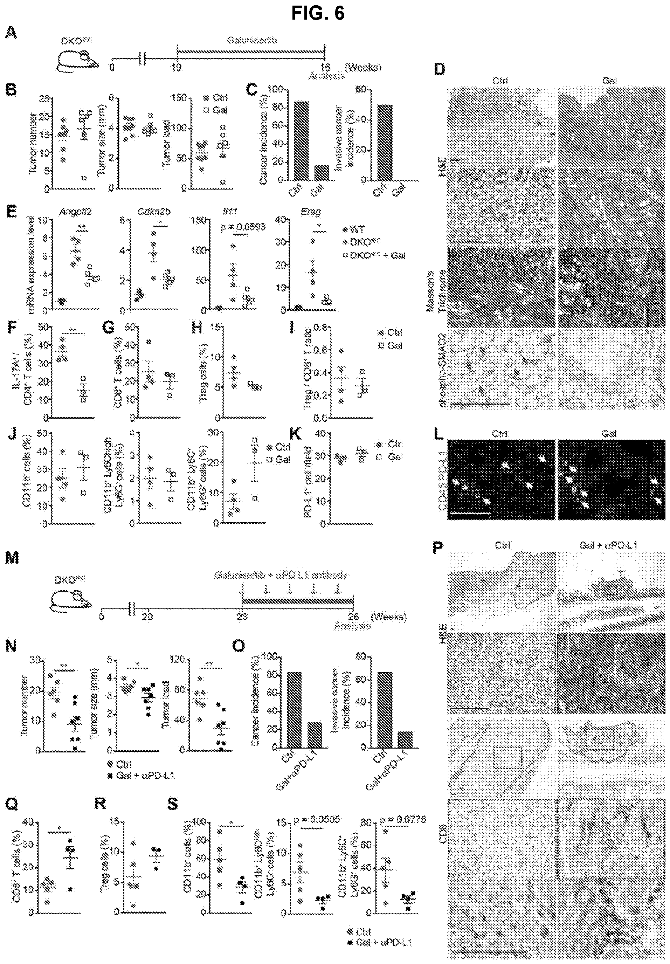

**FIG. 6** exemplifies therapeutic interventions in DKO^{IEC} mouse tumors. **FIG. 6A** exemplifies an experimental design for galunisertib (Gal) treatment. **FIG. 6B** and **FIG. 6C** exemplify quantification of the total number, average size, and load of small-intestinal tumors **(****FIG. 6B****)** and incidence of cancer and invasive cancer **(****FIG. 6C****)** in DKO^{IEC} mice in the Gal experiment. (n: Ctrl =8 and Gal-treated = 6). **FIG. 6D** exemplifies H&E, Masson's trichrome, phospho-Smad2, and CD45 and PD-L1 double staining in small intestinal tumors from DKO^{IEC} mice in the Gal experiment. Scale bars = 100 µm. **FIG. 6E** exemplifies qRT-PCR analyses of Angptl2, Cdkn2b, 1111, and Ereg mRNA levels in the Gal experiment (n:WT = 3, DKO^{IEC} Ctrl = 4, and DKO^{IEC} Gal-treated = 5). **FIG 6F****-****FIG. 6J** exemplifies flow cytometry analyses of immune cell populations in DKO^{IEC} tumors in the Gal experiment. Percentage of IL-17A-producing cells in CD4+ T cells **(****FIG. 6F****);** percentage of CD8+ T cells **(****FIG. 6G****)** and Foxp3+CD4+ Treg cells **(****FIG. 6H****)** cells in CD45+ cells; ratio of Treg to CD8+ T cells **(****FIG. 6I****);** and percentage of CD11b+, CD11b+Ly6ChighLy6G-, and CD1 1b+Ly6C+Ly6G+ cells in CD45+ cells **(****FIG. 6J****).** (n: Ctrl = 4 and Gal-treated = 3 DKO^{IEC} mice). **FIG. 6K** and **FIG. 6L** exemplify quantification **(****FIG. 6K****)** and images **(****FIG. 6L****)** of CD45/PD-L1 staining in DKO^{IEC} tumors in Gal experiment. Scale bar, 100 µm. Arrows: CD45+/PD-L1+. **FIG. 6M** exemplifies an experimental design for a combination therapy with an αPD-L1 antibody and Gal. **FIG. 6N** and **FIG. 6O** exemplify a quantification of the total number, average size, and load of small-intestinal tumors **(****FIG. 6N****)** and incidence of cancer and invasive cancer **(****FIG. 6O****)** in DKO^{IEC} mice in the combination therapy experiment **(****FIG. 6M****).** (n: Ctrl = 6 and αPD-L1 + Gal-treated = 7 DKO^{IEC} mice). **FIG. 6P** exemplifies H&E and CD8 staining in small-intestinal tumors from DKO^{IEC} mice in the combination therapy experiment **(****FIG.6M****).** Dotted lines outline tumors (FIG. 6T). Scale bars, 100 µm. FIG. 6Q-FIG. 6S exemplify flow cytometry analyses of immune cell populations in DKO^{IEC} tumors in the combination therapy experiment. Percentage of CD8+ T **(****FIG. 6Q****);** Foxp3+CD4+ Treg **(****FIG. 6R****);** and CD11b+, CD11b+Ly6ChighLy6G-, and CD11b+Ly6C+Ly6G+ **(****FIG. 6S****)** cells in CD45+ cells. (n: Ctrl = 5 and αPD-L1+ Gal-treated = 4 DKO^{IEC} mice). Results are shown as mean +/-SEM.

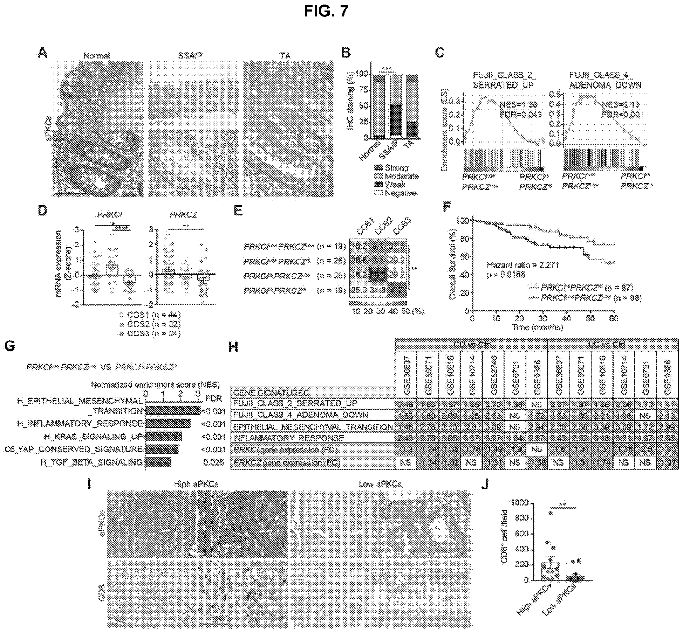

**FIG. 7** exemplifies human serrated tumors have reduced expression of both atypical PKCs. **FIG. 7A** and **FIG. 7** **B** exemplify aPKC immunohistochemistry **(****FIG. 7A****)** and quantification **(****FIG. 7B****)** of human normal colon (n = 20), sessile serrated adenomas/polyps (SSA/P; n = 30), and tubular adenomas (TA; n = 30). Scale bars, 50 µm. **FIG. 7C** exemplifies GSEA plots in CRC patient samples from TCGA stratified based on PRKCI and PRKCZ expression levels. **FIG. 7D** exemplifies PRKCI and PRKCZ mRNA levels in the AMC-AJCCII-90 set (GSE33113) stratified by the CCS classification. **FIG. 7E** exemplifies a proportion of CRC patients with the CCS1, CCS2, or CCS3 CRC subtype according to aPKC mRNA expression (GSE33113). **FIG. 7F** exemplifies Kaplan-Meier overall survival curve of CRC patients (TCGA) stratified based on aPKC expression levels. **FIG. 7G** exemplifies GSEA of transcriptomic data of CRC samples (TCGA) stratified based on aPKC expression levels. **FIG. 7H** exemplifies GSEA of transcriptomic data from the indicated datasets of IBD patients. Normalized enrichment scores (NES) are shown. Fold changes (FC) of expression of PRKCI and PRKCZ comparing Crohn's disease (CD) vs. healthy colon (Ctrl) or ulcerative colitis (UC) vs. Ctrl. FDR <0.1 was considered significant. NS, not significant. **FIG. 7I** and **FIG. 7J** exemplify aPKC and CD8 staining **(****FIG. 7I****)** and quantification **(****FIG. 7J****)** in human proximal CRCs (n: High aPKCs = 12, Low aPKCs = 13). Scale bars, 50 µm. Results are shown as mean +/- SEM.

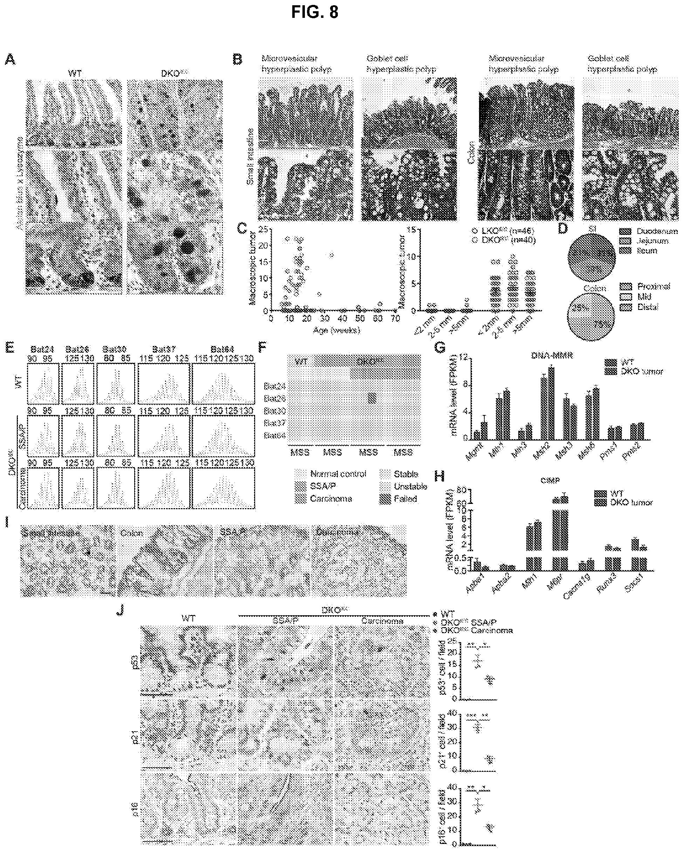

**FIG. 8** exemplifies DKO^{IEC} mice develop intestinal serrated tumors. **FIG. 8A** exemplifies alcian blue and lysozyme staining of sections of small intestine from DKO^{IEC} mice (n = 3). Scale bars, 50 .tm. **FIG. 8B** exemplifies microvesicular hyperplastic polyp and goblet cell hyperplastic polyp in small intestine and colon of DKO^{IEC} mice (n = 6). Scale bars, 100 .tm. **FIG.8C** exemplifies total macroscopic tumors in LKO^{IEC} and DKO^{IEC} small intestine at different ages (left) and fractionated by size (right). Each circle represents one mouse. **FIG. 8D** exemplifies a pie chart of the distribution of tumors in small intestine (n = 39) and colon (n = 27). **FIG. 8E** exemplifies images of electropherograms of five microsatellite markers (Bat24, Bat26, Bat30, Bat37, and Bat64) for normal tissue from WT and SSA/Ps and carcinomas from DKO^{IEC} mice. **FIG. 8F** exemplifies microsatellite instability status of WT and DKO^{IEC} mouse tissues (n = 3-4). Each column represents one sample. Allelic patterns were compared. **(****FIG. 8G** and **FIG. 8H****)** mRNA expression of genes associated with DNA mismatch repair (DNA-MMR, **FIG. 8G****)** and CpG island methylator phenotype (CIMP, **FIG. 8H****)** in WT and DKO^{IEC} tumor tissues from RNA-seq data. FIG. 8I exemplifies β-galactosidase staining in DKO^{IEC} non-tumor and tumor tissues (n = 3). **FIG. 8J** exemplifies p53, p21, and p16 staining and quantification in small-intestinal tissues from WT and DKO^{IEC} tumors (n = 3). Scale bars, 50 .tm. Results are shown as mean ± SEM.

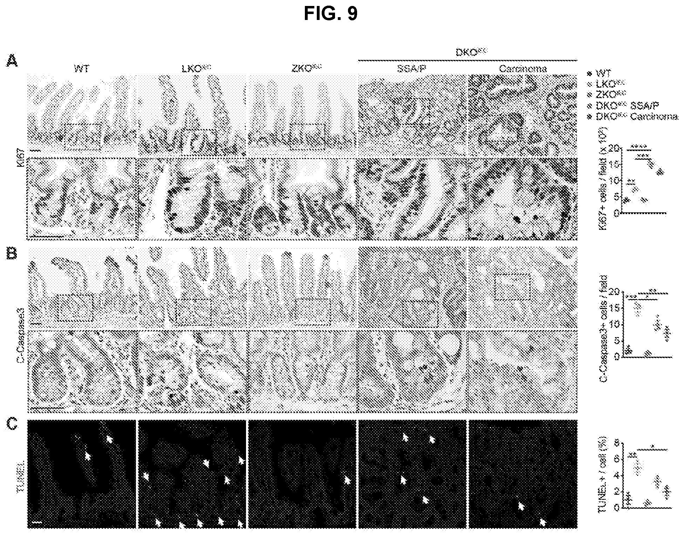

**FIG. 9** exemplifies increased cell proliferation and suppressed cell death in DKO^{IEC} tumors. **FIG. 9A****-****FIG. 9C** exemplifies Ki67 **(****FIG. 9A****),** cleaved caspase 3 (C-Caspase3) **(****FIG. 9B****),** and TUNEL **(****FIG. 9C****)** staining and quantification in small intestines from mice of the indicated genotypes (n = 3). Scale bars, 50 µm. Results are shown as mean ± SEM.

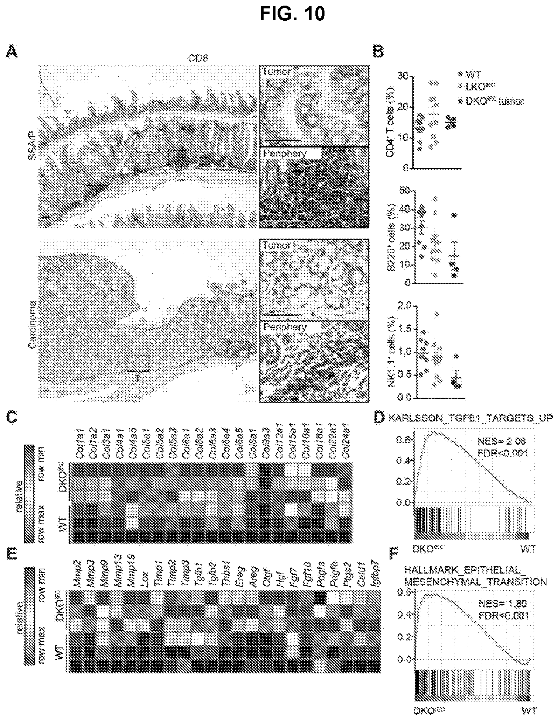

**FIG. 10** exemplifies immunosuppression and stromal activation in DKO^{IEC} tumors. **FIG. 10A** exemplifies CD8 staining in SSA/P and carcinoma from DKOIEC intestines (n = 6). Scale bars, 50 µm. Red lines mark borders between the tumor (T) and periphery (P). **FIG. 10B** exemplifies flow cytometric analyses of immune cell populations from mice of the indicated genotypes. Percentage of CD4+ T, B220+ B, and NK1.1+ NK cells in CD45+ cells. (n: WT = 7, LKO^{IEC} = 8, and DKO^{IEC} tumor = 4). Results are shown as mean ± SEM. **FIG. 10C** exemplifies heatmap of RNA-seq data of WT intestines and DKO^{IEC} tumors representing the differentially expressed genes related to collagen. **FIG. 10D** exemplifies GSEA plot of enrichment of TGF-β targets DKO^{IEC} tumor vs. WT intestine. **FIG. 10E** exemplifies heatmap of RNA-seq data of WT intestines and DKO^{IEC} tumors representing the differentially expressed genes related to stromal activation and stromal-derived factors. **FIG. 10F** exemplifies GSEA plot of enrichment in DKO^{IEC} tumor vs. WT intestine.
**FIG. 11** exemplifies crypt cell proliferation and activation of the YAP/ERK signaling pathway in LKO^{IEC} mice depends on inflammation. **FIG. 11A** and **FIG. 11B** exemplifies H&E, Ki67, phospho-ERK, and YAP staining in small intestine from WT and LKO^{IEC} mice with or without antibiotic (Abx) treatment **(****FIG. 11A****),** and quantification of Ki67 staining **(****FIG. 11B****)** (n = 3). **(****FIG. 11C****)** Flow cytometric analyses of the CD45+ cell population from WT and LKO^{IEC} mice with or without Abx treatment (n = 3). Scale bars = 50 µm. Results are shown as mean ± SEM.

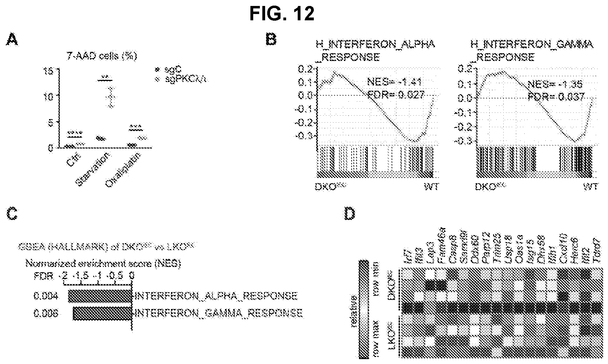

**FIG. 12** exemplifies impaired interferon response in DKO^{IEC} tumors and IECs. **FIG. 12A** exemplifies cell viability (7-AAD+ cells) in sgC and sgPKC 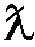 / 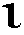 MODE-K cells after HBSS-starvation or treatment with Oxaliplatin (20 µM) 24 hr (n = 3). **FIG. 12B** exemplifies GSEA plots of enrichment in the indicated gene signatures from MSigDB in DKO^{IEC} tumor vs. WT intestine. **FIG. 12C** exemplifies GSEA of transcriptomic data from RNA-seq analysis on DKO^{IEC} vs. LKO^{IEC} IECs. Indicated gene signatures from the Hallmark collection of MSigDB were applied to the analyses. FDR, false discovery rate. **FIG. 12D** exemplifies heatmap of transcriptomic analysis of DKO^{IEC} and LKO^{IEC} IECs representing the differentially expressed genes related to interferon response.

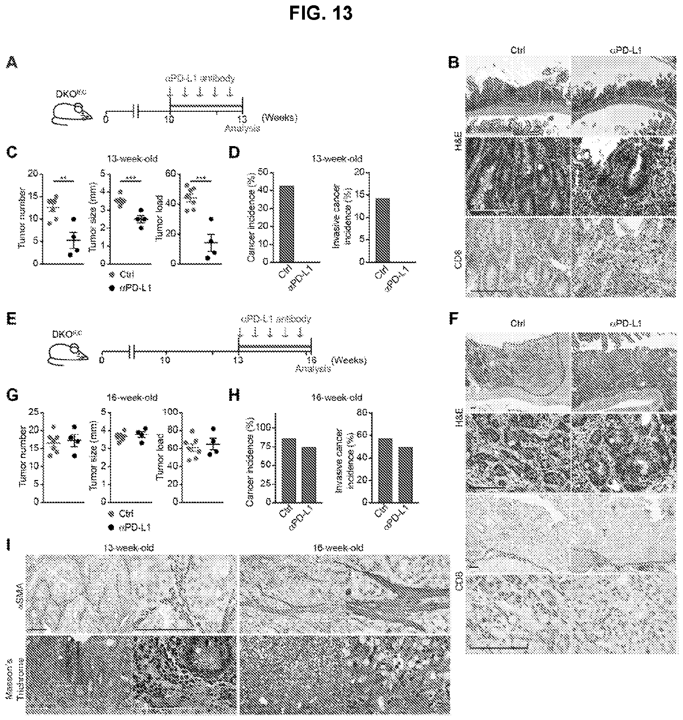

**FIG. 13** exemplifies the impact of anti-PD-L1 therapy on DKO^{IEC} mouse tumors. **FIG. 13A** exemplifies an experimental design for αPD-L1 treatment. **FIG. 13B** exemplifies H&E and CD8 staining in small-intestinal tumors from DKO^{IEC} mice with or without αPD-L1 treatment. Scale bars = 100 µm. **FIG. 13C** and **FIG. 13D** exemplify quantification of the total number, average size, and load of small-intestinal tumors **(****FIG. 13C****)** and incidence of cancer and invasive cancer **(****FIG.13D****)** in DKO^{IEC} mice with or without αPD-L1 treatment (n: Ctrl = 7 and αPD-L1-treated = 4). **FIG. 13E** exemplifies an experimental design for αPD-L1 treatment (n: Ctrl = 7, αPD-L1- treated = 4). **FIG. 13F** exemplifies H&E and CD8 staining in small-intestinal tumors from DKO^{IEC} mice with or without αPD-L1 treatment. **FIG. 13G** and **FIG. 13H** exemplifies quantification of the total number, average size, and load of small-intestinal tumors **(****FIG. 13G****)** and incidence of cancer and invasive cancer **(****FIG. 13H****)** in DKO^{IEC} mice with or without αPD-L1 treatment (n: Ctrl = 7 and αPD-L1-treated = 4). FIG. **13I** exemplifies αSMA and Masson's trichrome staining in small-intestinal tumors from DKO^{IEC} mice (n = 3 per age). Scale bars, 100 µm. Results are shown as mean ± SEM. **p < 0.01, ***p < 0.005.

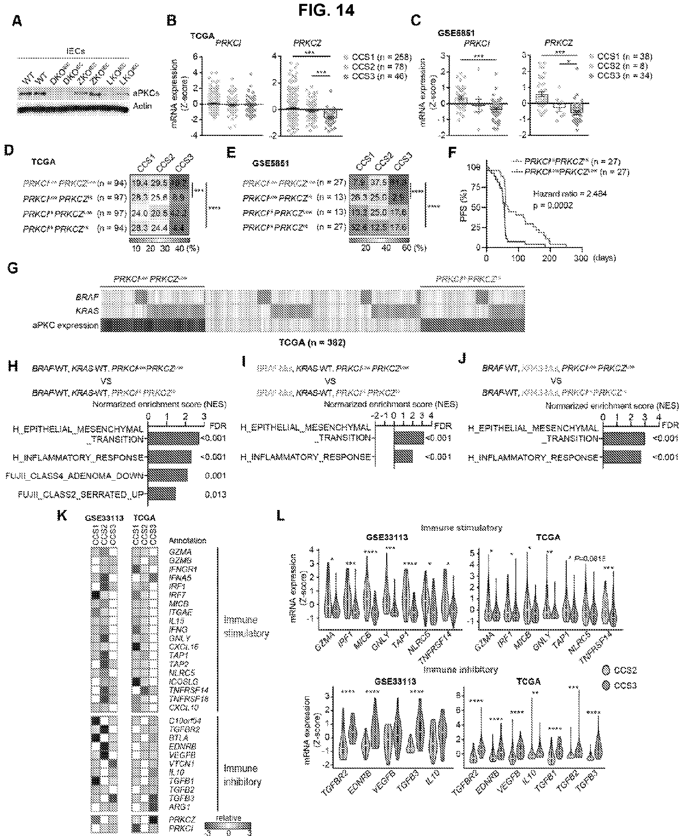

**FIG. 14** exemplifies CRC patients with low expression of both aPKCs demonstrate CCS3 phenotype with immunosuppression and mesenchymal phenotypes. **FIG. 14A** exemplifies immunoblot analysis of aPKCs in isolated IEC samples from indicated genotypes using the antibody recognizing both PKCζ and PKC 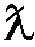 / 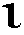 . **FIG. 14B** and **FIG. 14C** exemplify PRKCI and PRKCZ mRNA levels in TCGA **(****FIG. 14B****)** and the Khambata-Ford datasets **(****FIG. 14C****,** GSE5851). **FIG. 14D** and **FIG. 14E** exemplify proportion of CRC patients with the CCS1, CCS2, or CCS3 CRC subtype according to aPKC mRNA expression in **TCGA (****FIG. 14 D)** and in GSE5851 **(****FIG. 14E). FIG. 14F** exemplifies Kaplan-Meier progression-free survival (PFS) curve of CRC patients (GSE5851), stratified based on aPKC expression levels. **FIG. 14G** exemplifies mutation status for BRAF and KRAS and aPKC gene expression profile from the TCGA data. Green and orange rectangles indicate the presence of mutations in BRAF and KRAS, respectively. FDR, false discovery rate. **FIG. 14H****-****FIG. 14J****)** GSEA of transcriptomic data of CRC patients (from TCGA) stratified based on aPKC expression levels and mutation status of BRAF and KRAS: BRAF/KRAS WT **(****FIG. 14H****),** BRAF-mutant **(****FIG. 14I****),** or KRAS-mutant **(****FIG. 14). FIG. 14K** exemplifies heatmap of transcriptomic data (median normalized in each row) from CCS-classified patient samples in GSE33113 and TCGA using the mean expression values for indicated genes. **FIG. 14L** exemplifies violin plots comparing transcriptomic data of the indicated genes in GSE33113 and TCGA between the CCS2 and CCS3 subgroups. Results are shown as mean ± SEM.

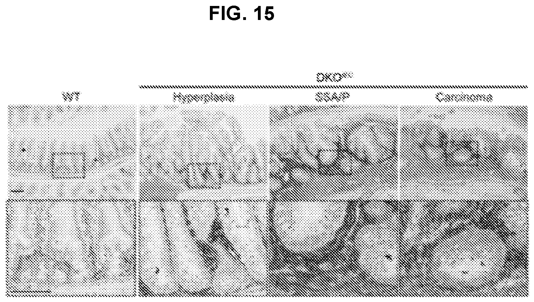

**FIG. 15** exemplifies stroma of aPKC-DKO^{IEC} serrated tumors display increased production of hyaluronan upon disease progression.

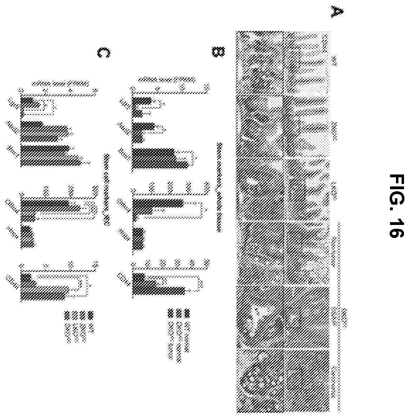

**FIG. 16** exemplifies epithelial tumor cells in aPKC-DKO^{IEC} mice have upregulated expression of CD44, which is a receptor for hyaluronan.

### DETAILED DESCRIPTION OF THE DISCLOSURE

### Definition of Certain Terminology

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviation, per the practice in the given value. The term "about" includes the exact amount. For example, "about 5 µL" means "about 5 µL" and also "5 µL." Where particular values are described in the application and claims, unless otherwise stated the term "about" should be assumed to mean an acceptable error range for the particular value.

The term "optional" or "optionally" denotes that a subsequently described event or circumstance can, but need not occur, and that the description includes instances where the event or circumstance occurs and instances in which it does not.

The term "nucleic acid" as used herein generally refers to one or more nucleobases, nucleosides, or nucleotides. For example, a nucleic acid may include one or more nucleotides selected from adenosine (A), cytosine (C), guanine (G), thymine (T) and uracil (U), or variants thereof. A nucleotide generally includes a nucleoside and at least 1, 2, 3, 4, 5, 6, 7, 8, 9, 10, or more phosphate (PO₃) groups. A nucleotide can include a nucleobase, a five-carbon sugar (either ribose or deoxyribose), and one or more phosphate groups. Ribonucleotides include nucleotides in which the sugar is ribose. Deoxyribonucleotides include nucleotides in which the sugar is deoxyribose. A nucleotide can be a nucleoside monophosphate, nucleoside diphosphate, nucleoside triphosphate or a nucleoside polyphosphate.

As used herein, the terms "polypeptide", "protein" and "peptide" are used interchangeably and refer to a polymer of amino acid residues linked via peptide bonds and which may be composed of two or more polypeptide chains. The terms "polypeptide", "protein" and "peptide" refer to a polymer of at least two amino acid monomers joined together through amide bonds. An amino acid may be the L-optical isomer or the D-optical isomer. More specifically, the terms "polypeptide", "protein" and "peptide" refer to a molecule composed of two or more amino acids in a specific order; for example, the order as determined by the base sequence of nucleotides in the gene or RNA coding for the protein. In some cases, a protein can be a portion of the protein, for example, a domain, a subdomain, or a motif of the protein. In some cases, a protein can be a variant (or mutation) of the protein, wherein one or more amino acid residues are inserted into, deleted from, and/or substituted into the naturally occurring (or at least a known) amino acid sequence of the protein. A protein or a variant thereof can be naturally occurring or recombinant.

As used herein, the term "biological sample" means any biological material from which polynucleotides, polypeptides, biomarkers, and/or metabolites can be prepared and examined. Non-limiting examples encompasses tissue biopsy, whole blood, plasma, saliva, serum, cheek swab, fecal specimen, urine specimen, cell mass, or any other bodily fluid or tissue.

The terms "administer," "administering", "administration," and the like, as used herein, refer to the methods that may be used to enable delivery of compounds or compositions to the desired site of biological action. These methods include, but are not limited to, oral routes (p.o.), intraduodenal routes (i.d.), parenteral injection (including intravenous (i.v.), subcutaneous (s.c.), intraperitoneal (i.p.), intramuscular (i.m.), intravascular or infusion (inf.)), topical (top.) and rectal (p.r.) administration. In some aspects, the compounds and compositions described herein are administered orally.

The terms "co-administration" or the like, as used herein, are meant to encompass administration of the selected therapeutic agents to a single patient, and are intended to include treatment regimens in which the agents are administered at the same or a different time; by the same route or a different route. In some aspects the selected therapeutic agents make up a single composition. In some aspects, the selected therapeutic agents are separate compositions.

The terms "effective amount" or "therapeutically effective amount," as used herein, refer to a sufficient amount of an agent or a compound being administered which will relieve to some extent one or more of the symptoms of the disease or condition being treated; for example a reduction and/or alleviation of one or more signs, symptoms, or causes of a disease, or any other desired alteration of a biological system. For example, an "effective amount" for therapeutic uses can be an amount of an agent that provides a clinically significant decrease in one or more disease symptoms. An appropriate "effective" amount may be determined using techniques, such as a dose escalation study, in individual cases. In some aspects, an "effective amount" for therapeutic uses is the amount of an agent that provides a clinically significant reduction in tumor size, tumor load, a number of tumors; or prevention of tumorigenesis.

The term "subject" or "patient" encompasses mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. In one aspect, the mammal is a human. The term "animal" as used herein comprises human beings and non-human animals. In one aspect, a "non-human animal" is a mammal, for example a rodent such as rat or a mouse.

The terms "treat," "treating" or "treatment," as used herein, include alleviating, abating or ameliorating at least one symptom of a disease or condition, preventing additional symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

The term "preventing" or "prevention" of a disease state denotes causing the clinical symptoms of the disease state not to develop in a subject that can be exposed to or predisposed to the disease state, but does not yet experience or display symptoms of the disease state.

As used herein, the term "comprise" or variations thereof such as "comprises" or "comprising" are to be read to indicate the inclusion of any recited feature but not the exclusion of any other features. Thus, as used herein, the term "comprising" is inclusive and does not exclude additional, unrecited features. In some aspects of any of the compositions and methods provided herein, "comprising" may be replaced with "consisting essentially of" or "consisting of." The phrase "consisting essentially of' is used herein to require the specified feature(s) as well as those which do not materially affect the character or function of the claimed disclosure. As used herein, the term "consisting" is used to indicate the presence of the recited feature alone. The section headings used herein are for organizational purposes only and are not to be construed as limiting the subject matter described.

Provided herein are methods for identifying a subject as having, or being susceptible to developing, serrated colorectal cancer (CRC) based on the detection of biomarkers disclosed herein in a biological sample obtained from the subject. Also provided are methods for treating serrated CRC in a subject with a course of treatment that targets the stromal activation and immunosuppression in the serrate CRC microenvironment. Further provided are models of human serrated CRC, including, but not limited to animal models and tissue models, as well as methods of producing the models disclosed herein. Also provided are methods of using the models disclosed herein to identify modulators of tumorigenesis that may be used as a therapeutic solution to serrated CRC.

### Methods of Diagnosis and Prognosis of Serrated Colorectal Cancer in a Subject

Aspects disclosed herein provide methods of diagnosing, characterizing, and/or prognosing serrated colorectal cancer (CRC) in a subject, provided certain biomarkers are detected in a biological sample obtained from the subject. The biological sample may be obtained directly, or indirectly from the subject. In some aspects, the biological sample comprises a tissue biopsy from the intestine, tumor, or both. In some instances, the tissue biopsy is a needle biopsy, a surgical biopsy or an aspiration biopsy. In some instances the fine needle biopsy comprises a fine need aspiration (FNA). In some aspects, the biological sample comprises whole blood, sera or plasma. In some instances, the subject is a mammal. In some instances, the subject is a mouse, rat, monkey, or rabbit. In some instances, the subject is a human. In some instances, the subject is suffering from, or has symptoms related to, a disease or condition. In some instances, the disease or condition comprises an inflammatory disease. Non-limiting examples of inflammatory disease include inflammatory bowel disease, Crohn's disease, ulcerative colitis, colitis, asthma, intestinal fibrosis, intestinal fibrostenotic disease, arthritic disease, autoimmune disease, and hepatitis. In some instances, the disease or condition comprises a cancer of the intestine. Non-limiting examples of cancer of the intestine include adeonocarcinoma, sarcoma, leiomyosarcoma, carcinoid tumors, gastrointestinal stromal tumor, and lymphoma.

### Biomarkers used in the Diagnosis and Prognosis of Serrated Colorectal Cancer

Atypical protein kinase C (aPKC) ζ and 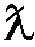 / 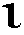 belong to a subfamily of PKCs. PKC 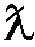 / 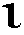 is encoded by the gene, *PRKCI* (Entrez Gene ID No. 5584). PKCζ is encoded by the gene, *PRKCZ* (Entrez Gene ID No. 5590). The disclosure herein provides that simultaneous deletions of PKCζ and PCK 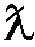 / 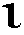 in intestinal epithelium cells results in the development of sessile serrated adenoma/polyps (SSA/P) that progress to advanced serrated colorectal cancer (CRC) with a strongly reactive and immunosuppressive stroma. The disclosure herein further provides that increased expression of hyaluronan (HA) and its receptor, CD44 - a growth factor implicated in CRC tumorigenesis, in intestinal epithelial cells (IECs) of MSS/serrated CRC tumors. The present disclosure provides that PKCζ, PCK 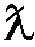 / 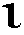 , HA and/or CD44 may be useful biomarkers for identifying subjects suffering from, or who will develop, MSS/serrated CRC. Subjects with serrated CRC lacking PKCζ and PCK 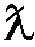 / 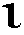 , and increased expression of HA and/or CD44, are resistant to many immunotherapy-based strategies. Thus, the biomarkers disclosed herein are useful to identify subjects suffering from, or susceptible to developing, serrated CRC, and enable the discovery and improvement of alternative therapeutic solutions.

### Methods of Diagnosing a Subject with Serrated CRC

Aspects disclosed herein provide methods of diagnosing a subtype of colorectal cancer (CRC) in a subject in need thereof, the method comprising: a) obtaining a biological sample from a subject in need thereof; b) subjecting the biological sample to an assay suitable to detect levels of expression of PKCζ and PCK 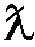 / 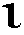 ; and c) diagnosing the subject with serrated colorectal cancer (CRC), provided the levels of expression of PKCζ and PCK 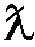 / 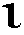 detected in the biological sample obtained from the subject are low, as compared to the levels of expression of PKCζ and PCK 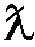 / 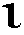 in an individual that does not have the CRC. The levels of expression of PKCζ and PCK 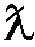 / 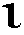 may be detected by measuring the expression of the genes *PKCZ* and *PKCI,* or gene products expressed from the genes *PKCZ* and *PKCI.* In some aspects, PKCζ and PCK 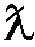 / 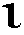 expression comprises ribonucleic acid (RNA) expression. In some aspects, PKCζ and PCK 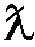 / 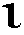 expression comprises protein expression. In some aspects, PKCζ and PCK 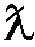 / 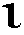 expression comprises deoxyribonucleic acid (DNA) expression. A "low" level of expression of PKCζ and PCK 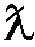 / 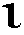 is a statistically significant amount of expression below the level of expression in a normal, or non-diseased, individual. In some aspects, a subject diagnosed with serrated CRC according to the present methods is administered an inhibitor of TGF-β1, PD-L1, hyaluronan, or CD44, or a combination of inhibitors thereof. In some aspects, the levels of expression of PKCζ and PCK 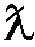 / 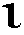 are performed using the methods of detection disclosed herein. In some instances, detecting low levels of expression of PKCζ and PCK 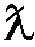 / 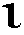 in a biological sample obtained from the subject is indicative that the subject has increased hyaluronan and/or CD44 expression in intestinal epithelial cells (IECs).

Aspects disclosed herein provide methods of diagnosing a subtype of colorectal cancer (CRC) in a subject in need thereof, the method comprising: a) obtaining a biological sample from a subject in need thereof; b) subjecting the biological sample to an assay suitable to detect levels of expression of hyaluronan and/or CD44; and c) diagnosing the subject with serrated colorectal cancer (CRC), provided the levels of expression of hyaluronan and/or CD44 detected in the biological sample obtained from the subject are high, as compared to the levels of expression of hyaluronan and/or CD44 in an individual that does not have the CRC. The levels of expression of hyaluronan and/or CD44 may be detected by measuring the expression of the genes Hyaluronan Synthase 2 (*HAS2*) and/or *CD44,* or gene products expressed from the genes *HAS2 and CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject diagnosed with serrated CRC according to the present methods is administered an inhibitor of TGF-β1, PD-L1, hyaluronan, or CD44, or a combination of inhibitors thereof. In some aspects, the levels of expression of hyaluronan and/or CD44 are performed using the methods of detection disclosed herein.

Aspects disclosed herein provide methods of diagnosing a subtype of colorectal cancer (CRC) in a subject in need thereof, the method comprising: a) obtaining a biological sample from a subject in need thereof; b) subjecting the biological sample to an assay suitable to detect levels of expression of PKCζ and PCK 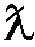 / 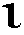 ; c) subjecting the biological sample to an assay suitable to detect levels of expression of hyaluronan and/or CD44; and d) diagnosing the subject with serrated colorectal cancer (CRC), provided the levels of expression of PKCζ and PCK 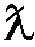 / 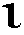 detected in the biological sample obtained from the subject are low, as compared to the levels of expression of PKCζ and PCK 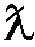 / 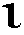 in an individual that does not have the CRC, and provided the levels of expression of hyaluronan and/or CD44 detected in the biological sample obtained from the subject are high, as compared to the levels of expression of hyaluronan and/or CD44 in an individual that does not have the CRC. The levels of expression of PKCζ and PCK 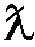 / 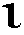 may be detected by measuring the expression of the genes *PKCZ* and *PKCI,* or gene products expressed from the genes *PKCZ* and *PKCI.* In some aspects, PKCζ and PCK 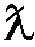 / 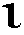 expression comprises RNA expression. In some aspects, PKCζ and PCK 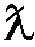 / 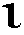 expression comprises protein expression. In some aspects, PKCζ and PCK 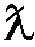 / 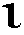 expression comprises DNA expression. A "low" level of expression of PKCζ and PCK 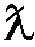 / 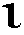 is a statistically significant amount of expression below the level of expression in a normal, or non-diseased, individual. The levels of expression of hyaluronan and/or CD44 may be detected by measuring the expression of the genes *HAS2* and/or *CD44,* or gene products expressed from the genes *HAS2 and CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject diagnosed with serrated CRC according to the present methods is administered an inhibitor of TGF-β1, PD-L1, hyaluronan, or CD44, or a combination of inhibitors thereof. In some aspects, the levels of expression of PKCζ, PCK 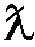 / 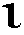 , hyaluronan, and CD44 are performed using the methods of detection disclosed herein.

### Characterizing a Subtype of Colorectal Cancer

Aspects disclosed herein provide methods of characterizing a subtype of colorectal cancer (CRC) in a subject, the method comprising: a) obtaining a biological sample from a subject; b) subjecting the biological sample to an assay suitable to detect levels of expression of PKCζ and PCK 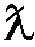 / 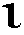 ; and c) characterizing the CRC in the subject as serrated CRC, provided the levels of expression of PKCζ and PCK 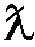 / 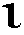 detected in the biological sample obtained from the subject are low, as compared to the levels of expression of PKCζ and PCK 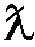 / 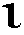 in an individual that does not have the CRC. The levels of expression of PKCζ and PCK 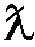 / 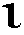 may be detected by measuring the expression of the genes *PKCZ* and *PKCI,* or gene products expressed from the genes *PKCZ* and *PKCI.* In some aspects, PKCζ and PCK 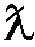 / 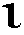 expression comprises RNA expression. In some aspects, PKCζ and PCK 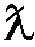 / 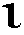 expression comprises protein expression. In some aspects, PKCζ and PCK 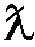 / 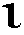 expression comprises DNA expression. A "low" level of expression of PKCζ and PCK 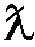 / 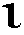 is a statistically significant amount of expression below the level of expression in a normal, or non-diseased, individual. In some aspects, a subject with the CRC characterized as serrated CRC according to the present methods is administered an inhibitor of TGF-β1, PD-L1, hyaluronan, or CD44, or a combination of inhibitors thereof. In some aspects, the levels of expression of PKCζ and PCK 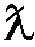 / 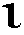 are performed using the methods of detection disclosed herein. In some instances, detecting low levels of expression of PKCζ and PCK 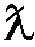 / 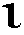 in a biological sample obtained from the subject is indicative that the subject has increased hyaluronan and/or CD44 expression in intestinal epithelial cells (IECs).

Aspects disclosed herein provide methods of characterizing a subtype of colorectal cancer (CRC) in a subject in need thereof, the method comprising: a) obtaining a biological sample from a subject in need thereof; b) subjecting the biological sample to an assay suitable to detect levels of expression of hyaluronan and/or CD44; and c) characterizing the CRC as serrated colorectal cancer (CRC), provided the levels of expression of hyaluronan and/or CD44 detected in the biological sample obtained from the subject are high, as compared to the levels of expression of hyaluronan and/or CD44 in an individual that does not have the CRC. The levels of expression of hyaluronan and/or CD44 may be detected by measuring the expression of the genes *HAS2* and/or *CD44,* or gene products expressed from the genes *HAS2 and CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject with the CRC characterized as serrated CRC according to the present methods is administered an inhibitor of TGF-β1, PD-L1, hyaluronan, or CD44, or a combination of inhibitors thereof. In some aspects, the levels of expression of hyaluronan and/or CD44 are performed using the methods of detection disclosed herein.

Aspects disclosed herein provide methods of characterizing a subtype of colorectal cancer (CRC) in a subject, the method comprising: a) obtaining a biological sample from a subject in need thereof; b) subjecting the biological sample to an assay suitable to detect levels of expression of PKCζ and PCK 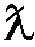 / 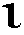 ; c) subjecting the biological sample to an assay suitable to detect levels of expression of hyaluronan and/or CD44; and d) characterizing a subtype of CRC as being serrated CRC, provided the levels of expression of PKCζ and PCK 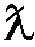 / 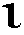 detected in the biological sample obtained from the subject are low, as compared to the levels of expression of PKCζ and PCK 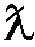 / 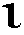 in an individual that does not have the CRC, and provided the levels of expression of hyaluronan and/or CD44 detected in the biological sample obtained from the subject are high, as compared to the levels of expression of hyaluronan and/or CD44 in an individual that does not have the CRC. The levels of expression of PKCζ and PCK 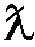 / 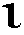 may be detected by measuring the expression of the genes *PKCZ* and *PKCI,* or gene products expressed from the genes *PKCZ* and *PKCI.* In some aspects, PKCζ and PCK 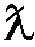 / 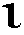 expression comprises RNA expression. In some aspects, PKCζ and PCK 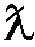 / 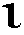 expression comprises protein expression. In some aspects, PKCζ and PCK 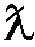 / 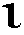 expression comprises DNA expression. A "low" level of expression of PKCζ and PCK 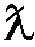 / 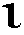 is a statistically significant amount of expression below the level of expression in a normal, or non-diseased, individual. The levels of expression of hyaluronan and/or CD44 may be detected by measuring the expression of the genes *HAS2* and/or *CD44,* or gene products expressed from the genes *HAS2 and CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject with the CRC characterized as serrated CRC according to the present methods is administered an inhibitor of TGF-β1, PD-L1, hyaluronan, or CD44, or a combination of inhibitors thereof. In some aspects, the levels of expression of PKCζ, PCK 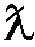 / 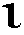 , hyaluronan, and CD44 are performed using the methods of detection disclosed herein.

### Determining whether a Subject Has or Will Develop Serrated Colorectal Cancer

Aspects disclosed herein provide methods of determining whether a subject has, or will develop, serrated colorectal cancer (CRC), the method comprising: a) obtaining a biological sample from a subject in need thereof; b) subjecting the biological sample to an assay suitable to detect levels of expression of PKCζ and PCK 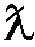 / 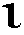 ; and c) determining that the subject has, or will develop, serrated CRC, provided the levels of expression of PKCζ and PCK 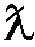 / 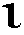 detected in the biological sample obtained from the subject are low, as compared to the levels of expression of PKCζ and PCK 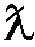 / 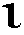 in an individual that does not have the CRC. The levels of expression of PKCζ and PCK 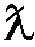 / 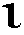 may be detected by measuring the expression of the genes *PKCZ* and *PKCI,* or gene products expressed from the genes *PKCZ* and *PKCI.* In some aspects, PKCζ and PCK 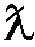 / 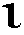 expression comprises ribonucleic acid (RNA) expression. In some aspects, PKCζ and PCK 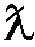 / 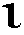 expression comprises protein expression. In some aspects, PKCζ and PCK 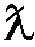 / 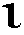 expression comprises deoxyribonucleic acid (DNA) expression. A "low" level of expression of PKCζ and PCK 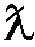 / 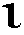 is a statistically significant amount of expression below the level of expression in a normal, or non-diseased, individual. In some aspects, a subject determined to have, or is predicted to develop, serrated CRC according to the present methods is administered an inhibitor of TGF-β1, PD-L1, hyaluronan, or CD44, or a combination of inhibitors thereof. In some aspects, the levels of expression of PKCζ and PCK 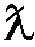 / 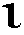 are performed using the methods of detection disclosed herein. In some instances, detecting low levels of expression of PKCζ and PCK 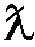 / 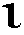 in a biological sample obtained from the subject is indicative that the subject has increased hyaluronan and/or CD44 expression in intestinal epithelial cells (IECs).

Aspects disclosed herein provide methods of determining whether a subject has, or will develop, serrated colorectal cancer (CRC), the method comprising: a) obtaining a biological sample from a subject in need thereof; b) subjecting the biological sample to an assay suitable to detect levels of expression of hyaluronan and/or CD44; and c) determining that the subject has, or will develop, serrated CRC, provided the levels of expression of hyaluronan and/or CD44 detected in the biological sample obtained from the subject are high, as compared to the levels of expression of hyaluronan and/or CD44 in an individual that does not have the CRC. The levels of expression of hyaluronan and/or CD44 may be detected by measuring the expression of the genes Hyaluronan Synthase 2 *(HAS2)* and/or *CD44,* or gene products expressed from the genes *HAS2 and CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject determined to have, or is predicted to develop, serrated CRC according to the present methods is administered an inhibitor of TGF-β1, PD-L1, hyaluronan, or CD44, or a combination of inhibitors thereof. In some aspects, the levels of expression of hyaluronan and/or CD44 are performed using the methods of detection disclosed herein.

Aspects disclosed herein provide methods of determining whether a subject has, or will develop, serrated CRC in a subject, the method comprising: a) obtaining a biological sample from a subject in need thereof; b) subjecting the biological sample to an assay suitable to detect levels of expression of PKCζ and PCK 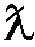 / 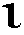 ; c) subjecting the biological sample to an assay suitable to detect levels of expression of hyaluronan and/or CD44; and d) determining that the subject has, or will develop, serrated CRC, provided the levels of expression of PKCζ and PCK 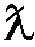 / 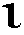 detected in the biological sample obtained from the subject are low, as compared to the levels of expression of PKCζ and PCK 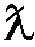 / 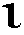 in an individual that does not have the CRC, and provided the levels of expression of hyaluronan and/or CD44 detected in the biological sample obtained from the subject are high, as compared to the levels of expression of hyaluronan and/or CD44 in an individual that does not have the CRC. The levels of expression of PKCζ and PCK 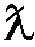 / 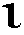 may be detected by measuring the expression of the genes *PKCZ* and *PKCI,* or gene products expressed from the genes *PKCZ* and *PKCI.* In some aspects, PKCζ and PCK 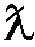 / 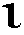 expression comprises RNA expression. In some aspects, PKCζ and PCK 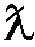 / 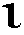 expression comprises protein expression. In some aspects, PKCζ and PCK 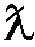 / 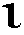 expression comprises DNA expression. A "low" level of expression of PKCζ and PCK 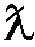 / 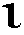 is a statistically significant amount of expression below the level of expression in a normal, or non-diseased, individual. The levels of expression of hyaluronan and/or CD44 may be detected by measuring the expression of the genes *HAS2* and/or *CD44,* or gene products expressed from the genes *HAS2* and *CD44.* In some aspects, hyaluronan and CD44 expression comprises RNA expression. In some aspects, hyaluronan and CD44 expression comprises protein expression. In some aspects, hyaluronan and CD44 expression comprises DNA expression. A "high" level of expression of hyaluronan and/or CD44is a statistically significant amount of expression above the level of expression in a normal, or non-diseased, individual. In some aspects, a subject determined to have, or is predicted to develop, serrated CRC according to the present methods is administered an inhibitor of TGF-β1, PD-L1, hyaluronan, or CD44, or a combination of inhibitors thereof. In some aspects, the levels of expression of PKCζ, PCK 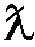 / 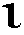 , hyaluronan, and CD44 are performed using the methods of detection disclosed herein.

Aspects disclosed herein provide methods of evaluating a biological sample obtained from the subject for the presence, absence, and/or quantity of a nucleic acid sequence from a gene comprising *HAS2, CD44, PKCZ* and/or *PKCI,* or gene product expressed from the gene comprising *HAS2, CD44, PKCZ* and/or *PKCI.* In some cases, the nucleic acid sequence comprises deoxyribonucleic acid (DNA). In some instances, the nucleic acid sequence comprises a denatured DNA molecule or fragment thereof. In some instances, the nucleic acid sequence comprises DNA selected from: genomic DNA, viral DNA, mitochondrial DNA, plasmid DNA, amplified DNA, circular DNA, circulating DNA, cell-free DNA, or exosomal DNA. In some instances, the DNA is single-stranded DNA (ssDNA), double-stranded DNA, denaturing double-stranded DNA, synthetic DNA, and combinations thereof. The circular DNA may be cleaved or fragmented. In some instances, the nucleic acid sequence comprises ribonucleic acid (RNA). In some instances, the nucleic acid sequence comprises fragmented RNA. In some instances, the nucleic acid sequence comprises partially degraded RNA. In some instances, the nucleic acid sequence comprises a microRNA or portion thereof. In some instances, the nucleic acid sequence comprises an RNA molecule or a fragmented RNA molecule (RNA fragments) selected from: a microRNA (miRNA), a pre-miRNA, a pri-miRNA, a mRNA, a pre-mRNA, a viral RNA, a viroid RNA, a virusoid RNA, circular RNA (circRNA), a ribosomal RNA (rRNA), a transfer RNA (tRNA), a pre-tRNA, a long non-coding RNA (lncRNA), a small nuclear RNA (snRNA), a circulating RNA, a cell-free RNA, an exosomal RNA, a vector-expressed RNA, an RNA transcript, a synthetic RNA, and combinations thereof.

Disclosed herein, in some aspects, are nucleic acid-based detection assays useful for the detection of a presence, absence, and/or quantity of a nucleic acid sequence from a gene comprising *HAS2, CD44, PKCZ* and/or *PKCI,* or gene product expressed from the gene comprising *HAS2, CD44, PKCZ* and/or *PKCI.* In some instances, the nucleic acid-based detection assay comprises quantitative polymerase chain reaction (qPCR), gel electrophoresis (including for *e.g.,* Northern or Southern blot), immunochemistry, *in situ* hybridization such as fluorescent *in situ* hybridization (FISH), cytochemistry, or sequencing. In some aspects, the sequencing technique comprises next generation sequencing. In some aspects, the methods involve a hybridization assay such as fluorogenic qPCR (e.g., TaqMan^{™} or SYBR green), which involves a nucleic acid amplification reaction with a specific primer pair, and hybridization of the amplified nucleic acid probes comprising a detectable moiety or molecule that is specific to a target nucleic acid sequence. An additional exemplary nucleic acid-based detection assay comprises the use of nucleic acid probes conjugated or otherwise immobilized on a bead, multi-well plate, or other substrate, wherein the nucleic acid probes are configured to hybridize with a target nucleic acid sequence. In some instances, the nucleic acid probe is specific to one or more genes disclosed herein is used. In some instances, the nucleic acid probe is specific to *HAS2, CD44, PKCZ* and/or *PKCI,* or gene product expressed from the gene comprising *HAS2, CD44, PKCZ* and/or *PKCI.*

Disclosed herein, in some aspects, are methods of detecting a gene of an individual by subject a sample obtained from the individual to a nucleic acid amplification assay. In some instances, the amplification assay comprises polymerase chain reaction (PCR), qPCR, self-sustained sequence replication, transcriptional amplification system, Q-Beta Replicase, rolling circle replication, or any suitable other nucleic acid amplification technique. A suitable nucleic acid amplification technique is configured to amplify a region of a nucleic acid sequence comprising one or more genes, or gene expression products thereof, disclosed herein. In some instances, the amplification assays requires primers. The nucleic acid sequence for the gene, or gene expression products thereof, known or provided herein is sufficient to enable one of skill in the art to select primers to amplify any portion of the gene or genetic variants. A DNA sample suitable as a primer may be obtained, e.g., by polymerase chain reaction (PCR) amplification of genomic DNA, fragments of genomic DNA, fragments of genomic DNA ligated to adaptor sequences or cloned sequences. A person of skill in the art would utilize computer programs to design of primers with the desired specificity and optimal amplification properties, such as Oligo version 7.0 (National Biosciences). It will be apparent to one skilled in the art that controlled robotic systems are useful for isolating and amplifying nucleic acids and can be used.

In some aspects, detecting the presence or absence and/or quantity of a gene comprising *HAS2, CD44, PKCZ* and/or *PKCI,* or gene expression produced expressed from the gene *HAS2, CD44, PKCZ* and/or *PKCI,* comprises sequencing genetic material obtained from a biological sample from the subject. Sequencing can be performed with any appropriate sequencing technology, including but not limited to single-molecule real-time (SMRT) sequencing, Polony sequencing, sequencing by ligation, reversible terminator sequencing, proton detection sequencing, ion semiconductor sequencing, nanopore sequencing, electronic sequencing, pyrosequencing, Maxam-Gilbert sequencing, chain termination (e.g., Sanger) sequencing, +S sequencing, or sequencing by synthesis. Sequencing methods also include next-generation sequencing, e.g., modern sequencing technologies such as Illumina sequencing (e.g., Solexa), Roche 454 sequencing, Ion torrent sequencing, and SOLiD sequencing. In some cases, next-generation sequencing involves high-throughput sequencing methods. Additional sequencing methods available to one of skill in the art may also be employed.

In some instances, a number of nucleotides that are sequenced are at least 5, 10, 15, 20, 25, 30, 35, 40, 45, 50, 100, 150, 200, 300, 400, 500, 2000, 4000, 6000, 8000, 10000, 20000, 50000, 100000, or more than 100000 nucleotides. In some instances, the number of nucleotides sequenced is in a range of about 1 to about 100000 nucleotides, about 1 to about 10000 nucleotides, about 1 to about 1000 nucleotides, about 1 to about 500 nucleotides, about 1 to about 300 nucleotides, about 1 to about 200 nucleotides, about 1 to about 100 nucleotides, about 5 to about 100000 nucleotides, about 5 to about 10000 nucleotides, about 5 to about 1000 nucleotides, about 5 to about 500 nucleotides, about 5 to about 300 nucleotides, about 5 to about 200 nucleotides, about 5 to about 100 nucleotides, about 10 to about 100000 nucleotides, about 10 to about 10000 nucleotides, about 10 to about 1000 nucleotides, about 10 to about 500 nucleotides, about 10 to about 300 nucleotides, about 10 to about 200 nucleotides, about 10 to about 100 nucleotides, about 20 to about 100000 nucleotides, about 20 to about 10000 nucleotides, about 20 to about 1000 nucleotides, about 20 to about 500 nucleotides, about 20 to about 300 nucleotides, about 20 to about 200 nucleotides, about 20 to about 100 nucleotides, about 30 to about 100000 nucleotides, about 30 to about 10000 nucleotides, about 30 to about 1000 nucleotides, about 30 to about 500 nucleotides, about 30 to about 300 nucleotides, about 30 to about 200 nucleotides, about 30 to about 100 nucleotides, about 50 to about 100000 nucleotides, about 50 to about 10000 nucleotides, about 50 to about 1000 nucleotides, about 50 to about 500 nucleotides, about 50 to about 300 nucleotides, about 50 to about 200 nucleotides, or about 50 to about 100 nucleotides.

In some aspects, detecting the presence or absence and/or quantity of a gene comprising *HAS2, CD44, PKCZ* and/or *PKCI,* or gene expression produced expressed from the gene comprising *HAS2, CD44, PKCZ* and/or *PKCI,* comprises hybridizing a probe or reporting sequence to a target nucleic acid described herein. Examples of molecules that are utilized as probes include, but are not limited to, RNA and DNA. In some aspects, the term "probe" with regards to nucleic acids, refers to any molecule that is capable of selectively binding to a specifically intended target nucleic acid sequence. In some instances, probes are specifically designed to be labeled, for example, with a radioactive label, a fluorescent label, an enzyme, a chemiluminescent tag, a colorimetric tag, or other labels or tags that are known in the art. In some instances, the fluorescent label comprises a fluorophore. In some instances, the fluorophore is an aromatic or heteroaromatic compound. In some instances, the fluorophore is a pyrene, anthracene, naphthalene, acridine, stilbene, benzoxaazole, indole, benzindole, oxazole, thiazole, benzothiazole, canine, carbocyanine, salicylate, anthranilate, xanthenes dye, coumarin. Exemplary xanthene dyes include, e.g., fluorescein and rhodamine dyes. Fluorescein and rhodamine dyes include, but are not limited to 6-carboxyfluorescein (FAM), 2'7'-dimethoxy-4'5'-dichloro-6-carboxyfluorescein (JOE), tetrachlorofluorescein (TET), 6-carboxyrhodamine (R6G), N,N,N; N'-tetramethyl-6-carboxyrhodamine (TAMRA), 6-carboxy-X-rhodamine (ROX). Suitable fluorescent probes also include the naphthylamine dyes that have an amino group in the alpha or beta position. For example, naphthylamino compounds include 1-dimethylaminonaphthyl-5-sulfonate, 1-anilino-8-naphthalene sulfonate and 2-p-toluidinyl-6-naphthalene sulfonate, 5-(2'-aminoethyl)aminonaphthalene-1-sulfonic acid (EDANS). Exemplary coumarins include, e.g., 3-phenyl-7-isocyanatocoumarin; acridines, such as 9-isothiocyanatoacridine and acridine orange; N-(p-(2-benzoxazolyl)phenyl) maleimide; cyanines, such as, e.g., indodicarbocyanine 3 (Cy3), indodicarbocyanine 5 (Cy5), indodicarbocyanine 5.5 (Cy5.5), 3-(-carboxy-pentyl)-3'-ethyl-5,5'-dimethyloxacarbocyanine (CyA); 1H, 5H, 11H, 15H-Xantheno[2,3, 4-ij: 5,6, 7-i'j']diquinolizin-18-ium, 9-[2 (or 4)-[[[6-[2,5-dioxo-1-pyrrolidinyl)oxy]-6-oxohexyl]amino]sulfonyl]-4 (or 2)-sulfophenyl]-2,3, 6,7, 12,13, 16,17-octahydro-inner salt (TR or Texas Red); or BODIPYTM dyes. In some cases, the probe comprises FAM as the dye label.

In some instances, primers and/or probes described herein for detecting a target nucleic acid are used in an amplification reaction. In some instances, the amplification reaction is qPCR. An exemplary qPCR is a method employing a TaqMan^{™} assay.

In some instances, qPCR comprises using an intercalating dye. Examples of intercalating dyes include SYBR green I, SYBR green II, SYBR gold, ethidium bromide, methylene blue, Pyronin Y, DAPI, acridine orange, Blue View or phycoerythrin. In some instances, the intercalating dye is SYBR.

In some instances, a number of amplification cycles for detecting a target nucleic acid in an amplification assay is about 5 to about 30 cycles. In some instances, the number of amplification cycles for detecting a target nucleic acid is at least about 5 cycles. In some instances, the number of amplification cycles for detecting a target nucleic acid is at most about 30 cycles. In some instances, the number of amplification cycles for detecting a target nucleic acid is about 5 to about 10, about 5 to about 15, about 5 to about 20, about 5 to about 25, about 5 to about 30, about 10 to about 15, about 10 to about 20, about 10 to about 25, about 10 to about 30, about 15 to about 20, about 15 to about 25, about 15 to about 30, about 20 to about 25, about 20 to about 30, or about 25 to about 30 cycles.

In one aspect, the methods provided herein for determining the presence, absence, and/or quantity of a nucleic acid sequence from a particular genotype comprise an amplification reaction such as qPCR. In an exemplary method, genetic material is obtained from a sample of a subject, e.g., a sample of blood or serum. In certain aspects where nucleic acids are extracted, the nucleic acids are extracted using any technique that does not interfere with subsequent analysis. In certain aspects, this technique uses alcohol precipitation using ethanol, methanol or isopropyl alcohol. In certain aspects, this technique uses phenol, chloroform, or any combination thereof. In certain aspects, this technique uses cesium chloride. In certain aspects, this technique uses sodium, potassium or ammonium acetate or any other salt commonly used to precipitate DNA. In certain aspects, this technique utilizes a column or resin based nucleic acid purification scheme such as those commonly sold commercially, one non-limiting example would be the GenElute Bacterial Genomic DNA Kit available from Sigma Aldrich. In certain aspects, after extraction the nucleic acid is stored in water, Tris buffer, or Tris-EDTA buffer before subsequent analysis. In an exemplary aspect, the nucleic acid material is extracted in water. In some cases, extraction does not comprise nucleic acid purification.

In the exemplary qPCR assay, the nucleic acid sample is combined with primers and probes specific for a target nucleic acid that may or may not be present in the sample, and a DNA polymerase. An amplification reaction is performed with a thermal cycler that heats and cools the sample for nucleic acid amplification, and illuminates the sample at a specific wavelength to excite a fluorophore on the probe and detect the emitted fluorescence. For TaqMan^{™} methods, the probe may be a hydrolysable probe comprising a fluorophore and quencher that is hydrolyzed by DNA polymerase when hybridized to a target nucleic acid. In some cases, the presence of a target nucleic acid is determined when the number of amplification cycles to reach a threshold value is less than 30, 29, 28, 27, 26, 25, 24, 23, 22, 21, or 20 cycles.

In some aspects, detecting and quantifying soluble protein levels of hyaluronan, CD44, PKCζ, and/or PCK 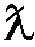 / 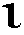 in a subject by detecting and quantifying said levels from a biological sample obtained from the subject are provided. Hyaluronan, CD44, PKCζ, and/or PCK 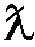 / 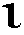 may be detected by use of an antibody-based assay, where an antibody specific to hyaluronan, CD44, PKCζ, and/or PCK 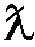 / 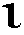 *(e.g.,* anti-hyaluronana, anti-CD44, anti-PKCζ, and/or anti- PCK 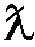 / 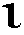 antibodies) is utilized. For antibody-based detection methods, the antibody may bind to any region of hyaluronan, CD44, PKCζ, and/or PCK 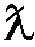 / 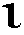 . An exemplary method of analysis comprises performing an enzyme-linked immunosorbent assay (ELISA). The ELISA assay may be a sandwich ELISA or a direct ELISA. Another exemplary method of analysis comprises a single molecule array, e.g., Simoa. Other exemplary methods of detection includes immunohistochemistry and lateral flow assay.

In some cases, hyaluronan and/or CD44 protein may be detected by detecting binding between hyaluronan and/or CD44 and other binding partners of hyaluronan and/or CD44. Methods of analysis of binding between hyaluronan and/or CD44, as well as with other binding partners comprise performing an assay *in vivo* or *in vitro, or ex vivo.* In some instances, the assay may comprise co-immunoprecipitation (co-IP), pull-down, crosslinking protein interaction analysis, labeled transfer protein interaction analysis, or Far-western blot analysis, FRET based assay, including, for example FRET-FLIM, a yeast two-hybrid assay, BiFC, or split luciferase assay.

### Methods of Treating Serrated Colorectal Cancer

The present disclosure provides that treatment of subjects with the microsatellite stable (MSS) serrated colorectal cancer (CRC) with an antagonist to programmed cell death ligand-1 (PD-L1) caused regression in serrated tumors in the initiation/adenoma stage. Whereas, treatment of MSS/serrated CRC subjects with advanced adenocarcinomas characterized by a strongly reactive and immunosuppressive stroma with and antagonist to PD-L1 caused regression in serrated tumors only when co-administration of an inhibitor of transforming growth factor beta 1 (TGF-β1) receptor inhibitor was administered, which serves to sensitize the serrated tumors to anti-PD-L1 treatment. Further disclosed, increased expression of hyaluronan (HA) and its receptor, CD44 - a growth factor implicated in tumorigenesis, in intestinal epithelial cells (IECs) of MSS/serrated CRC tumors, suggests that the interrupting the interaction between HA and CD44 may provide a suitable therapeutic solution for ameliorating tumorigenesis in a subject with MSS/serrated CRC.

Aspects disclosed herein provide methods of treating a disease or a condition, or a subtype of the disease or condition, in a subject by administering to the subject a therapeutically effective amount of one or more of the therapeutic agents disclosed herein. In some instances, the disease or condition comprises colorectal cancer (CRC), pancreatic cancer, inflammatory bowel disease, ulcerative colitis, Crohn's disease, fibrosis, fibrostenosis, or a combination thereof. In some aspects, the subject is a mammal. In some instances, the subtype of the CRC comprises serrated CRC. In some instances, the serrated CRC comprises the MSS subtype of serrated CRC. In some aspects, the subject is human. In some aspects the subject is a mouse, rat, monkey or rabbit.

### Therapeutic agents

Aspects disclosed herein provide methods of treating a a disease or condition, or a subtype of a disease or condition, in a subject by administering to the subject a therapeutically effective amount of a therapeutic agent disclosed herein, provided low levels of expression of PKCζ and PCK 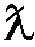 / 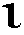 are detected in a biological sample obtained from the subject, as compared to levels of expression of PKCζ and PCK 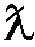 / 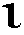 in an individual who does not have the disease or condition. In some aspects, the therapeutic agent is effective to reduce or increase the activity or expression of a therapeutic target. Non-limiting examples of therapeutic targets include PD-L1, PD-1, TGF-β1, TGF-βR1, hyaluronan, CD44, and hyaluronidase. Non-limiting examples of therapeutic agents include agonists and antagonists of the above therapeutic targets.

### Inhibitor of PDL-1 Activity or Expression

Aspects disclosed herein provide methods of treating a disease or condition, or subtype of a disease or condition, in a subject by administering to the subject a therapeutically effective amount of an inhibitor of programmed death-ligand 1 (PD-L1) activity or expression, provided low levels of expression of PKCζ and PCK 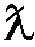 / 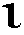 are detected in a biological sample obtained from the subject, as compared to levels of expression of PKCζ and PCK 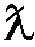 / 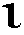 in an individual who does not have the disease or condition. Disclosed herein, in certain aspects, are methods of inhibiting or reducing programmed death-ligand 1 (PD-L1) activity or expression in a subject suffering from a disease or condition, comprising: a) identifying the subject as having low levels of expression of PKCζ and PCK 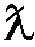 / 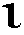 , as compared to the levels of expression of PKCζ and PCK 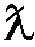 / 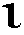 in an individual who does not have the disease or condition; and b) administering to the subject a therapeutically effective amount of an inhibitor of PD-L1 activity or expression. A subject may be identified as having low levels of expression of PKCζ and PCK 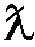 / 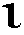 using any of the methods of detection disclosed herein.

In some instances, the inhibitor of PD-L1 activity or expression is an indirect inhibitor. In some instances, the inhibitor of PD-L1 activity or expression is a direct inhibitor. In some instances, the inhibitor of PD-L1 activity or expression comprises an allosteric modulator of PD-L1, or programmed cell death protein 1 (PD-1), or both. In some instances, the inhibitor of PD-L1 activity or expression comprises an antibody, or antibody fragment. In some aspects, the antibody comprises a monoclonal antibody, a chimeric antibody, a CDR-grafted antibody, a humanized antibody, a Fab, a Fab', a F(ab')2, a Fv, a disulfide linked Fv, a scFv, a single domain antibody, a diabody, a multispecific antibody, a dual specific antibody, an anti-idiotypic antibody, a bispecific antibody, or a combination thereof. In some aspects, the antibody or antigen-binding fragment comprises an IgG antibody. In some instances, the antibody or antibody fragment binds to PD-L1. In some instances, the antibody or antibody fragment binds to programmed cell death protein 1 (PD-1). In some instances, the inhibitor of PD-L1 activity of expression comprises a small molecule. In some instances, the small molecule inhibitor of PD-L 1 specifically binds to PD-L1. In some instances, the small molecule inhibitor of PD-L1 specifically binds to PD-1. Non-limiting examples of inhibitors of PD-L1 activity or expression include pembrolizumab, atezolizumab (*e*.*g*., Tecentriq^{®}), avelumab *(e.g.,* Bavencio^{®}), durvalumab (e.g., Imfinzi^{®}), nivolumab *(e.g.,* Opdivo^{®}), eFT508, LY3300054, HMS-936559, CK-301, pidilzumab, AMP-224, AM P-514, PDR001, cemiplimab.

### Inhibitor of TGF-β1 Activity or Expression

Aspects disclosed herein provide methods of treating a disease or condition, or subtype of a disease or condition, in a subject by administering to the subject a therapeutically effective amount of an inhibitor of TGF-β1 activity or expression, provided low levels of expression of PKCζ and PCK 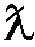 / 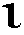 are detected in a biological sample obtained from the subject, as compared to levels of expression of PKCζ and PCK 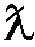 / 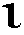 in an individual who does not have the disease or condition. Disclosed herein, in certain aspects, are methods for inhibiting or reducing TGF-β1 activity or expression in a subject suffering from a disease or condition, comprising: a) identifying the subject as having low levels of expression of PKCζ and PCK 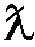 / 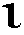 , as compared to the levels of expression of PKCζ and PCK 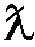 / 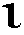 in an individual who does not have the disease or condition; and b) administering to the subject a therapeutically effective amount of an inhibitor of TGF-β1 activity or expression. A subject may be identified as having low levels of expression of PKCζ and PCK 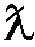 / 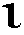 using any of the methods of detection disclosed herein.

In some instances, the inhibitor of TGF-β1 activity or expression is an indirect inhibitor. In some instances, the inhibitor of TGF-β1 activity or expression is a direct inhibitor. In some instances, the inhibitor of TGF-β1 activity or expression comprises an allosteric modulator of TGF-β1, or TGF-β1 receptor (TGF-βR1), or both. In some instances, the inhibitor of TGF-β1 activity or expression comprises an antibody, or antibody fragment. In some aspects, the antibody comprises a monoclonal antibody, a chimeric antibody, a CDR-grafted antibody, a humanized antibody, a Fab, a Fab', a F(ab')2, a Fv, a disulfide linked Fv, a scFv, a single domain antibody, a diabody, a multispecific antibody, a dual specific antibody, an anti-idiotypic antibody, a bispecific antibody, or a combination thereof. In some aspects, the antibody or antigen-binding fragment comprises an IgG antibody. In some instances, the antibody or antibody fragment binds to TGF-β1. In some instances, the antibody or antibody fragment binds to TGF-βR1. In some instances, the inhibitor of TGF-β1 activity of expression comprises a small molecule. In some instances, the small molecule inhibitor of TGF-β1 specifically binds to TGF-β1. In some instances, the small molecule inhibitor of TGF-β1 specifically binds to TGF-βR1. Non-limiting examples of inhibitors of TGF-β1 activity or expression include galunisertib (LY2157299), fresolimumab (GC1008), lucanix (belagenpumatucel-L) LY550410, LY580276, and Trabedersen.

### Inhibitor of Hyaluronan Activity or Expression

Aspects disclosed herein provide methods of treating a disease or condition, or subtype of a disease or condition, in a subject by administering to the subject a therapeutically effective amount of an inhibitor of hyaluronan activity or expression, provided low levels of expression of PKCζ and PCK 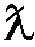 / 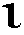 are detected in a biological sample obtained from the subject, as compared to levels of expression of PKCζ and PCK 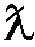 / 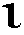 in an individual who does not have the disease or condition. Disclosed herein, in certain aspects, are methods for inhibiting or reducing hyaluronan activity or expression in a subject suffering from a disease or condition, comprising: a) identifying the subject as having low levels of expression of PKCζ and PCK 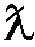 / 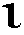 , as compared to the levels of expression of PKCζ and PCK 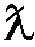 / 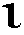 in an individual who does not have the disease or condition; and b) administering to the subject a therapeutically effective amount of an inhibitor of hyaluronan activity or expression. A subject may be identified as having low levels of expression of PKCζ and PCK 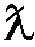 / 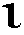 using any of the methods of detection disclosed herein.

In some instances, the inhibitor of hyaluronan activity or expression is an indirect inhibitor. In some instances, the inhibitor of hyaluronan activity or expression is a direct inhibitor. In some instances, the inhibitor of hyaluronan activity or expression comprises an allosteric modulator of hyaluronan or CD44, or both.

In some instances, the inhibitor of hyaluronan activity of expression comprises a hyaluronidase, or an agonist of hyaluronidase activity or expression. A non-limiting example of a hyaluronidase includes Pegvorhyaluronidase alfa (PVHA). In some instances, the inhibitor of hyaluronan activity or expression comprises exogenous hyaluronidase. In some instances, the exogenous hyaluronidase comprises a recombinant hyaluronidase. In some instances, the recombinant hyaluronidase comprises human recombinant hyaluronidase.

In some instances, the inhibitor of hyaluronan activity or expression comprises an antibody, or antibody fragment. In some instances, the antibody or antibody fragment binds to hyaluronan. In some instances the antibody or antibody fragment binds to 70%, 80%, 90%, or 100% of the hyaluronan molecule. In some instances, the antibody or antibody fragment binds to CD44. In some instances, the antibody or antibody fragment inhibits the binding and/or agonism between hyaluronan and CD44.

In some instances, the inhibitor of hyaluronan activity of expression comprises a small molecule. In some instances, the inhibitor of hyaluronan activity of expression comprises a small molecule effective to inhibit binding and/or agonism between hyaluronan and CD44. In some instances, the small molecule inhibitor of hyaluronan specifically binds to hyaluronan. In some instances, the small molecule inhibitor of hyaluronan specifically binds to CD44. Non-limiting examples of inhibitors of hyaluronan activity or expression include 4-methylumbelliferone (4-MU), hyaluronidase PH20 (rHuPH20), recombinant human hyaluronidase, PEGylated recombinant human hyaluronidase.

In some instances, the indirect inhibitor of hyaluronan comprises a direct inhibitor of CD44. In some instances, the direct inhibitor of CD44 comprises an anti-CD44 antibody or antibody fragment. In some instances, the direct inhibitor of CD44 comprises a DNA vaccine. In some instances, the direct inhibitor of CD44 comprises CD44siRNA. In some instances, the indirect inhibitor of hyaluronan comprises a direct inhibitor of CD44. In some instances, the direct inhibitor of CD44 comprises an anti-CD44 conjugated cell therapy. A Non-limiting examples of inhibitors of CD44 activity or expression include AMC303, and RG7356.

### Pharmaceutical Compositions

Pharmaceutical compositions containing an inhibitor of PD-L1 activity or expression, an inhibitor of TGF-β1 activity or expression, and/or an inhibitor of hyaluronan activity or expression, another therapeutic agent, or any combinations thereof, are provided in some aspects of this disclosure. In some aspects, the pharmaceutical composition comprises an inhibitor of PD-L1 activity or expression. In some aspects, the pharmaceutical composition comprises an inhibitor of TGF-β1 activity or expression. In some aspects, the pharmaceutical composition comprises an inhibitor of PD-L1 activity or expression and an inhibitor of TGF-β1 activity or expression. In some aspects, the pharmaceutical composition comprises an inhibitor of PD-L1 activity or expression and an inhibitor of hyaluronan activity or expression. In some aspects, the pharmaceutical composition comprises an inhibitor of TGF-β1 activity or expression and an inhibitor of hyaluronan activity or expression. In some aspects, the pharmaceutical composition comprises an inhibitor of hyaluronan. In some aspects, the pharmaceutical compositions of this disclosure are prepared as solutions, dispersions in glycerol, liquid polyethylene glycols, and any combinations thereof in oils, in solid dosage forms, as inhalable dosage forms, as intranasal dosage forms, as liposomal formulations, dosage forms comprising nanoparticles, dosage forms comprising microparticles, polymeric dosage forms, or any combinations thereof. In some aspects, a pharmaceutical composition as described herein comprises an excipient. An excipient is, in some examples, an excipient described in the Handbook of Pharmaceutical Excipients, American Pharmaceutical Association (1986). Non-limiting examples of suitable excipients include a buffering agent, a preservative, a stabilizer, a binder, a compaction agent, a lubricant, a chelator, a dispersion enhancer, a disintegration agent, a flavoring agent, a sweetener, a coloring agent.

In some aspects an excipient is a buffering agent. Non-limiting examples of suitable buffering agents include histidine, sodium citrate, magnesium carbonate, magnesium bicarbonate, calcium carbonate, and calcium bicarbonate. As a buffering agent, histidine, sodium bicarbonate, potassium bicarbonate, magnesium hydroxide, magnesium lactate, magnesium glucomate, aluminium hydroxide, sodium citrate, sodium tartrate, sodium acetate, sodium carbonate, sodium polyphosphate, potassium polyphosphate, sodium pyrophosphate, potassium pyrophosphate, disodium hydrogen phosphate, dipotassium hydrogen phosphate, trisodium phosphate, tripotassium phosphate, potassium metaphosphate, magnesium oxide, magnesium hydroxide, magnesium carbonate, magnesium silicate, calcium acetate, calcium glycerophosphate, calcium chloride, calcium hydroxide and other calcium salts or combinations thereof is used, in some aspects, in a pharmaceutical composition of the present disclosure.

In some aspects an excipient comprises a preservative. Non-limiting examples of suitable preservatives include antioxidants, such as alpha-tocopherol and ascorbate, and antimicrobials, such as parabens, chlorobutanol, and phenol. In some examples, antioxidants further include but are not limited to EDTA, citric acid, ascorbic acid, butylated hydroxytoluene (BHT), butylated hydroxy anisole (BHA), sodium sulfite, p-amino benzoic acid, glutathione, propyl gallate, cysteine, methionine, ethanol and N- acetyl cysteine. In some instances preservatives include validamycin A, TL-3, sodium ortho vanadate, sodium fluoride, N-a-tosyl-Phe- chloromethylketone, N-a-tosyl-Lys-chloromethylketone, aprotinin, phenylmethylsulfonyl fluoride, diisopropylfluorophosphate, kinase inhibitor, phosphatase inhibitor, caspase inhibitor, granzyme inhibitor, cell adhesion inhibitor, cell division inhibitor, cell cycle inhibitor, lipid signaling inhibitor, protease inhibitor, reducing agent, alkylating agent, antimicrobial agent, oxidase inhibitor, or other inhibitor.

In some aspects a pharmaceutical composition as described herein comprises a binder as an excipient. Non-limiting examples of suitable binders include starches, pregelatinized starches, gelatin, polyvinylpyrolidone, cellulose, methylcellulose, sodium carboxymethylcellulose, ethylcellulose, polyacrylamides, polyvinyloxoazolidone, polyvinylalcohols, C₁₂-C₁₈ fatty acid alcohol, polyethylene glycol, polyols, saccharides, oligosaccharides, and combinations thereof. The binders used in a pharmaceutical formulation are, in some examples, selected from starches such as potato starch, corn starch, wheat starch; sugars such as sucrose, glucose, dextrose, lactose, maltodextrin; natural and synthetic gums; gelatine; cellulose derivatives such as microcrystalline cellulose, hydroxypropyl cellulose, hydroxyethyl cellulose, hydroxypropyl methyl cellulose, carboxymethyl cellulose, methyl cellulose, ethyl cellulose; polyvinylpyrrolidone (povidone); polyethylene glycol (PEG); waxes; calcium carbonate; calcium phosphate; alcohols such as sorbitol, xylitol, mannitol and water or any combinations thereof.

In some aspects a pharmaceutical composition as described herein comprises a lubricant as an excipient. Non-limiting examples of suitable lubricants include magnesium stearate, calcium stearate, zinc stearate, hydrogenated vegetable oils, sterotex, polyoxyethylene monostearate, talc, polyethyleneglycol, sodium benzoate, sodium lauryl sulfate, magnesium lauryl sulfate, and light mineral oil. The lubricants that are used in a pharmaceutical formulation, in some aspects, are be selected from metallic stearates (such as magnesium stearate, calcium stearate, aluminium stearate), fatty acid esters (such as sodium stearyl fumarate), fatty acids (such as stearic acid), fatty alcohols, glyceryl behenate, mineral oil, paraffins, hydrogenated vegetable oils, leucine, polyethylene glycols (PEG), metallic lauryl sulphates (such as sodium lauryl sulphate, magnesium lauryl sulphate), sodium chloride, sodium benzoate, sodium acetate and talc or a combination thereof.

In some aspects a pharmaceutical formulation comprises a dispersion enhancer as an excipient. Non-limiting examples of suitable dispersants include, in some examples, starch, alginic acid, polyvinylpyrrolidones, guar gum, kaolin, bentonite, purified wood cellulose, sodium starch glycolate, isoamorphous silicate, and microcrystalline cellulose as high HLB emulsifier surfactants.

In some aspects a pharmaceutical composition as described herein comprises a disintegrant as an excipient. In some aspects a disintegrant is a non-effervescent disintegrant. Non-limiting examples of suitable non-effervescent disintegrants include starches such as corn starch, potato starch, pregelatinized and modified starches thereof, sweeteners, clays, such as bentonite, micro-crystalline cellulose, alginates, sodium starch glycolate, gums such as agar, guar, locust bean, karaya, pecitin, and tragacanth. In some aspects a disintegrant is an effervescent disintegrant. Non-limiting examples of suitable effervescent disintegrants include sodium bicarbonate in combination with citric acid, and sodium bicarbonate in combination with tartaric acid.

In some aspects an excipient comprises a flavoring agent. Flavoring agents incorporated into an outer layer are, in some examples, chosen from synthetic flavor oils and flavoring aromatics; natural oils; extracts from plants, leaves, flowers, and fruits; and combinations thereof. In some aspects a flavoring agent can be selected from the group consisting of cinnamon oils; oil of wintergreen; peppermint oils; clover oil; hay oil; anise oil; eucalyptus; vanilla; citrus oil such as lemon oil, orange oil, grape and grapefruit oil; and fruit essences including apple, peach, pear, strawberry, raspberry, cherry, plum, pineapple, and apricot.

In some aspects an excipient comprises a sweetener. Non-limiting examples of suitable sweeteners include glucose (corn syrup), dextrose, invert sugar, fructose, and mixtures thereof (when not used as a carrier); saccharin and its various salts such as a sodium salt; dipeptide sweeteners such as aspartame; dihydrochalcone compounds, glycyrrhizin; Stevia Rebaudiana (Stevioside); chloro derivatives of sucrose such as sucralose; and sugar alcohols such as sorbitol, mannitol, sylitol, and the like.

In some instances, a pharmaceutical composition as described herein comprises a coloring agent. Non-limiting examples of suitable color agents include food, drug and cosmetic colors (FD&C), drug and cosmetic colors (D&C), and external drug and cosmetic colors (Ext. D&C). A coloring agents can be used as dyes or their corresponding lakes.

In some instances, a pharmaceutical composition as described herein comprises a chelator. In some cases, a chelator is a fungicidal chelator. Examples include, but are not limited to: ethylenediamine-N,N,N',N'-tetraacetic acid (EDTA); a disodium, trisodium, tetrasodium, dipotassium, tripotassium, dilithium and diammonium salt of EDTA; a barium, calcium, cobalt, copper, dysprosium, europium, iron, indium, lanthanum, magnesium, manganese, nickel, samarium, strontium, or zinc chelate of EDTA; trans-1,2-diaminocyclohexane-N,N,N',N'-tetraaceticacid monohydrate; N,N-bis(2-hydroxyethyl)glycine; 1,3-diamino-2-hydroxypropane-N,N,N',N'-tetraacetic acid; 1,3-diaminopropane-N,N,N',N'-tetraacetic acid; ethylenediamine-N,N'-diacetic acid; ethylenediamine-N,N'-dipropionic acid dihydrochloride; ethylenediamine-N,N'-bis(methylenephosphonic acid) hemihydrate; N-(2-hydroxyethyl)ethylenediamine-N,N',N'-triacetic acid; ethylenediamine-N,N,N',N'-tetrakis(methylenephosponic acid); O,O'-bis(2-aminoethyl)ethyleneglycol-N,N,N',N'-tetraacetic acid; N,N-bis(2-hydroxybenzyl)ethylenediamine-N,N-diacetic acid; 1,6-hexamethylenediamine-N,N,N',N'-tetraacetic acid; N-(2-hydroxyethyl)iminodiacetic acid; iminodiacetic acid; 1,2-diaminopropane-N,N,N',N'-tetraacetic acid; nitrilotriacetic acid; nitrilotripropionic acid; the trisodium salt of nitrilotris(methylenephosphoric acid); 7,19,30-trioxa-1,4,10,13,16,22,27,33-octaazabicyclo[11,11,11] pentatriacontane hexahydrobromide; or triethylenetetramine-N,N,N',N",N"',N"'-hexaacetic acid.

Also contemplated are combination products that include one or more immunotherapeutic agents disclosed herein and one or more other antimicrobial or antifungal agents, for example, polyenes such as amphotericin B, amphotericin B lipid complex (ABCD), liposomal amphotericin B (L-AMB), and liposomal nystatin, azoles and triazoles such as voriconazole, fluconazole, ketoconazole, itraconazole, pozaconazole and the like; glucan synthase inhibitors such as caspofungin, micafungin (FK463), and V-echinocandin (LY303366); griseofulvin; allylamines such as terbinafine; flucytosine or other antifungal agents, including those described herein. In addition, it is contemplated that a peptide can be combined with topical antifungal agents such as ciclopirox olamine, haloprogin, tolnaftate, undecylenate, topical nysatin, amorolfine, butenafine, naftifine, terbinafine, and other topical agents. In some instances, a pharmaceutical composition comprises an additional agent. In some cases, an additional agent is present in a therapeutically effective amount in a pharmaceutical composition.

Under ordinary conditions of storage and use, the pharmaceutical compositions as described herein comprise a preservative to prevent the growth of microorganisms. In certain examples, the pharmaceutical compositions as described herein do not comprise a preservative. The pharmaceutical forms suitable for injectable use include sterile aqueous solutions or dispersions and sterile powders for the extemporaneous preparation of sterile injectable solutions or dispersions. The pharmaceutical compositions comprise a carrier which is a solvent or a dispersion medium containing, for example, water, ethanol, polyol (e.g., glycerol, propylene glycol, and liquid polyethylene glycol, and the like), and/or vegetable oils, or any combinations thereof. Proper fluidity is maintained, for example, by the use of a coating, such as lecithin, by the maintenance of the required particle size in the case of dispersion and by the use of surfactants. The prevention of the action of microorganisms is brought about by various antibacterial and antifungal agents, for example, parabens, chlorobutanol, phenol, sorbic acid, thimerosal, and the like. In many cases, isotonic agents are included, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the liquid dosage form is suitably buffered if necessary and the liquid diluent rendered isotonic with sufficient saline or glucose. The liquid dosage forms are especially suitable for intravenous, intramuscular, subcutaneous, intratumoral, and intraperitoneal administration. In this connection, sterile aqueous media that can be employed will be known to those of skill in the art in light of the present disclosure. For example, one dosage is dissolved, in certain cases, in 1mL to 20 mL of isotonic NaCl solution and either added to 100 mL to 1000 mL of a fluid, e.g., sodium-bicarbonate buffered saline, or injected at the proposed site of infusion.

In certain aspects, sterile injectable solutions is prepared by incorporating a therapeutic agent, in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. The compositions disclosed herein are, in some instances, formulated in a neutral or salt form. Pharmaceutically-acceptable salts include, for example, the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups are, in some cases, derived from inorganic bases such as, for example, sodium, potassium, ammonium, calcium, or ferric hydroxides, and such organic bases as isopropylamine, trimethylamine, histidine, procaine and the like. Upon formulation, the pharmaceutical compositions are administered, in some aspects, in a manner compatible with the dosage formulation and in such amount as is therapeutically effective.

In certain aspects, a pharmaceutical composition of this disclosure comprises an effective amount of a therapeutic agent, as disclosed herein, combined with a pharmaceutically acceptable carrier. "Pharmaceutically acceptable," as used herein, includes any carrier which does not interfere with the effectiveness of the biological activity of the active ingredients and/or that is not toxic to the patient to whom it is administered. Non-limiting examples of suitable pharmaceutical carriers include phosphate buffered saline solutions, water, emulsions, such as oil/water emulsions, various types of wetting agents and sterile solutions. Additional non-limiting examples of pharmaceutically compatible carriers can include gels, bioadsorbable matrix materials, implantation elements containing the immunotherapeutic agents or any other suitable vehicle, delivery or dispensing means or material. Such carriers are formulated, for example, by conventional methods and administered to the subject at an effective amount.

In some aspects, the pharmaceutical composition is a formulation comprising an immunotherapy agent (e.g., an immune check point inhibitor, regulator, or activator) and a buffering agent. In some aspects, the immunotherapy agent is present at a concentration of about 10 to about 50 mg/mL, about 15 to about 50 mg/mL, about 20 to about 45 mg/mL, about 25 to about 40 mg/mL, about 30 to about 35 mg/mL, about 25 to about 35 mg/mL, or about 30 to about 40 mg/mL, about 15 mg/mL, about 20 mg/mL, about 25 mg/mL, about 30 mg/mL, about 33.3 mg/mL, about 35 mg/mL, about 40 mg/mL, about 45 mg/mL, or about 50 mg/mL. In some aspects, the formulation comprises a buffering agent comprising histidine (e.g., a histidine buffer). In certain aspects, the buffering agent (e.g., histidine buffer) is present at a concentration of about 1 mM to about 20 mM, about 2 mM to about 15 mM, about 3 mM to about 10 mM, about 4 mM to about 9 mM, about 5 mM to about 8 mM, or about 6 mM to about 7 mM, about 1 mM, about 2 mM, about 3 mM, about 4 mM, about 5 mM, about 6 mM, about 6.7 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, or about 20 mM. In some aspects, the buffering agent (e.g., histidine buffer) is present at a concentration of about 6 mM to about 7 mM, about 6.7 mM. In other aspects, the buffering agent (e.g., a histidine buffer) has a pH of about 4 to about 7, about 5 to about 6, about 5.5, or about 6.

In some aspects, the formulation further comprises a carbohydrate. In certain aspects, the carbohydrate is sucrose. In some aspects, the carbohydrate (e.g., sucrose) is present at a concentration of about 50 mM to about 150 mM, about 25 mM to about 150 mM, about 50 mM to about 100 mM, about 60 mM to about 90 mM, about 70 mM to about 80 mM, or about 70 mM to about 75 mM, about 25 mM, about 50 mM, about 60 mM, about 70 mM, about 80 mM, about 90 mM, about 100 mM, or about 150 mM.

In some aspects, the formulation further comprises a surfactant. In certain aspects, the surfactant is polysorbate 20. In some aspects, the surfactant or polysorbate 20) is present at a concentration of about 0.005 % to about 0.025% (w/w), about 0.0075% to about 0.02% or about 0.01 % to 0.015% (w/w), about 0.005%, about 0.0075%, about 0.01%, about 0.013%, about 0.015%, or about 0.02% (w/w). In certain aspects, the formulation is a reconstituted formulation. For example, a reconstituted formulation is prepared, in some instances, by dissolving a lyophilized formulation in a diluent such that the immunotherapy agent is dispersed in the reconstituted formulation. In some aspects, the lyophilized formulation is reconstituted with about 0.5 mL to about 2 mL, such as about 1 mL, of water or buffer for injection. In certain aspects, the lyophilized formulation is reconstituted with 1 mL of water for injection at a clinical site.

### Combination therapies

In certain aspects, the methods of this disclosure comprise administering a therapeutic agent as disclosed herein, followed by, and preceded by or in combination with one or more further therapy. Examples of the further therapy can include, but are not limited to, chemotherapy, radiation, an anti-cancer agent, or any combinations thereof. The further therapy can be administered concurrently or sequentially with respect to administration of the immunotherapy. In certain aspects, the methods of this disclosure comprise administering an immunotherapy as disclosed herein, followed by, preceded by, or in combination with one or more anti-cancer agents or cancer therapies. Anti-cancer agents include, but are not limited to, chemotherapeutic agents, radiotherapeutic agents, cytokines, immune checkpoint inhibitors, anti-angiogenic agents, apoptosis-inducing agents, anti-cancer antibodies and/or anti-cyclin-dependent kinase agents. In certain aspects, the cancer therapies include chemotherapy, biological therapy, radiotherapy, immunotherapy, cell therapy, hormone therapy, anti-vascular therapy, cryotherapy, toxin therapy and/or surgery or combinations thereof. In certain aspects, the methods of this disclosure include administering an immunotherapy, as disclosed herein, followed by, preceded by or in combination with one or more further immunomodulatory agents. An immunomodulatory agent includes, in some examples, any compound, molecule or substance capable of suppressing antiviral immunity associated with a tumor or cancer. Non-limiting examples of the further immunomodulatory agents include anti-CD33 antibody or variable region thereof, an anti-CD11b antibody or variable region thereof, a COX2 inhibitor, e.g., celecoxib, cytokines, such as IL-12, GM-CSF, IL-2, IFN3 and 1FNy, and chemokines, such as MIP-1, MCP-1 and IL-8.

In certain examples, where the further therapy is cell therapy, exemplary cell therapies include without limitation immune effector cell therapy, chimeric antigen receptor T-cell (CAR-T) therapy, natural killer cell therapy and chimeric antigen receptor natural killer (NK) cell therapy. Either NK cells, or CAR-NK cells, or a combination of both NK cells and CAR-NK cells can be used in combination with the methods disclosed herein. In some aspects, the NK cells and CAR-NK cells are derived from human induced pluripotent stem cells (iPSC), umbilical cord blood, or a cell line. In some aspects, the NK cells and CAR-NK cells comprise a cytokine receptor and a suicide gene. In some aspects, the NK cells and CAR-NK cells are characterized by the presence of CD56 and the absence of CD3 surface markers. In some aspects, the NK cells and CAR-NK cells target tumor cells (including solid tumors) and/or cells harboring viruses. In some aspects, the NK cells and CAR-NK cells are administered in combination with an inhibitor of hyaluronan. In some aspects, the NK cells and CAR-NK cells are administered in combination with an anti-PD-L1 therapy.

In certain examples, where the further therapy is radiation exemplary doses are 5,000 Rads (50 Gy) to 100,000 Rads (1000 Gy), or 50,000 Rads (500 Gy), or other appropriate doses within the recited ranges. Alternatively, the radiation dose are about 30 to 60 Gy, about 40 to about 50 Gy, about 40 to 48 Gy, or about 44 Gy, or other appropriate doses within the recited ranges, with the dose determined, example, by means of a dosimetry study as described above. "Gy" as used herein can refer to a unit for a specific absorbed dose of radiation equal to 100 Rads. Gy is the abbreviation for "Gray."

In certain examples, where the further therapy is chemotherapy, exemplary chemotherapeutic agents include without limitation alkylating agents (e.g., nitrogen mustard derivatives, ethylenimines, alkylsulfonates, hydrazines and triazines, nitrosureas, and metal salts), plant alkaloids (e.g., vinca alkaloids, taxanes, podophyllotoxins, and camptothecan analogs), antitumor antibiotics (e.g., anthracyclines, chromomycins, and the like), antimetabolites (e.g., folic acid antagonists, pyrimidine antagonists, purine antagonists, and adenosine deaminase inhibitors), topoisomerase I inhibitors, topoisomerase II inhibitors, and miscellaneous antineoplastics (e.g., ribonucleotide reductase inhibitors, adrenocortical steroid inhibitors, enzymes, antimicrotubule agents, and retinoids). Exemplary chemotherapeutic agents can include, without limitation, anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}), Ibrutinib, idelalisib, and brentuximab vedotin.

Exemplary alkylating agents include, without limitation, nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen Mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana^{®}), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

Exemplary anthracyclines can include, without limitation, e.g., doxorubicin (Adriamycin^{®} and Rubex^{®}); bleomycin (Lenoxane^{®}); daunorubicin (dauorubicin hydrochloride, daunomycin, and rubidomycin hydrochloride, Cerubidine^{®}); daunorubicin liposomal (daunorubicin citrate liposome, DaunoXome^{®}); mitoxantrone (DHAD, Novantrone^{®}); epirubicin (Ellence^{™}); idarubicin (Idamycin^{®}, Idamycin PFS^{®}); mitomycin C (Mutamycin^{®}); geldanamycin; herbimycin; ravidomycin; and desacetylravidomycin.

Exemplary vinca alkaloids include, but are not limited to, vinorelbine tartrate (Navelbine^{®}), Vincristine (Oncovin^{®}), and Vindesine (Eldisine^{®})); vinblastine (also known as vinblastine sulfate, vincaleukoblastine and VLB, Alkaban-AQ^{®} and Velban^{®}); and vinorelbine (Navelbine^{®}).

Exemplary proteosome inhibitors can, but are not limited to, bortezomib (Velcade^{®}); carfilzomib (PX-171-007, (S)-4-Methyl-N-((S)-1-(((S)-4-methyl-1-((R)-2-methyloxiran-2-yl)-1-oxopentan-2-yl)amino)-1-oxo-3-phenylpropan-2-yl)-2-((S)-2-(2-morpholinoac etamido)-4-phenylbutanamido)-pentanamide); marizomib (NPI-0052); ixazomib citrate (MLN-9708); delanzomib (CEP-18770); and O-Methyl-N-[(2-methyl-5-thiazolyl)carbonyl]-L-seryl-O-methyl-N-[(1S)-2-[(2R)-2-methyl-2-oxiranyl]-2-oxo-1-(phenylmethyl)ethyl]-L-serinamide (ONX-0912).

"In combination with," as used herein, means that the therapeutic agent and the further therapy are administered to a subject as part of a treatment regimen or plan. In certain aspects, being used in combination does not require that the immunotherapy and the further therapy are physically combined prior to administration or that they be administered over the same time frame. For example, and not by way of limitation, the immunotherapy and the one or more agents are administered concurrently to the subject being treated, or are administered at the same time or sequentially in any order or at different points in time.

The further therapy is administered, in various aspects, in a liquid dosage form, a solid dosage form, a suppository, an inhalable dosage form, an intranasal dosage form, in a liposomal formulation, a dosage form comprising nanoparticles, a dosage form comprising microparticles, a polymeric dosage form, or any combinations thereof. In certain aspects, the further therapy is administered over a period of about 1 week to about 2 weeks, about 2 weeks to about 3 weeks, about 3 weeks to about 4 weeks, about 4 weeks to about 5 weeks, about 6 weeks to about 7 weeks, about 7 weeks to about 8 weeks, about 8 weeks to about 9 weeks, about 9 weeks to about 10 weeks, about 10 weeks to about 11 weeks, about 11 weeks to about 12 weeks, about 12 weeks to about 24 weeks, about 24 weeks to about 48 weeks, about 48 weeks or about 52 weeks, or longer. The frequency of administration of the further therapy is, in certain instances, once daily, twice daily, once every week, once every three weeks, once every four weeks (or once a month), once every 8 weeks (or once every 2 months), once every 12 weeks (or once every 3 months), or once every 24 weeks (once every 6 months).

### Models of Human Serrated Colorectal Cancer

### Models

Aspects disclosed herein provide a model of human serrated colorectal cancer (CRC). In some instances the model is a tissue model. In some instances, the tissue model comprises an organoid. In some instances, the model comprises an animal model. In some instances, the model comprises genetic information that specifically inhibits expression of *PRKCI* and *PRKCZ* such that PKC 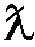 / 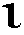 and PKCζ are not expressed. In some instances, the model comprises genetic information that specifically reduces expression of *PRKCI* and *PRKCZ,* resulting in reduced expression of PKC 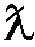 / 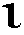 and PKCζ.

Disclosed herein in some aspects, is an animal model of human serrated CRC. In some instances, the animal model comprises a knockout or knockdown of *PRKCI* and *PRKCZ.* In some instances, the knockout or knockdown is produced using homologous recombination, nuclease-based gene editing (e.g., CRISPR/Cas9), site-specific recombination (e.g., recombination-mediated cassette exchange, flip-excisionswitch, Cre-loxP recombination system), tetracycline-based systems (e.g., Tet-On/Off), internal ribosome entry site, or a combination thereof. In some instances, the animal model comprises a mammal. In some instances, the animal model comprises a mouse, a rat, a monkey. In some instances, the

Disclosed herein, in some aspects are methods of producing an animal model of serrated colorectal cancer comprising: a)providing an animal that is Prkci ^{fl/fl} and Prkcz ^{fl/fl}, wherein the animal has loxP sites flanking genes comprising Prkci and Prkcz; and crossing the animal that is Prkci ^{fl/fl} and Prkcz ^{fl/fl} with an animal expressing a Cre recombinase operably linked to an ubiquitous promoter in villus and crypt epithelial cells of a small and a large intestine, thereby generating a transgenic animal with deletions in Pkcζ and Pkc 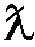 / 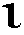 in the villus and the crypt epithelial cells of the small and the large intestine.

Aspects disclosed herein provide an animal model of human serrated CRC with deletions of PKC 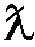 / 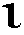 and PKCζ of human serrated CRC. The animal model may be characterized by reduced body size and weight, as well as worse survival rate than wild-type (WT) animals **(****FIGS. 1A-1C****).** In some instances, the intestines of the animal model are dilated and full, suggesting altered intestinal function **(****FIG. 1A****).** The animal model may be characterized by a reduction in the number of Paneth cells in the crypts of the intestine, and/or mislocalization of Paneth cells **(****FIG. 8A****).** In some instances, the animal model comprises a deformed hyperplastic appearance in both the small intestine and colon, resembling human serrated phenotype **(****FIG. 1D****).** In some instances, the animal model comprises cytomorphologic features that are similar to human microvesicular or goblet cell-rich serrated hyperplasia **(****FIG. 8B****),** which is a premalignant lesion that can progress to CRC through an alternative pathway. In some instances, the animal model develops spontaneous sessile serrated adenomas/polyps (SSA/Ps) in the colon and intestine with dilated and distorted crypts **(****FIGS. 1D and 1E****).** In some instances, the SSA/Ps develop into intestinal tumors, which increase in number as the animal ages **(****FIG. 1F** and **FIG. 9C****).** In some instances the tumors localize in the proximal location of the colon **(****FIG. 1G, FIG. 1H****,** and **FG. 8D).** In some instances the tumors progress into intramucosal **(****FIG. 1D****)** and/or invasive adenocarcinoma (**FIG.1I**). In some instances, the animal model may be characterized a DKO^{IEC} transcriptomic signature comprising a plurality of genes selected from **Table 7.** In some instances, the transcriptomic profile comprises 2, 3, 4, 5, 6, 7, 8, 9, 10, 15, 20, 25, or 30 genes from **Table 7.**

Aspects disclosed herein provide an animal model of human serrated CRC with deletions of PKC 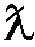 / 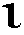 and PKCζ that are characterized by a distinct cancer stem cell transcriptomic profile. In some instances, the animal model may be characterized by an increase in (*e.g.*, overexpression) hyaluronan expression, as compared to an animal without deletion of PKC^/i and PKCζ. In some instances, the overexpression of hyaluronan is localized in stroma of serrated tumors of the animal model. In some instances, the animal model may be characterized by an increase in CD44 expression. In some instances, the overexpression of CD44 is localized in the intestinal epithelial cells. In some instances, hyaluronan and/or CD44 expression increase as the serrated CRC progresses from hyperplasia to sessile serrated adenoma/polyps (SSA/P), and from SSA/P to carcinoma. In some instances, the animal model is characterized by decrease in expression of interferon-responsive genes comprising Irf7, Oas1a, Isg15, and/or Cxcl10, as compared to an animal with deletion of either PKC 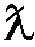 / 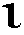 or PKCζ; and/or as compared to a wild-type (WT) animal. In some instances, the animal model may be characterized by a decrease in expression of stem cell biomarkers comprising Olfm4, Ascl2, and/or Lgr5.

Aspects disclosed herein provide an animal model of human serrated CRC with deletions of PKC 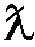 / 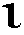 and PKCζ that develop tumors that are highly similar to human MMS serrated tumors at the transcriptional level. In some instances, animal model is further characterized by a microsatellite stable (MSS) transcriptomic profile of serrated CRC **(****FIG. 8E and FIG. 8F****).** Non-limiting examples of genes associated with the MSS phenotype in CRC include *FLNB, GLCE, CCDCI46, GTF2IRD2, GTF2IRD2B, GTF2IRD2P1, INADL, DOCK5, MACF1, AKAP7, TTC19, MAPIB, PTK7, BIK, SHROOM3, KLK7, RHBDL2, PLK2, EPM2AIP1, AHNAK, PSTPIP2, NMU, EPB41L4A, ACTA2, PHLDB2, ISM1, KITLG, PTPRD, SLC7A8, RASGRF1, YPEL1, UBASH3B, VSIG2, MYCL1, IL8, TCF7, TLR4, ACAA2, TET2, ENPP3, ST6GAL2, CFTR, MYCN, PALLD, ARAP2, MYOF, ARHGAP29, MT1E, BICC1, ABCC3, NRXN3, C19orf45 SFRP5, REN, NEDD9, APCDD1, CXCL6, C6orf15, INPP4B, SGPL1, KIT, TMEM211, CCDC3, CXCL14, UCA1, C16orf62, HSPA2, SPATA18, MPZL2, FHL2, CSGALNACTI, LOC100506403, RUNX1, ID4, ACTG1P4, AMY1A, AMY1B, AMYIC, AMY2A, AMY2B, NDP, CD200, PDE9A, SLCO1B3, TUBA1A, ANXA3, ILI7RD, PTCHD4, NTRK2, LPAR6, CHRNB1, ANXA1, CAPN8, EDN1, TSPAN2, KLK10, PDE4D, CYP4X1, ATP8A1, TWSG1, MYLK, TGFB3, TRIM22,* and *FSTL1.* In some instances, the animal model is enriched for an expression of one or more genes in the Extracellular Signal-Regulated Kinase (ERK) pathway and/or Yes Associated Protein (YAP) pathway. In some instances, enrichment of one or more genes is detected in intestinal epithelium cells (IEC). Non-limiting examples of genes in the ERK pathway that may be enriched in the animal model include *ARAF, DLK1, MAP3K1 (MEKKI), MAP3K2 (MEKK2), MAP3K3 (MEKK3), MAP3K4 (MEKK4), MAP4K1 (HPKI), MOS, PAK1, RAF1, MAP2K1 (MEK1), MAP2K2 (MEK2), MAP2K3 (MEK3), MAP2K4 (MKK4, JNKK1), MAP2K5 (MEK5), MAP2K6 (MEK6, MKK6), MAP2K7 (JNKK2), MAPKI (ERK2), MAPK3 (ERKI), MAPK6 (ERK3), MAPK7 (ERKS), MAPK8 (JNK1), MAPK9 (JNK2), MAPK10 (JNK3), MAPK11 (P38BETA), MAPK12 (P38GAMMA), MAPK13 (SAPK4, SERK4), MAPK14 (P38ALPHA), ATF2 (CREB2), CREB1, CREBBP (CBP), EGFR, ELK1, ETS1, ETS2, JUN, KCNNI (KCA2.1), MAPKAPK2, MAPKAPK5, MAX, MEF2C, MKNK1, MYC, NFATC4 (NFAT3), SMAD4 (MADH4), TRP53 (P53). COL1A1, EGR1, FOS, HSPA5 (GRP78), HSPBI (HSP27), JUN, MYC, TRP53 (P53), HRAS, KRAS, KSR1, MAP2K1 (MEKI), MAP2K2 (MEK2), NRAS, CDC42, CHUK (IKBKA), GRB2, HRAS, KCNNI (KCA2.1), KRAS, MAP2K1 (MEK1), MAP2K2 (MEK2), MAP2K4 (MKK4, JNKK1), MAP4K1 (HPKI), NRAS, RAC1, SFN (14-3-3Σ), LAMTOR3 (MPI), MAP3K1 (MEKK1), MAPK8IP1 (JIPI), MAPK8IP2 (JIP2), MAPK8IP3 (JIP3), CCNA1, CCNA2, CCNB1, CCNB2, CCND1, CCND2, CCND3, CCNE1, CDK2, CDK4, CDK6, CDKN1A (P21CIP1, WAF1), CDKN1B (P27KIP1), CDKN1C (P57KIP2), CDKN2A (P16INK4A), CDKN2B (P15INK4B), CDKN2C (P18INK4C), CDKN2D (P19INK4D), AND E2F1, RBI, or analogues thereof. Non-limiting examples of genes in the YAP pathway that may be enriched in the animal model include ACTG1, AMOT, AMOTL1, AMOTL2, CASP3, CRB1, CRB2, CRB3, CSNK1D, CSNK1E, DCHS1, DCHS2, DIAP2, DLG1, DVL2, FAT1, FAT2, FAT3, FAT4, FJX1, AJUBA, LIMD1, LIX1L, LLGL1, LLGL2, LPP, NF2, RASSF2, RASSF4, SCRIB, WTIP, WWC1, ZDHHC18, LATS2, MOBIB, MOB1A, SAV1, STK3, STK4, TAZ, YAP1, MST1, WWTR1, HIPK2, MAPK10 (JNK3), MEIS1, PRKCI, PRKCZ, SMAD1 (MADH1), TEAD1, TEAD2, TEAD3, TEAD4, TRP63 (TP73L), TSHZ1, TSHZ2, TSHZ3, WNT1, CASP3, CCNE1, CCNE2, MYC, PPP2CB, PPP2R1A, PPP2R2D, PTPN14, RERE, NF2, TAZ, YAP1, CRB1, CRB2, CRB3, DCHS1, DCHS2, FAT1, FAT2, FAT3, FAT4, LATS1, LATS2, MST1, SCRIB, STK3, STK4, GPC5, HMCN1, POTEG, TAOK1, TAOK2, TAOK3, TJP1, TJP2, MPDZ, MPP5, NPHP4, PARD3, PARD6G, YWHAB, YWHAE, YWHAQ,* and *YWHAZ,* or analogues thereof.

Aspects disclosed herein provide an animal model of human serrated CRC with deletions of PKC 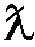 / 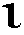 and PKCζ that develop an immunosuppressive tumor environment similar to human serrated CRC. In some instances, the animal model may be characterized by an increase in CD45+ cells, as compared to an animal without deletions of PKC 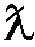 / 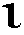 and PKCζ; and as compared to an animal with deletions of either PKC 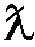 / 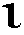 or PKCζ (**FIG. 3A****).** In some instances, the animal model may be characterized by an increase in a level of programmed death-ligand 1 (PD-L1) expression, as compared to the level of PD-L1 expression in a WT animal. In some instances, the increase in the level of PD-L1 expression in detectable in the CD45+ cells. In some instances, the animal model may be characterized by a decrease in CD8+ cells as compared to a WT animal. In some instances, the animal model may be characterized by an impaired interferon response. Non-limiting examples of genes involved in the interferon response include *IRF7, OAS1A, ISG15,* and *CXCL10.*

Aspects disclosed herein provide an animal model of human serrated CRC with deletions of PKCk/i and PKCζ that develop an activated stromal response similar to human serrated CRC. In some instances the animal model may be characterized by an increase in alpha-smooth muscle actin (αSMA) **(****FIG. 3K****).** In some instances, the animal model may be characterized by an increase in collagen deposition (**FIG. 3L** and **FIG. 10C****).** In some instances, the animal model may be characterized by an increase in stroma-derived growth factors comprising ligands for epidermal growth factor receptor (EGFR), amphiregulin (Areg), and epiregulin (Ereg).

### Methods of Using a Model of Human Serrated Colorectal Cancer

Aspects disclosed herein provide methods of screening for modulators of serrated tumorigenesis using the models disclosed herein. Modulators of serrated tumorigenesis may include the therapeutic agents, or further therapies, disclosed herein. Disclosed herein in some aspects are methods of screening for modulators of serrated tumorigenesis, the method comprising: a) providing the model of human serrated colorectal cancer (CRC) disclosed herein; b) contacting the model with a putative modulator of tumorigenesis; and c) detecting one or more changes in one or more tumor-associated parameters in the model. Non-limiting examples of tumor-associated parameters include recruitment of CD8+ T cells to a serrated tumor, progression of a sessile serrated adenoma (SSA) to adenocarcinoma, a number of serrated tumors, a size of serrated tumors, a total load of serrated tumors, an increase in an expression of alpha-smooth muscle actin (αSMA), or collagen deposition, or a combination thereof. In non-limiting examples of how tumor-associated parameters may be used according to the present methods, a putative modulator negatively modulates tumorigenesis, provided an increase in CD8+ T cells to a serrated tumor is observed; a putative modulator negatively modulates tumorigenesis, provided the progression of the SSA to adenocarcinoma is not observed; a putative modulator negatively modulates tumorigenesis, provided the number of serrated tumors decreases, as compared to a number of serrated tumors in the model before contacting the model with the putative modulator, is observed; a putative modulator negatively modulates tumorigenesis, provided the size of the serrated tumors is smaller, as compared to a size of serrated tumors in the model before contacting the model with the putative modulator, is observed; a putative modulator negatively modulates tumorigenesis, provided the total load of the serrated tumors decreases, as compared to a tumor load of the serrated tumors in the model before contacting the model with the putative modulator, is observed; a putative modulator negatively modulates tumorigenesis, provided the increase in the expression of αSMA, as compared to an expression of αSMA in the model before contacting the model with the putative modulator, is not observed; a putative modulator negatively modulates tumorigenesis, provided the increase in the collagen deposition, as compared to collagen deposition in the model before contacting the model with the putative modulator, is not observed; a putative modulator is selected from the group consisting of an inhibitor of programmed death-ligand 1 (PD-L1) activity or expression, an inhibitor of transforming growth factor beta 1 (TGF-β1) activity or expression, and an inhibitor of hyaluronan activity of expression.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention

### Example 1. Loss of both aPKc results in serrated tumor development in small intestine and colon

To test whether the combined loss of PKC 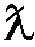 / 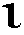 and PKCζ in the intestinal epithelium is sufficient to drive tumorigenesis in the absence of other oncogenic insults, Prkci^{fl/fl} and Prkcz^{fl/fl} mice were crossed with Villin-cre mice to generate mouse lines with deletions in PKC 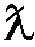 / 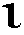 (LKO^{IEC}), PKCζ (ZKO^{IEC}), or both aPKCs (DKO^{IEC}) in IECs. DKO^{IEC} mice were born at the expected Mendelian ratios but showed reduced body size and weight, as well as a worse survival rate than wild-type (WT) mice **(****FIG. 1A****-****FIG. 1C****).** DKO^{IEC} intestines often appeared dilated and full, suggesting altered intestinal function and obstruction **(****FIG. 1A****),** and had a severe reduction in the number of Paneth cells in the crypts, as determined by lysozyme immunohistochemistry (IHC) **(****FIG. 8A****).** Remaining Paneth cells were mislocalized at the upper crypt and villi, and a population of immature differentiated cells that stained positive for markers of both Paneth cells (lysozyme) and goblet cells (Alcian blue) was also found **(****FIG. 8A****).** DKOIEC mice exhibited a deformed, "saw-tooth" hyperplastic appearance in both the small intestine and colon, which resembled the human serrated phenotype **(****FIG.1D****).** This resemblance extended to the existence in the DKO^{IEC} intestines of cytomorphologic features that were similar to human microvesicular or goblet cell-rich serrated hyperplasia (FI**G**. **8B),** which is a premalignant lesion that can progress to CRC through an alternative pathway to the conventional "adenoma-carcinoma" sequence. After 7 weeks of age, both the small intestines and colons of DKO^{IEC} mice developed spontaneous sessile serrated adenomas/polyps (SSA/Ps) with dilated and distorted crypts **(****FIG. 1D** and **FIG. 1E****).** In sharp contrast with the single-aPKC-deficient mice (LKO^{IEC} and ZKO^{IEC}), which almost never developed tumors **(****FIG. 8C****),** DKO^{IEC} mice showed efficient and progressive development of intestinal tumors, which increased as the mice aged **(****FIG. 1F** and **FIG. 8C****).** Although no significant differences were found in tumor distribution among distinct regions of small intestine, there was a preference for the proximal location in the colon **(****FIG. 1G, FIG. 1H****,** and **FIG. 8D****),** which closely mirrors the behavior of human serrated CRC. Colon tumors were occasionally accompanied by mucin inclusions **(****FIG.1H****).** Aberrant crypt formation in the form of lateral budding of crypts and cytological atypia were also observed, indicating the presence of accompanying dysplastic changes **(****FIG. 1D****).** Furthermore, a large subset of DKOIEC intestinal tumors progressed to intramucosal **(****FIG. 1D****)** and even invasive adenocarcinoma **(****FIG. 1I**). That is, 72.2% (13/18) and 81.3% (13/16) of mice developed adenocarcinoma in the small intestine and colon, respectively.

Invasive adenocarcinoma includes not only a tubular adenocarcinoma component, but also occasionally poorly differentiated carcinoma **(****FIG. 1J****)** or signet-ring cell carcinoma, which is morphologically characterized by abundant intracytoplasmic mucin that pushes the nucleus to the periphery as indicated by Ki67 and Alcian blue double-positive cells **(****FIG 1K****).** We also observed a severe desmoplastic change indicating the high aggressiveness of those tumors (FIG. 1L). Considering that poorly differentiated mucinous and signet-ring cell appearances have been associated with the MSS and aggressive phenotypes of human serrated tumors, strongly suggests that DKOIEC mouse tumors morphologically mimic MSS serrated human disease. Indeed, analysis of microsatellite repeat markers demonstrated that DKOIEC tumors were MSS (FI**G. 8E** and **FIG. 8F****).** No changes in the mRNA expression of representative DNA-MMR genes in these tumors **(****FIG. 8G****)** were observed. In agreement with the positive association in human serrated tumors between MSS/MSI-L and CpG island methylator phenotype (CIMP)-low/CIMP-negative profiles, none of the markers defining CIMP was altered **(****FIG. 8H****).** Furthermore, senescence was not detected in DKO^{IEC} mouse intestines (FIG. 8I). Similar to what has been reported in serrated tumors driven by KRAS or BRAF, and consistent with the notion that SSA/Ps a premalignant lesions that are not fully transformed, we observed increased expression of p53, p21, and p16 in the SSA/Ps, which all reverted when the tumors reached the carcinoma stage (FIG. 8J). Collectively, these results demonstrate that the simultaneous loss of PKC 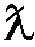 / 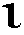 and PKCζ in the intestinal epithelium leads to the development of SSA/Ps that further progress to MSS invasive carcinomas with a highly desmoplastic aggressive phenotype.

### Example 2. ERK but not Wnt/β-catenin signaling is activated in DKO^{IEC} intestinal epithelium

Colorectal cancer (CRC) generated through the serrated pathway differs from the conventional adenoma-carcinoma phenotype not only morphologically but also from the signaling and genetic point of view. The genetic differences are characterized by mutations in the Wnt/β-catenin signaling pathway, which are much less frequent in serrated polyps. Notably, neither SSA/P nor adenocarcinoma cells in DKO^{IEC} tumors displayed an accumulation of nuclear β-catenin staining. However accumulation of nuclear β-catenin staining was observed in tumors from Apc^{fl/+};LKO^{IEC} mice (**FIG. 2A** and **FIG. 2B****).** To determine the signaling pathways underlying the phenotype of the DKO^{IEC} intestines, an unbiased transcriptomic analysis (RNA-seq) of intestinal samples from these mice was performed. Interrogation of the transcriptomic data from DKO^{IEC} tumors revealed positive enrichment in *MEK*, *EGFR*, and *KRAS* signatures (F**IG**. **2C).** Immunohistochemistry (IHC) of DKO^{IEC} intestinal tissues demonstrated a strong activation of the *ERK* pathway, as determined by increased staining for phospho-*ERK* and phospho-*EGFR* **(****FIG. 2D****).** Targeted genome sequencing did not identify any alterations in potential coding hot spots for mutations in the murine Braf (B637E) and Kras (G12D, G12V, and G13D) genes, which have been associated with serrated tumorigenesis. This strongly suggests that the simultaneous inactivation of both aPKCs activates the ERK pathway independently of genetic alterations in Braf or Kras. Notably, gene set enrichment analysis (GSEA) of the transcriptomic data from DKO^{IEC} intestinal tissues also revealed activation of the *YAP* pathway **(****FIG. 2E****),** which was confirmed by IHC **(****FIG. 2D****).** The comparison of these transcriptomic data with gene signatures generated from human organoids from serrated lesions, tubular or tubulovillous adenoma, and MSI or MSS CRCs demonstrated that genes upregulated in the DKO^{IEC} tumors positively correlated with the gene signatures of serrated and of MSS CRC (**FIG. 2F****).** Similarly, a gene signature from DKO^{IEC} IECs was significantly upregulated when comparing human serrated tumors to conventional carcinomas, tubular adenomas, adenomatous polyps, or normal colon **(****FIG. 2G****).** These two complementary approaches revealed that DKO^{IEC} tumors are highly similar to human MSS serrated tumors at the transcriptional level.

### Example 3. Intestinal immunosuppression and stromal activation in DKO^{IEC} mice

The combined loss of both aPKCs in IECs promoted a synergistic increase in cell proliferation, as determined with Ki67 staining, that correlated with the spontaneous generation of serrated tumors **(****FIG. 9A****).** LKO^{IEC} intestinal epithelia also displayed increased cell death, as shown by staining for cleaved caspase 3 and TUNEL, but cell death was significantly lower in DKO^{IEC} tumors than in LKO^{IEC} tumors (**FIG. 9B** and **FIG. 9C****).** The reduction in cell death in DKO^{IEC} tumors might explain why these mice developed cancer whereas LKO^{IEC} mice did not, and were unable to go beyond the preneoplastic stage. Notably, there was a robust infiltration of CD45⁺ cells in LKO^{IEC}, but not in ZKO^{IEC}, tissues **(****FIG. 3A****).** Importantly, this infiltration was synergistically increased in DKO^{IEC} SSA/Ps and carcinomas **(****FIG.3A****),** which indicates that the simultaneous loss of both aPKCs in the intestinal epithelium triggers an immunological response that could be critical for the tumorigenic phenotype of DKO^{IEC} mice. Without being bound by a particular theory, these finds suggest that the immune microenvironment in DKO^{IEC} mice is permissive to the development of spontaneous serrated CRC. LKO^{IEC} intestinal tissue displayed significantly higher infiltration of CD8⁺ T cells than WT tissue **(****FIG.3B****),** suggesting that the loss of PKC 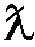 / 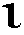 results in the activation of a CD8+-driven immune response. However, CD8+ T cells were excluded from the SSA/P and carcinoma areas of the DKO^{IEC} mice **(****FIG. 3B** and **FIG. 10A****).** There was an observed dramatic expression of Programmed death-ligand 1 (PD-L1) in the CD45+ cells in DKOIEC tumors **(****FIG. 3C****),** in agreement with the notion that the simultaneous loss of both aPKCs leads to a strong immunosuppressive environment, likely conducive to cancer. Flow cytometric analysis of immune cell populations showed that the proportion of CD8+ T cells was increased in LKO^{IEC} intestinal tissue and decreased to normal in DKOIEC tumors **(****FIG. 3D****),** whereas the proportion of PD-L1+/CD45+ cells was specifically increased in DKOIEC tumors **(****FIG. 3E****).** Furthermore, DKO^{IEC} tumors also showed a significant increase in the proportion of Foxp3+ Treg cells **(****FIG. 3F****),** which resulted in a robust enhancement of the Treg/CD8+ ratio in the tumor **(****FIG.3G****).** In addition, a significant increase in the proportion of IL-17A-producing CD4+ T cells (Th17 cells) was observed in DKO^{IEC} tumors **(****FIG. 3H****).** Moreover, these tumors showed a drastic accumulation of CD11b+ myeloid cells (Figure 3I), including both Ly6ChighLy6G-monocytic cells and Ly6C+Ly6G+ polymorphonuclear cells **(****FIG. 3J****).** These populations are reminiscent of monocytic (M-MDSC) and granulocytic (G-MDSC) myeloid-derived suppressor cells, respectively. However, the proportion of total CD4+ T, B220+ B, and NK1.1+ NK cells did not change **(****FIG. 10B****).** These data strongly suggest that DKO^{IEC} tumors exhibit an immunosuppressive microenvironment that inhibits the CD8+ T cell response and therefore allows preneoplastic cells to progress to malignancy through the serrated pathway.

DKO^{IEC} intestinal lesions showed enhanced expression of αSMA, a bona-fide marker of cancer-associated fibroblasts, with the highest expression in carcinomas **(****FIG. 3K****).** This stromal response was accompanied by increased expression and deposition of collagen in tumor areas (FI**G. 3L** and **FIG. 10C****)** and enrichment of TGF-β-dependent transcripts **(****FIG. 10D****).** Moreover, in DKO^{IEC} tumors there was an upregulation of transcripts related to stromal activation and stroma-derived growth factors, such as the ligands for EGFR, Areg, and Ereg **(****FIG. 10E****),** which was confirmed by in situ hybridization for Ereg **(****FIG. 3M****).** Supporting the activated stromal/mesenchymal phenotype of DKO^{IEC} tumors, GSEA of DKO^{IEC} tumor transcripts revealed that the gene signature of the epithelial mesenchymal transition (EMT) was positively enriched **(****FIG. 10F****).** These results, together with the increased immunosuppressive phenotype and inhibition of CD8+ T cell infiltration in DKOIEC tumors, demonstrate that the loss of both aPKCs in IECs results in the induction of an intestinal microenvironment conducive to cancer. This microenvironment is driven through the serrated pathway and has strong desmoplastic and mesenchymal responses.

### Example 4. Intestinal inflammation drives serrated tumorigenesis

Treatment of DKO^{IEC} mice with a combination of broad-spectrum antibiotics, which abolish intestinal inflammation and the immune response strongly decreased the infiltration of CD45⁺ cells as indicated by IHC and FACS analyses **(****FIG. 4A****-****FIG. 4C****).** More importantly, this treatment significantly abrogated tumorigenesis (**FIG. 4D** and **FIG. 4E**). This effect was accompanied by a decrease in the number of Ki67⁺ cells and a reduction in phospho-*ERK* and *YAP* staining **(****FIG. 4F** and **FIG. 4G**). Similar to DKO^{IEC} mice, LKO^{IEC} mice had increased crypt cell proliferation and CD45⁺ cell infiltration, as well as the augmented expression of phospho-*ERK* and *YAP*, which was reverted to normal levels by antibiotic treatment **(****FIG. 11A****-****FIG. 11C****).** However, DKO^{IEC} mice developed spontaneous serrated tumors and LKO^{IEC} mice did not **(****FIG. 1****).** Therefore, although serrated tumorigenesis largely depends on an inflammation-driven process, inflammation is not sufficient. The lack of PKCζ in the DKO^{IEC} intestinal epithelium provides additional alterations that are required for tumor development.

### Example 5. Role of CD8⁺ T cells in tumorigenesis driven by the serrated pathway

If enhanced accumulation of CD8⁺ T cells account for the inability of LKO^{IEC} mice to develop spontaneous tumors, then CD8⁺ depletion *in vivo* should promote serrated tumorigenesis in LKO^{IEC} mice to an extent comparable to that in DKO^{IEC}. Administration of a neutralizing anti-CD8 antibody effectively depleted approximately 70% of infiltrating CD8⁺ T cells **(****FIG. 4H****)** and, importantly, recapitulated the serrated tumor phenotype of DKO^{IEC} mice (**FIG. 4I****-** **FIG.4K**). Similar to DKO^{IEC} tumors, LKO^{IEC} tumors induced by depletion of CD8⁺ T cells displayed the cytomorphologic features of SSA/P, including the development of adenocarcinoma **(****FIG. 4L****),** as well as increased proliferation, reduced cell death, and enhanced stromal activation **(****FIG. 4L** and **FIG. 4M****).** Therefore, the lack of a CD8⁺T cell response in DKO^{IEC} IECs underlies the spontaneous initiation of intestinal serrated tumors. In this context, the fact that cell death is reduced in DKO^{IEC} tumors that are devoid of CD8⁺ T cell infiltration suggests that, whereas increased cell death and the ensuing inflammation are likely important for tumor initiation, cell death has to be reduced for tumors to progress to more malignant stages. Therefore, a break in immunosurveillance contributes to the reduction in cell death in DKO^{IEC} tumors; this allows DKO^{IEC} mice to develop cancer more efficiently than LKO^{IEC} mice, which are unable to go beyond the preneoplastic stage.

### Example 6. Impaired Interferon response in DKO^{IEC} intestinal epithelial cells

To better understand the mechanisms whereby DKO^{IEC} IECs impair the CD8⁺ T cell response and drive serrated tumor initiation, pathways whereby the loss of PKC 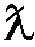 / 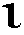 in IECs promotes the CD8⁺ T cell response were explored. One key characteristic of the PKC 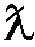 / 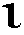 - deficient IECs is increased cell death, which might initiate a CD8⁺ T cell response, linked to immunogenic cell death (ICD). The RNA-Seq data demonstrated the upregulation of several danger signals that conform the ICD response **(****FIG. 5A****),** along with ATP secretion **(****FIG. 5B****).** In keeping with these data, PKC 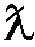 / 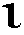 -deficient intestinal epithelial MODE-K cells showed increased apoptosis in response to several ICD stimuli **(****FIG. 5C** and **FIG.12**A**)** suggesting that IECs are more prone to undergo ICD in the absence of PKC 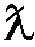 / 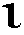 .

The interferon pathway is also a critical cascade responsible for CD8⁺ T cell-mediated anti-tumor immunity. Therefore, the role of PKC 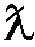 / 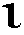 in the interferon response of MODE-K cells upon stimulation with poly(I:C) was explored. Interestingly, MODE-K cells deficient for PKC 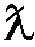 / 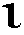 showed a stronger induction of interferon related transcripts including *Cxcl10,* an essential chemokine for CD8⁺ recruitment **(****FIG. 5D****).** Since the interferon response regulates antigen presentation, expression of MHC class I proteins by flow cytometry were analyzed. Consistently, the loss of PKC 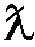 / 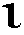 increases significantly the expression of both H-2K^{k} and H-2D^{k} heavy chains at the surface of MODE-K cells **(****FIG. 5E****).** All these data point to a higher CD8⁺ T cell response in the absence of PKC 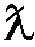 / 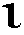 . To strengthen these results, a CD8⁺ T cell mediated cytotoxicity assay was performed using MC38 cells expressing the model antigen ovalbumin (OVA) as target cells and CD8⁺ T cells isolated from OT-I mice as specific effector cells. Importantly, the absence of PKC 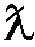 / 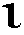 in MC38 cells increased significantly the OT-I cells mediated killing **(****FIG. 5F****)** demonstrating that the loss of PKC 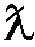 / 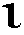 in intestinal epithelial cells promotes the CD8⁺ T cell response, which is instrumental to prevent tumor initiation.

To determine how the loss of PKCζ impairs the CD8⁺ T cell response induced by the loss of PKC 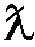 / 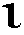 , analysis of the transcriptomic data was performed. The analysis revealed that the interferon alpha and interferon gamma response genes were highly downregulated gene signatures in the DKO^{IEC} tumors as compared with WT tissues **(****FIG. 12B****).** However, this analysis did not establish whether the defect in the interferon pathway is a direct consequence of the deletion of the aPKCs in the IECs or whether it involves another infiltrating cell type in the tumor. Transcriptomic analysis of IEC samples from WT, LKO^{IEC}, ZKO^{IEC} and DKO^{IEC}
mice, showed genes involved in the interferon gamma response as being the most downregulated gene signatures in DKO^{IEC} Furthermore, these two signatures were also the most downregulated in ZKO^{IEC} IECs when compared with WT IECs **(****Figure 5G****).** A decrease in interferon-responsive genes was also observed in ZKO^{IEC} IECs when compared with WT, and in DKO^{IEC} compared with LKO^{IEC} **(****FIG. 5H** and **FIG. 12D****).** These data establish that the loss of PKCζ contributes to serrated tumorigenesis by suppressing the interferon cellular response in a cell autonomous manner.

A series of *ex vivo* experiments using 3D cultures of organoids was performed that either contained (WT) or lacked (ZKO) PKCζ. DNA methyltransferase inhibitors (DNMTi) have recently been used to transcriptionally upregulate human endogenous activators of the double-stranded RNA sensing pathway that culminates in the stimulation of the Type I and III interferon responses. Both types of organoids were incubated with the DNMTi 5-aza-2-deoxycytidine (5-AZA-CdR) for 24 hours, followed by cell culture in the absence of this drug. Importantly, the 5-AZA-CdR treatment robustly induced the expression of the key interferon-responsive genes *Irf7, Oas1a, Isg15,* and *Cxcl10* in WT organoids, and this expression was severely abrogated in ZKO organoids (FIG. 5I). These results demonstrate that PKCζ in IECs is required for the endogenous activation of the interferon pathway ex *vivo.* Importantly, the genetic deletion of PKCζ in the context of PKC 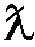 / 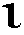 -deficient cells severely inhibited the expression of interferon transcripts **(****FIG. 5J****)** and that of key elements in the interferon signaling cascades **(****FIG. 5K****)** that are hyperactivated in PKC 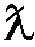 / 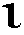 -deficient cells. Negative enrichment of the "ALLOGRAFT REJECTION" signature in the ZKO^{IEC} IECs **(****FIG.5G****)** was also observed. NextBio analysis of genes downregulated in the ZKO^{IEC} IECs revealed significant correlations with the GO term "antigen processing and presentation of peptide antigen via MHC class I" **(****FIG. 5L****).** Accordingly, the stimulation with 5-AZA-CdR upregulated MHC class I long-chain H-2K^{b} on the surface of WT organoids, but not on ZKO organoids **(****FIG. 5M****),** demonstrating a defect in antigen presentation under PKCζ-deficient condition. Moreover, IHC analysis demonstrated in intestinal tissues the upregulation of MHC class I marker in LKO^{IEC} mice that was completely abrogated in DKO^{IEC} mice **(****FIG. 5N****).**

Collectively, these results establish that PKCζ is a critical player in the control of the interferon response in IECs and support the notion that an impaired interferon response in the DKO^{IEC} mice accounts for the defect in CD8⁺ infiltration and the spontaneous development of intestinal serrated tumors.

### Example 7. Therapeutic interventions in serrated tumors driven by aPKC loss

To therapeutically exploit the increased infiltration of DKO^{IEC} tumors by PD-L1⁺/CD45⁺ cells, DKO^{IEC} mice were treated with an anti-PD-L1 antibody for 3 weeks, starting at 10 weeks of age **(****FIG. 13A****).** This treatment resulted in a robust reduction in the number, average size, and total load of tumors, as well as in cancer incidence, which correlated with a concomitant increase in the recruitment of CD8⁺ cells into the tumor areas **(****FIG. 13B****-****FIG. 13D****).** However, anti-PD-L1 treatment did not show any therapeutic effect when mice were treated starting at 13 weeks of age **(****FIG. 13E****),** at which point these mice presented with a more advanced tumor burden and increased tumor aggressiveness **(****FIG. 13F****-** **FIG. 13H****).** Thus, once serrated tumors are established and become more aggressive, they become resistant to anti-PD-L1 therapy. One of the characteristics of the advanced DKO^{IEC} tumors is a high desmoplastic response, which has been proposed to be a critical mechanism for CRC progression towards high malignancy and invasion and poor patient survival. Notably, tumors from 16-week-old DKO^{IEC} mice showed increased expression of αSMA and collagen deposition when compared with tumors from 13-week-old mice (FIG. 13I), suggesting the potential contribution of an activated stroma to the resistance of DKO^{IEC} tumors to therapy.

Next, whether the strong stromal response of serrated DKO^{IEC} tumors might be central to tumor progression and the acquisition of a more aggressive phenotype was investigated. Because stromal activation in CRC relies on TGF-β signaling, we treated 10-week-old DKO^{IEC} mice with the TGF-βR1-specific inhibitor galunisertib (LY2157299) **(****FIG. 6A****).** Although this treatment did not reduce the tumor number, size, or load in the DKO^{IEC} mice **(****FIG. 6B****),** it suppressed the progression of SSA/P to the adenocarcinoma stage, and especially reduced the incidence of invasive cancer **(****FIG. 6C** and **FIG. 6D****).** Consistent with the well-established role of TGF-β in stromal activation, treatment with galunisertib resulted in the inhibition of stromal phospho-SMAD2 and collagen deposition **(****FIG. 6D****),** as well as in the reduced expression of TGF-β-responsive genes, such as *Angptl2*, *Cdkn2b, Il11*, and *Ereg* **(****FIG. 6E****).** Moreover, flow cytometric analyses demonstrated a reduction in the proportion of Th17 cells in the tumors upon this treatment (FIG. 6F), which is consistent with the crucial role of TGF-β in Th17 cell differentiation. However, this treatment did not significantly change the proportions of CD8⁺ T, Treg, or CD11b⁺ myeloid cells **(****FIG. 6G-****FIG. 6J****).**

The proportion of PD-L1⁺/CD45⁺ cells infiltrating DKO^{IEC} tumors was not affected by galunisertib **(****FIG. 6K** and **FIG. 6L****),** which could explain why this treatment did not restore the infiltration of CD8⁺ T cells. It also suggests that the activated stroma contributes to the tumorigenesis of DKO^{IEC} mice by promoting tumor progression but is not critical for the initiation of serrated tumors, which is most likely mediated by inactivation of the CD8⁺ T cell response. Of potential relevance from a therapeutic point of view, when a parallel cohort of 23-week-old DKO^{IEC} mice were treated with a combination of galunisertib and anti-PD-L1 **(****FIG. 6M****),** there was a dramatic reduction in the number, size, load, and aggressiveness of tumors **(****FIG. 6N****-** **FIG. 6O****),** with a concomitant increase in CD8⁺ T cell infiltration into the tumors **(****FIG. 6P** and **FIG. 6Q****).** However, there were no changes in the proportion of Treg cells, although there was a significant reduction in CD11b⁺ myeloid cells **(****FIG. 6R** and **FIG. 6S****).** These results demonstrate that, although anti-PD-L1 therapy is not effective in more advanced and desmoplastic serrated cancer, these cancers are rendered sensitive to anti-PD-L1 treatment when the progression of SSA/P to adenocarcinoma is blunted by suppressing TGF-β signaling.

### Example 8. Human serrated tumors display reduced expression of aPKCs

To establish the relevance of the aPKC loss in human serrated tumors, polyps that were pathologically classified as SSA/P or tubular adenoma (TA) were analyzed. SSA/Ps are considered to be the epithelial precursor lesions that will progress towards serrated colorectal cancer (CRC), whereas TAs follow the conventional CRC. An antibody that recognizes both aPKCs **(****FIG. 14A****)** was ised to immunostain 30 SSA/P and 30 TA samples, along with 20 controls from healthy individuals. The aPKC levels were significantly reduced only in SSA/P and not in TA samples **(****FIG. 7A** and **FIG. 7B****),** which established the relevance of the aPKC loss in human serrated tumors.

To determine whether the low levels of both aPKCs correlated with serrated features in CRC samples, bioinformatics was used to interrogate the large dataset from patients with colorectal adenocarcinoma in The Cancer Genome Atlas Network (TCGA). Because these CRC samples are genetically and morphologically heterogeneous and are not annotated for their precursor origin through the serrated or conventional pathway, patients were first stratified patients based on aPKC expression and performed GSEA with serrated or traditional adenoma gene signatures. Patients were classified according to the median expression of *PRKCI* and *PRKCZ.* This analysis demonstrated a significant positive enrichment of serrated gene signatures in the *PRKCI*^{Low}*PRKCZ*^{Low} group as compared with the *PRKCI*^{Hi}*PRKCZ*^{Hi} group **(****FIG. 7C****).**

Next, CRC patients were classified based on molecular profiles using the CCS (colon cancer subtypes) classification. This classification includes three subgroups: CCS 1 represents chromosomal unstable; CCS2 is MSI/CIMP⁺; and CCS3 is largely MSS and relates to serrated tumors, including those with upregulation of stromal and EMT genes, and defines a subset of patients with the worst survival. The mRNA levels of both aPKCs were significantly reduced in the CCS3 subtype **(****FIG. 7D****,** **FIG. 14B,** and **FIG. 14C****).** Also, samples stratified by the expression of both aPKCs showed a marked segregation of patients by CCS classification. The CCS3 subtype was predominantly made up of the *PRKCI*^{Low}*PRKCZ*^{Low} group **(****FIG. 7E****,** **FIG. 14D,** and **FIG. 14E****),** which was associated with a worse prognosis than the *PRKCI*^{Hi}*PRKCZ*^{Hi} subgroup **(****FIG.7F** and **FIG. 14F****),** in keeping with the unfavorable prognosis associated with CCS3 patients. Stratification of TCGA patients according to their aPKC levels also validated the critical role of these kinases in the negative regulation of the EMT and of the KRAS and YAP pathways, as well as in TGF-β signaling activation **(****FIG.7G****).** Furthermore, analysis of CRC patients according to their *KRAS* or *BRAF* mutational status revealed the existence of a subset characterized as *PRKCI*^{Low}*PRKCZ*^{Low} that were also WT for *BRAF* and *KRAS* **(****FIG. 14G****).** GSEA of the transcriptomes of these patients showed positive correlation with upregulated serrated and downregulated adenoma signatures, and a positive enrichment in EMT and inflammatory signatures **(****FIG. 14H****),** in excellent agreement with the phenotype of the DKO^{IEC} tumors. *PRKCI*^{Low}*PRKCZ*^{Low} samples that also had mutations in either *KRAS or BRAF* displayed a more mesenchymal and inflammatory phenotype than those that were mutant for either of these two oncogenes but were *PRKCI*^{Hi}*PRKCZ*^{Hi} (FIG. 14I and **FIG. 14J****).** These results demonstrate that the simultaneous loss of both aPKCs in human CRCs generates an additional phenotype to that of the serrated *KRAS* or *BRAF* mutant group that is characterized by a stromal and inflammatory response. Therefore, the poorer prognosis of *PRKCI*^{Low}*PRKCZ*^{Low} patients **(****FIG. 7F** and **FIG.14F****)** could be accounted for by the EMT/inflammatory environments created by the inactivation of both aPKCs.

The negative correlation between aPKC levels and the inflammatory response in CRC patients of the *PRKCI*^{Low}*PRKCZ*^{Low} group **(****FIG. 7G** and **FIG. 14H****-****FIG. 14J****)** suggested a potential link between human intestinal inflammation and serrated tumorigenesis. Human inflammatory bowel disease (IBD) datasets that included data from both Crohn's disease (CD) and ulcerative colitis (UC) patients were interrogated. The majority of datasets showed reduced levels of both aPKC transcripts, along with increased enrichment in the serrated, EMT, and inflammatory gene signatures in both types of IBD patients as compared with healthy individuals **(****FIG. 7H****).** In contrast, the CCS3 subtype displayed an immunosuppressive phenotype characterized by lower levels of immune stimulatory molecules (especially when compared with the CCS2 [MSI/CIMP⁺] subtype) and increased levels of immunoinhibitory molecules, both of which were associated with reduced levels of *PRKCI* and *PRKCZ* **(****FIG. 14K** and **FIG. 14L****).** The results from DKO^{IEC} tumors described above demonstrated an immunosurveillance defect that accounts for serrated tumor initiation in the DKO^{IEC} mouse model, characterized by the exclusion of CD8⁺ T cells from the DKO^{IEC} tumors. In order to validate this phenotype in human patients, we used anti-CD8⁺ and anti-aPKC antibodies to immunostain a cohort of human CRC samples collected from proximal colons, where it is well established that SSA/Ps and serrated cancers are frequently located. Notably, CRC samples with low levels of aPKCs showed significantly lower infiltration of CD8⁺ T cells than those with high aPKC expression levels (FIG. 7I and **FIG. 7J****).** Collectively, these results strongly support the notion that chronic inflammation is critical for the development of CRC through the serrated pathway, and that the loss of aPKC is a central event in that process and in the development of an immunosuppressive phenotype.

### Example 9. General Methodology

### Mice

*Prkcz*^{fl/fl}, *Prkci*^{fl/fl}, *Apc*^{fl/fl}, Villin-cre and Lgr5-EGFP-ires-creERT2 (Lgr5-creERT2) mice were acquired in accordance with procedures set forth in (Llado et al., Repression of Intestinal Stem Cell Function and Tumorigenesis through Direct Phosphorylation of β-catenin and Yap by PKCζ. Cell Reports (2015) 10,740-754; Nakanishi et *al*., Control of Paneth Cell Fate, Intestinal Inflammation, and Tumorigenesis by PKC 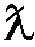 / 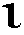 . Cell Reports (2016) 16, 3297-3310. For CD8+ T cell mediated cytotoxicity assay **(****FIG. 5****),** OT-I TCR Tg (OT-I) mice (Jackson Laboratory) were used between 8 and 12 weeks of age. All mouse strains were generated in a C57BL/6 background, and were born and maintained under pathogen-free conditions. Mice were sacrificed, and small intestines and colons were collected for analyses. Animal handling and experimental procedures conformed to institutional guidelines and were approved by the Sanford Burnham Prebys Medical Discovery Institute Institutional Animal Care and Use Committee. All genotyping was done by PCR. Age- and sex-matched mice were used for all experiments. For antibiotic treatment, 10-week-old DKO^{IEC} or LKO^{IEC} mice were administered a combination of ampicillin (1 g/l), neomycin (1 g/l), metronidazole (1 g/l), and vancomycin (0.5 g/l) in drinking water for 6 or 4 weeks, respectively, until they were sacrificed.

For CD8+ T cell depletion **(****FIG. 4****),** 10-week-old LKO^{IEC} mice were injected intraperitoneally with 200 µg of anti-CD8 antibody (clone 2.43) or control IgG twice a week for 5 weeks until they were sacrificed. For the TGF-βR1-specific inhibition experiment, 10-week-old DKO^{IEC} mice were treated twice a day with a dose of 10 mg of galunisertib (Tauriello et al., 2018) per os for 6 weeks until they were sacrificed. Control mice were treated with vehicle. For checkpoint immunotherapy, either 10-week-old or 13-week-old DKO^{IEC} mice were injected intraperitoneally with a dose of 10 mg/kg (body mass) of anti-PD-L1 antibody (clone 6E11) twice a week for 3 weeks until they were sacrificed. For the combination therapy of anti-PD-L1 antibody and TGF-βR1-specific inhibitor, 23-week-old DKO^{IEC} mice were treated twice a day with a dose of 10 mg of galunisertib per os and injected intraperitoneally with a dose of 10 mg/kg (body mass) of anti-PD-L1 antibody twice a week for 3 weeks until they were sacrificed. Anti-PD-L1 antibody was provided by de Sauvage FJ (Genentech).

### Human Samples

Tissue samples were collected during a colonoscopy or surgery from the colon of 20 normal healthy individuals, 30 sessile serrated adenoma/polyp, 30 tubular adenoma, 25 proximal colon cancer. Written informed consent was obtained from all patients with the protocol approved by the Ethics Committee of Scripps Green Hospital. De-identified samples were sent to Sanford Burnham Prebys (SBP) Medical Discovery Institute and used for histological analyses. The study was approved by the IRB Committee of SBP Medical Discovery Institute.

### Cell culture experiments

MODE-K cells, 293T and MC38OVA cells were cultured in DMEM (Cellgro, #15-017-CV) supplemented with 10% FBS, 2 mM of Glutamine and 100 U/ml penicillin and 100 µg/ml streptomycin, in an atmosphere of 95% air and 5% CO2. For ATP measurement and immunogenic cell death western blots, cells were treated with Oxaliplatin (20 µM), Bortezomib (0.1 µm) or 10 ng/ml of TNFα (Sigma) and 2.5 µg/ml of CHX (Sigma) for 24 hours. Extracellular ATP levels were measured by the luciferin-based ATPlite Assay (e.g., Perkin Elmer) using Varioskan Lux reader (*e.g*., Thermo Fisher, as indicated by the manufacturer). For poly(I:C) treatment, cells were transfected with 0.5 ug/ml of poly(I:C) (*e.g.,* InVivoGen) using Lipofectamine 2000 *(e.g.,* Invitrogen). Cells for protein analysis were lysed in RIPA buffer (20 mM Tris-HCl, 37 mM NaCl, 2 mM EDTA, 1% Triton-X, 10% glycerol, 0.1% SDS, and 0.5% sodium deoxycholate), with phosphatase and protease inhibitors. Cell extracts were denatured, subjected to SDS-PAGE, transferred to polyvinylidene difluoride (PVDF) membranes (e.g., GE Healthcare), and immunoblotted with the specific antibodies as listed in STAR methods Reagents table.

To knock down PKCζ in 293T sgPKC 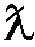 / 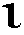 cells and PKC 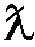 / 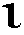 in MC38OVA cells, TRC lentiviral shRNA targeting human PKCζ (TRCN0000001219) and TRC shRNA targeting mouse PKC? / 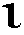 (TRCN000022757) were obtained from Open Biosystems. shRNA- encoding plasmids were co-transfected with psPAX2 (e.g., Addgene plasmid #12260) and pMD2.G *(e.g.,* Addgene plasmid #12259) packaging plasmids into actively growing 293T cells by using XtremeGene HP transfection reagent *(e.g.,* Roche). Virus-containing supernatants were collected 48 hour after transfection, filtered to eliminate cells, and then used to infect the target cells in the presence of 8 µg/ml polybrene *(e.g.,* Millipore). Cells were analyzed after puromycin selection (1µg/ml) to confirm knockdown. To knock out PKCζ in 293T sgPKC 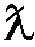 / 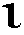 cells, lentiviral guide RNA plasmid targeting human PKCζ (BRDN0001149519) and lentiviral vector expressing Cas9 and blasticidin resistance were obtained from Addgene. Cells were treated virus-containing supernatants and selected with puromycin and blasticidin. Colonies from single cell were expanded and screened for PKCζ by immunoblotting. To knock out PKC 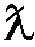 / 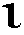 in MODE-K cells, a 20-nucleotide single-guide RNA (sgRNA) sequence targeting mouse PKC 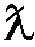 / 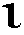 was designed using the CRISPR design tool. The sgRNA was cloned in the lentiCRISPR v2 vector and transduced into MODE-K cells. Cells were selected with puromycin and colonies from single cell were expanded and screened for PKC 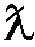 / 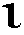 by immunoblotting.

### Histology, immunohistochemistry, immunofluorescence, and in situ hybridization

Organs were isolated, rinsed in ice-cold PBS, fixed in 10% neutral buffered formalin overnight at 4 °C, dehydrated, and embedded in paraffin. Sections (5 µm) were stained with hematoxylin and eosin (H&E). For immunohistochemistry, sections were deparaffinized, rehydrated, and then treated for antigen retrieval. After blocking in Protein Block Serum-Free solutions (DAKO), tissues were incubated with primary antibody overnight at 4 °C followed by incubation with biotinylated secondary antibody. Endogenous peroxidase was quenched in 3% H2O2 in water for 10 min at room temperature. Antibodies were visualized with avidin/biotin complex *(e.g.,* Vectastain Elite; Vector Laboratories) using diaminobenzidine as the chromagen. For immunofluorescence, fresh frozen organs were embedded in Tissue-Tek OCT compound *(e.g.,* Sakura) and section of 10 µm were fixed in methanol, washed with PBS and then incubated with the primary antibodies. Washed sections were incubated with Alexa-conjugated secondary antibodies *(e.g.,* Life Technologies) and examined with a Zeiss LSM 710 NLO Confocal Microscope. In situ hybridization for murine Ereg was carried out using RNAscope Multiplex Fluorescent Assay kit (Advanced Cell Diagnostics) following manufacturer's instructions. The probe corresponding to CDS region of murine Ereg (Accession #: NM_007950.2, probe region: 116-1496; Advanced Cell Diagnostics; Cat#: 437981). Cell death was analyzed using the In-Situ Cell Death Detection kit, TMR red (Thermo) for TUNEL. Masson's Trichrome staining was carried out following manufacturer's instructions (Sigma HT15-1KT).

### Flow cytometry analysis

Flow cytometry experiments were performed using fresh small intestinal tissue and organoid preparations. For the analyses of small intestinal tissue, a half part of duodenum, jejunum, and ileum was used for quantitative flow cytometry analysis of immune cell population of lamina propria. Briefly, small intestines were rinsed in cold PBS, minced into small pieces, incubated in HBSS with EDTA (5 mM) and digested with collagenase (0.05 mg/ml) for 30 min at 37 °C, followed by a discontinuous Percoll separation method (40 and 75%) to purify immune cells. The cells concentrated at the interface were collected and washed in cold PBS. Red blood cells were removed with lysing buffer (BD Pharm Lyse) and live cells were counted using Trypan blue and then saturated with mouse Fc Block (purified anti-mouse CD16/CD32; 1:50; clone 2.4G2; BD Pharmingen) 30 min at 4°C before incubating with specific dyed antibodies.

For IL-17A intracellular staining, cells were pretreated with 50 ng/ml of phorbolmyristate acetate and 500 ng/ml of ionomycin in the presence of 10 µg/ml of brefeldin A for 3 hr at 37°C 5% CO2. Cells were then stained with specific extracellular antibodies, permeabilized with Fixation/Permeabilization solution kit *(e.g.,* BD Cytofix/Cytoperm) according to manufacturer's instructions and finally incubated with anti-IL-17A (clone TC11-18H10; BD Pharmingen) specific antibody in Perm/Wash buffer overnight at 4°C. For FOXP3 intracellular staining, cells were stained with specific extracellular antibodies, permeabilized with the same BD Cytofix/Cytoperm kit and finally stained with anti-FOXP3 (clone FJK-16s) specific antibody in Perm/Wash buffer overnight at 4°C.

For generation of small intestine organoids, organoids were collected in ice-cold TrypLE solution *(e.g.,* Life Technologies) to remove Matrigel, then pipetted 5 cycles and incubated 5 min at 37°C to obtain single-cell suspension. Cells were washed twice in PBS 3% FBS and resuspended at room temperature with PBS with 100 µg/ml DNase I for 10 min before staining with anti-H-2Kb (clone AF6-88.5.5.3; eBioscience). For antigen presentation in MODE-K cells, cells were washed in PBS, trypsinized and wash with warm medium. Then, cells were resuspended and stained with anti-H-2Kk (clone 36-7-5; BioLegend) and anti-H-2Dk (clone 15-5-5; BD Pharmingen). Dead cells were excluded after 7-AAD staining. All flow cytometry experiments were performed on a BD LSRFortessa 14-colors analyzer at the Sanford Burnham Prebys Medical Discovery Institute Shared FACS Facility and flow cytometry data were analyzed using FlowJo software (Tree Star Inc.).

For T cell killing assay of MC38OVA cells, CD8+ T cells were isolated from the spleen of OT-I mice by magnetic sorting using EasySep Mouse CD8+ T Cell Isolation Kit *(e.g.,* Stemcell Technologies) according to the manufacturer's instructions. To generate effector cells, naive T cells were cultured at 106/mL for 3 days with immobilized anti-CD3 (10 µg/mL) and soluble anti-CD28 (5 µg/mL) (Bio X Cell). After 3 days, effector CD8+ T cells were co-cultured with 1x104 OVA-expressing MC38 target cells (shNT and sh 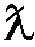 / 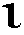 ) or MC38 target cells at different ratios (2: 1; 1:1; 1:2; 1: 10; 1:20; 1:50) for 24 hours at 37°C, 5% CO2. MC38 cell line was used as a negative control. The supernatants were harvested and analyzed using a Pierce LDH Cytotoxicity Assay Kit (Thermo Scientific) to determine the specific cytotoxicity according to the manufacturer's instructions. For the PI staining, 100µM propidium iodide was added to the culture medium when the OT-I cells were added. PI signal was measured 24 hour after coculture with OT-I cells using a Varioskan LUX multimode microplate reader (e.g., ThermoFisher Scientific).

### Intestinal epithelial cell isolation and small intestinal organoid culture

Intestinal epithelial cell (IEC) isolation was performed as described previously Llado et al., Repression of Intestinal Stem Cell Function and Tumorigenesis through Direct Phosphorylation of β-catenin and Yap by PKCζ. Cell Reports (2015) 10,740-754, with some modification. Briefly, small intestines were removed, washed with cold PBS without magnesium chloride and calcium (PBS-/-) opened longitudinally, cut into 5 mm fragments, and then washed several times with cold PBS-/- until clean. Cut tissue fragments were incubated with PBS-/- EDTA (5 mM) containing 10% FBS for 40-60 min at 4°C. IECs were then mechanically separated from the connective tissue by rigorous shaking, and then filtered through a 100-µm mesh into a 50 ml conical tube to remove tissue fragments. Isolated IECs were pelleted and then lysed for RNA or protein extraction. For small intestine organoid culture, we collected small intestinal adenoma tissues from Lgr5-creERT2; Apc^{fl/fl} (WT) or Lgr5-creERT2;Apc^{fl/fl};Prkcz^{fl/fl} (ZKO) mice injected with tamoxifen (e.g., Sigma) intraperitoneally. Isolated adenoma tissues were minced into small pieces and incubated in digestion buffer (Dulbecco's modified Eagle medium with 10% FBS, penicillin/streptomycin, 1 mg/ml collagenase type Xl, 0.1 mg/ml DNase l, 10 µM Y-27632) for 15-20 minutes at 37°C. The digested adenoma fragments were washed with PBS-/- and then filtered through a 100-µm mesh into a 50-ml conical tube to remove stromal fragments. Adenoma fragments were counted after isolation and a total of 250-500 fragments were mixed with 100 µl of growth factor reduced-Matrigel and plated in 12- or 24-well plates. After polymerization of Matrigel, 1 ml of culture medium (Advanced DMEM/F12 containing 10 mM HEPES, 1X Glutamax, 1X B27 supplement, 50 ng/ml EGF and 10 µM Y-27632) was added. For experiment to examine the interferon response, organoids were treated with 3 µM of 5-AZA-CdR (Sigma). After 24 hr incubation, the medium was replaced with fresh drug-free medium, and then organoids were cultured for an additional 3 days for RNA or flow cytometry analyses.

### Microsatellite instability y (MSI) Analysis

For examination of microsatellite instability (MSI) five microsatellite repeat markers were amplified by PCR using DNA isolated from WT and DKO^{IEC} mouse tissues. 10 ng of DNA was amplified with FAM-labeled forward primers and reverse primers provided in **Table 1** specific for the previously validated murine microsatellite markers Bat24, Bat26, Bat30, Bat37, Bat64. Hi-Di and LIZ size standard were added to the samples and separation and detection of amplified fragments was performed on the 3730xl DNA Analyzer (Eton Bioscience). Data were analyzed with Peak Scanner^{™} Software from Applied Biosystems to identify the allelic patterns for each marker. Allelic patterns for normal and tumor tissues were compared and scored based on the size of the predominant allele and the presence of allelic variants. Tumor samples with greater or equal 40% MSI were classified as MSI-high (MSI-H), less than 40% as MSI-low (MSI-L), and samples without alterations were classified as microsatellite stable (MSS).

### Table 1. Exemplary Primer Sequences for MSI Analysis

| **Gene Symbol** | **Forward Primer Sequence** | **Reverse Primer Sequence** |
|---|---|---|
| *Bat24* | 6FAM-CATAGACCCAGTGCTCATCTTCGT | CATTCGGTGGAAAGCTCTGA |
| *Bat26* | 6FAM-TCACCATCCATTGCACAGTT | CTGCGAGAAGGTACTCACCC |
| *Bat30* | 6FAM-ATTTGGCTTTCAAGCATCCATA | GGGAAGACTGCTTAGGGAAGA |
| *Bat37* | 6FAM-TCTGCCCAAACGTGCTTAAT | CCTGCCTGGGCTAAAATAGA |
| *Bat64* | 6FAM-CCCACACTCCTGAAAACAGTCAT | CCCTGGTGTGGCAACTTTAAGC |

### RNA extraction and analysis

Total RNA from mouse tissues and cultured organoids was extracted using the TRIZOL reagent *(e.g.,* Invitrogen) and the RNeasy Mini Kit (e.g., Qiagen), followed by DNase treatment. After quantification using a Nanodrop 1000 spectrophotometer *(e.g.,* Thermo Scientific), RNA was either processed for RNA-seq or reverse-transcribed using random primers and MultiScribe Reverse Transcriptase *(e.g.,* Applied Biosystems). Gene expression was analyzed by amplifying 20 ng of the complementary DNA using the CFX96 Real Time PCR Detection System with SYBR Green Master Mix (BioRad) and primers described in **Table 2.** The amplification parameters were set at 95°C for 30 seconds, 58°C for 30 seconds, and 72°C for 30 seconds (40 cycles total). Gene expression values for each sample were normalized to the 18S RNA.

**Table 2. Exemplary Primer Sequences for qRT-PCR Analysis**

| **Gene Symbol** | **Forward Primer Sequence** | **Reverse Primer Sequence** |
|---|---|---|
| *Angptl2* | GGAGGTTGGACTGTCATCCAGAG | GCCTTGGTTCGTCAGCCAGTA |
| *Cdkn2b* | ACGCTGCCACTGGAGATTG | ATGGGGCTGGGGAGAAAGA |
| *Il11* | CTGCACAGATGAGAGACAAATTCC | GAAGCTGCAAAGATCCCAATG |
| *Ereg* | ATCCGAGGATAACTGTACCG | TCACATCGCAGACCAGTGTAG |
| *Irf7* | ATGCACAGATCTTCAAGGCCTGGGC | GTGCTGTGGAGTGCACAGCGGAAGT |
| *Oas1a* | GAGGTTCAGCATGAGAGACGTT | ACACAGTTGGTACCAGTGCTTG |
| *Isg15* | TGGGACCTAAAGGTGAAGATGCTG | TCAGGCGCAAATGCTTGATCAC |
| *Cxcl10* | GGATCCCTCTCGCAAGGA | ATCGTGGCAATGATCTCAACA |
| *18s* | GTAACCCGTTGAACCCATT | CCATCCAATCGGTAGTAGCG |
| *Ifnb* | TTGCCATCCAAGAGATGCTCCAGA | AGAAACACTGTCTGCTGGTGGAGT |
| *Nlrc5* | TGGAGGAGGTCAGTTTGC | ATGCTCCTGATTGCTGTGTAG |
| *CXCL10* | GTGGCATTCAAGGAGTACCTC | GCCTTCGATTCTGGATTCAG |
| *IFNB* | AGGACAGGATGAACTTTGAC | TGATAGACATTAGCCAGGAG |

### RNA sequencing (RNA-seq)

For RNA-seq of whole small intestinal tissues, total RNA was extracted from 3 WT jejunums, 3 DKO^{IEC} non-tumor jejunums, and 3 DKOIEC tumors. For RNA-seq of intestinal epithelial cells (IECs), total RNA was extracted from normal jejunum IECs of 3 WT, 4 LKO^{IEC}, 3 ZKO^{IEC}, and 4 DKO^{IEC} mice. Each sample was extracted from independent mice. RNA-seq studies were performed in the Genomics Core at SBP Medical Discovery Institute. Briefly, poly(A) RNA was isolated using the NEBNext^{®} Poly(A) mRNA Magnetic Isolation Module and barcoded libraries were made using the NEBNext^{®} UltraTM Directional RNA Library Prep Kit for Illumina^{®} (NEB, Ipswich MA). Libraries were pooled and single end sequenced (1X75) on the Illumina NextSeq 500 using the High output V2 kit (Illumina). Sequencing Fastq files were uploaded to BaseSpace and processed with RNAexpress App (Illumina) to obtain raw reads counts for each gene. The gene matrix files were generated with RNAexpress App and compared using T-test comparison and log2 ratio of classes.

### Bioinformatics analyses

Raw gene expression data from serrated tumor (GSE4045, GSE76987, GSE79460, GSE43841, GSE36758, GSE46513, GSE45270), CRC (GSE5851, GSE33113), and inflammatory bowel disease sample datasets (GSE36807, GSE59071, GSE 10616, GSE10714, GSE52746, GSE6731, and GSE9386) were directly accessed through the Gene Expression Omnibus (GEO). RNS-seq data of The Cancer Genome Atlas (TCGA) Colorectal Adenocarcinoma (Tumor Samples with mRNA data (RNA Seq V2), 382 tumor samples from 379 patients) was downloaded through cBioportal. GenePattern was used to collapse gene matrix files or to assess the statistical significance of differential gene expression. Heat-map representation of gene expression was generated using GENE-E sotware. Gene Set Enrichment Analysis (GSEA) was performed using GSEA 3.0 software with 1000 gene-set permutations using the gene-ranking metric T-test with the collections h.all.v6.1.symbols (H), c2.all.v6.1.symbols (C2), c6.all.v6.1.symbols (C6), or customized gene sets. **Table 3** shows a gene list comprising genes associated with the microsatellite stable colorectal cancer phenotype (MSS CRC). **Table 4** provides a gene signature comprising genes upregulated in MSS CRC, serrated lesions, and tubular or tubulovillous adenomoa. **Table 5** provides a gene signature comprising genes downregulated in MSS CRC, serrated lesions, and tubular or tubulovillous adenomoa. **Table 6** provides a gene signature that was generated based on a list of top 50 gene transcripts increased by RNA-seq in sessile serrated polyps (SSA/P) in serrated polyposis patients compared to controls. **Table 7** provides a genet signature that was generated based on a list of gene transcripts significantly increase by RNA-seq in DKO^{IEC} IECs compared to WT IECs (logFC>1, FDR<0.01).

**Table 3. Microsatellite stable (MSS) colorectal cancer (CRC) gene signature**

| | | | | |
|---|---|---|---|---|
| FLNB | EPB41L4A | MYCN | CCDC3 | NTRK2 |
| GLCE | ACTA2 | PALLD | CXCL14 | LPAR6 |
| CCDC146 | PHLDB2 | ARAP2 | UCA1 | CHRNB1 |
| GTF2IRD2 | ISM1 | MYOF | C16orf62 | ANXA1 |
| INADL | KITLG | ARHGAP29 | HSPA2 | CAPN8 |
| DOCK5 | PTPRD | MT1E | SPATA18 | EDN1 |
| MACF1 | SLC7A8 | BICC1 | MPZL2 | TSPAN2 |
| AKAP7 | RASGRF1 | ABCC3 | FHL2 | KLK10 |
| TTC19 | YPEL1 | NRXN3 | CSGALNACT1 | PDE4D |
| MAP1B | UBASH3B | C19orf45 | RUNX1 | CYP4X1 |
| PTK7 | VSIG2 | SFRP5 | ID4 | ATP8A1 |
| BIK | MYCL1 | REN | ACTG1P4 | TWSG1 |
| SHROOM3 | IL8 | NEDD9 | NDP | MYLK |
| KLK7 | TCF7 | APCDD1 | CD200 | TGFB3 |
| RHBDL2 | TLR4 | CXCL6 | PDE9A | TRIM22 |
| PLK2 | ACAA2 | C6orf15 | SLCO1B3 | FSTL1 |
| EPM2AIP1 | TET2 | INPP4B | TUBA1A | GTF2IRD2B |
| AHNAK | ENPP3 | SGPL1 | ANXA3 | GTF2IRD2P1 |
| PSTPIP2 | ST6GAL2 | KIT | IL17RD | |
| NMU | CFTR | TMEM211 | PTCHD4 | |

**Table 4. Gene signature upregulated in MSS-CRC, serrated lesions, and tubular or tubulovillous adenoma**

| ZIC5 | PCSK7 | CEACAM 6 | CCDC68 | FAM214 B | SAMD9 | CCRL1 |
|---|---|---|---|---|---|---|
| SDR16C5 | FKBP1A-SDCBP2 | ANXA1 | OBFC1 | SLC16A5 | BTNL3 | IL1RN |
| SLCO1B3 | IL18 | C Worf 129 | CA2 | LRRC66 | CD55 | DHRS9 |
| CIDEC | SLC16A4 | DSG3 | VPS13D | GCNT3 | INSC | NR1H4 |
| AHCYL2 | ANTXR2 | CAPN8 | ABCG2 | TSPAN1 | SULT1C2 | TCN1 |
| IFI27 | RSAD2 | GPR128 | DUOX2 | ITGA3 | HRASLS 2 | BHLHE4 1 |
| TWSG1 | SERPINB8 | KRT7 | NXF3 | ARL 14 | HPGD | SULT1C3 |
| SSPN | CLIC5 | SEMG1 | EMP1 | F3 | CHST5 | CLCA4 |
| SLC35A3 | CYP3A4 | LIMA1 | PTPRH | REG4 | EVI2B | ANXA10 |
| KRT18P55 | TMEM92 | RHOF | IL33 | HYAL1 | TM4SF4 | CTSE |
| SERPINB5 | CRIP1 | KLK10 | MYOF | MUC17 | FOS | APOL2 |
| BAK1 | APOL1 | APOL6 | KLHDC7 A | GPRC5A | C2orf88 | |
| MAP3K5 | PPP1R36 | SLC17A5 | MLPH | ATP8A1 | HSD17B2 | |
| C12orf36 | FA2H | BCAS1 | SGMS2 | XKR9 | MMP1 | |
| GSDMB | C19orf33 | FBLIM1 | GJB5 | BTNL8 | PTAFR | |
| GJB4 | PLCD3 | TIMP2 | COL17A1 | MMP7 | PI3 | |
| CARD6 | GBP1 | TFF2 | LIPH | RGS2 | CXCL11 | |
| AQP5 | PRDM8 | RARRES3 | KCTD1 | TFF1 | VSIG2 | |
| AGPAT9 | VILL | MSLN | ALOX5 | GPR110 | RAB27B | |
| CEACAM 5 | DDX60L | TLR4 | CDKN2B | PADI2 | C12orf28 | |

**Table 5. Gene signature downregulated in MSS-CRC, serrated lesions, and tubular or tubulovillous adenoma**

| | | | | | | |
|---|---|---|---|---|---|---|
| CTSE | DDB2 | CDKN2B | SYTL1 | TFF1 | KLHDC7A | MARCH3 |
| TCN1 | PLA2G4A | CDKN1A | INPP4B | ALOX5 | DDX60L | RNF144A |
| PDE4D | KLK10 | AKR1A1 | ATL3 | OSBPL1A | TXK | DNAH2 |
| DUSP4 | SDR16C5 | ANXA10 | NTRK2 | ANTXR2 | SREBF1 | PTCHD4 |
| BHLHE41 | CAV2 | KLK11 | CBR4 | RAET1L /// | SHROOM3 | MXD1 |
| AFAP1L2 | C12orf57 | KITLG | TNFSF9 | ANXA3 | PDLIM5 | RAET1G |
| ANXA1 | GPR110 | LDLR | HOXC6 | TP53I3 | SCG5 | KRT19 |
| TRIB2 | TSPAN1 | COL17A1 | RHBDL2 | PPP4R1 | SPRED1 | SGPP2 |
| RAB27B | EPB41L4A | O3FAR1 | IL8 | FAM169A | RBBP8 | EGLN3 |
| REG4 | TLR4 | PELI2 | RASSF6 | CAMK2D | MAP3K6 | CD44 |
| MLPH | HSD17B2 | RNF144B | LMO4 | MB21D2 | ULBP2 | GDA |
| SGMS2 | ZBTB7C | CD109 | LPIN1 | SEMA3B | IL18 | FLRT3 |
| TWSG1 | DAPK1 | IERS | SH3PXD2A | KLF2 | HIST1H1C | PRDM1 |
| TRIM22 | MYOF | KRT7 | EMP1 | TRIM16 | KIAA0040 | OXCT1 |
| CA2 | CAPN8 | LGMN | TLR3 | TTC9 | DST | SH2D4A |
| SPATA18 | PRDM8 | SERPINB5 | SYNJ2BP | CYB5D2 | NXN | C19orf33 |
| F3 | TTC19 | ANXA2 | PLA2G3 | UGT8 | TRIM16-TRIM16L | ARNT2 |
| AGR3 | TET2 | FRMD4B | SGCB | RPL17-C18ORF32 | ZNF488 | RALBP1 |
| ATP8A1 | SBSPON | VSIG2 | EXPH5 | RUNX1 | SULT1C3 | FUT8 |
| PALLD | SMCHD1 | FRAS1 | KIAA1211 | SSPN | LIF | MED11 |
| TRIM7 | PADI2 | IL17RD | C14orf129 | BACE2 | PPP1R9A | PPP2CB |
| SAMD9 | LYZ | TNFAIP8 | PNPLA3 | IGFL2 | KLF4 | NRP1 |
| DOCKS | BLVRA | CFD | PLCG2 | GPR126 | S100A13 | ID2 |
| UBASH3B | MT1E | CCDC68 | TCF7L2 | LCK | FSTL1 | PSD3 |
| FOS | SLCO1B3 | PHLDB2 | AHNAK | USP18 | ARAP2 | FGFR2 |
| KCTD1 | CLCA4 | FHL2 | FSCN1 | C2orf88 | ADORA2B | TRIOBP |
| IL1RN | IMPACT | C11orf9 | NUDT6 | FAM63B | GSN | PPFIBP1 |
| CRIP1 | ABHD3 | RPS27L | TGFA | ARHGAP29 | FAS | CHST5 |
| PLK2 | CD55 | CCNDBP1 | LPAR6 | SERPINB8 | GCNT3 | CHMP1B |
| NAPG | SLC6A14 | TMEM66 | ANXA2 | PLA2G16 | ITGA3 | |
| INO80C | SS18 | HPGD | ZBTB8A | NMU | ULBP2 | |
| PAPSS1 | S100A2 | C12orf28 | PLXNB2 | ANXA2P2 | ULBP2 | |
| SDC3 | RARRES3 | FAM46A | GPRC5A | RAET1L | ABCD4 | |

**Table 6. Gene signature from RNA-seq upregulated in sessile serrated polyps (SSA/P)**

| | | | | |
|---|---|---|---|---|
| MUC5AC | TFF1 | AQP5 | ST3GAL4 | DPCR1 |
| KLK10 | SLC6A14 | PI3 | SDR16C5 | RAB3B |
| TM4SF4 | DUOX2 | CLDN1 | ALDOB | FIBCD1 |
| CTSE | ANXA10 | S100P | HOXB13 | NXF3 |
| VSIG1 | HTR1D | DUSP4 | KRT7 | PDZK1IP1 |
| TFF2 | KLK11 | GJP5 | GJB4 | ZIC5 |
| SERPINB5 | DUOXA2 | SLC6A20 | APOB | CEACAM18 |
| KLK7 | CDH3 | TRIM29 | PSCA | CXCL1 |
| REG4 | VNN1 | PRSS22 | CIDEC | MDF1 |
| MUC17 | SULT1C2 | TACSTD2 | XKR9 | ONECUT2 |

**Table 7. Transcriptomic signature of DKO intestinal epithelial cells**

| | | | | | |
|---|---|---|---|---|---|
| ANXA10 | KCNQ1OT1 | MIRA | PPL | AREG | MVD |
| CLU | PCSK9 | MT1H | KRT7 | ANXA3 | PIR |
| 4930461G14RIK | MYL7 | EMP2 | PMVK | NIPAL2 | IL3RA |
| IL1RN | HLA-A | DDAH1 | C15orf62 | APOBEC2 | LSS |
| ANXA1 | AIM2 | HOXA7 | HOXA3 | CAPN2 | TMSB10 |
| ETV4 | CES1 | BTBD19 | MACC1 | SLAMF9 | C10orf99 |
| NES | CAPG | FDPS | LRP8 | KHDC1 | SLC45A3 |
| MT1E | SYT8 | ANXA5 | ACAT2 | GSTA3 | PLAUR |
| MBOAT1 | PARP3 | LPCAT4 | PAQR8 | SPRY4 | GSTA4 |
| AKR1B10 | TLR4 | LY6A | MIF | CLMP | CD9 |
| SPTSSB | TCF23 | MAPK 11 | PLAT | LY6G6E | TSTD1 |
| ECSCR | HAUS7 | IIGP1 | GBP2 | PRDX6 | ANKRD22 |
| LRFN4 | AK4 | KRT19 | N4BP3 | SYNGR1 | FDFT1 |
| ALDOC | ZNF185 | FA2H | ADAMTS1 | TSPAN1 | TNNI2 |
| RBPMS | GCNT1 | IDI1 | C17orf96 | ANKRD37 | ELAVL2 |
| CNN2 | RSU1 | SH3TC1 | GLIPR1 | SIGMAR1 | GSTA5 |
| R3HDML | S100A16 | NT5DC3 | SLIT2 | FAM 126A | GP1BB |
| ATF5 | HOXA2 | D930048N14RIK | ADAM8 | 2210407C18RIK | BEND5 |
| DPYSL3 | FAM 162A | MAGI2 | BTBD3 | SOLE | IGFBP4 |
| DDIT4 | IGF2BP3 | ACSL4 | MYEF2 | PDX1 | NR4A1 |
| 9230102K24RIK | TRIP6 | ANXA9 | VSTM2L | ARHGAP44 | IMPDH1 |
| UFSP1 | PLK3 | TULP3 | TBC1D9 | NCMAP | NQO1 |
| PRR5-ARHGAP8 | NBEAL2 | FBXW9 | BTC | PIEZO1 | CEACAM4 |
| PPARG | CERS5 | CCDC134 | FAR1 | LDLR | SOCS5 |
| KRT18 | ARNT2 | CTSE | CBR3 | 4930546K05RIK | POP1 |
| AVPI1 | FAM57A | GBP8 | FAM43A | S100A14 | C17orf97 |
| 1110038B12RIK | MTAP | KRT23 | OPN3 | RRP1B | HAGHL |
| UBE2E2 | BNIPL | CD276 | SMTNL1 | RRAS | LAMC1 |
| NHP2 | LAMC2 | PITPNM3 | PIP | ZNF296 | HMGN1 |
| BGLAP | RENBP | SRM | CSF1 | CHML | GM766 |
| CD44 | GUSB | NSDHL | MS4A15 | MAPT | AIF1L |
| TMEM184C | MMP14 | 3930402G23RIK | TNIP3 | MYCN | TMEM237 |
| GSTA5 | LY6D | ALDOA | BAIAP2 | DCBLD2 | MYO15A |
| THBS1 | ABCC4 | SLC2A1 | CLDN2 | CELA1 | ADRB3 |
| CYP51A1 | HOXA5 | PHLDA2 | PHGDH | SLC2A6 | HDDC2 |
| CKB | GRHL3 | PPFIBP1 | TGFBR2 | PQLC3 | CTSH |
| FADS3 | S100A6 | PFKL | OXCT1 | MDFI | ABCC8 |
| PGAP1 | CXCL9 | CA9 | RNF157 | RGCC | SLC37A2 |
| C12orf75 | MVK | MATN2 | GSTA5 | CES2 | PENK |
| FGFR4 | SCD | ACACA | AACS | S100A9 | 4930526I15RIK |
| CSRP1 | LYSMD2 | SLC29A1 | ECM1 | DUSP3 | THEM6 |
| PRSS16 | INSIG1 | CLCN1 | HK1 | MID1IP1 | FHDC1 |
| FAM3D | STARD5 | ETV5 | NEK8 | PMM1 | RBP1 |
| TRIM35 | NKAIN1 | SSSCA1 | FLNA | LONRF1 | SNAI3 |
| SETD4 | SCMH1 | FAM13A | THOP1 | AKR1C13 | TFRC |
| DYNLL1 | CCDC86 | RHBDF1 | WWTR1 | KIAA1841 | GSPT2 |
| DMWD | GSTP1 | F3 | MNS1 | STX1A | CA8 |
| FASN | TNF | STEAP1 | ARHGAP40 | RTN4 | TMED1 |
| ADAT3 | NAP1L1 | NT5C2 | NOC4L | RBMS1 | SGCB |
| PSAT1 | MFGE8 | FAM101B | NOXI | PCOLCE | TMEFF1 |
| SPATA6 | HUNK | SNHG3 | S100B | 1500011B03RIK | LAMA5 |
| ABCC1 | EIF2B3 | CEACAM4 | EMP3 | GM11974 | TRAIP |
| ID4 | FCHO1 | C630043F03RIK | SUSD1 | ZNF593 | SLC20A1 |
| VILL | SNHG5 | PRRG4 | TTR | WDR31 | KIAA1107 |
| PINX1 | HPDL | SH3PXD2B | HEBP2 | SLC1A4 | SUN3 |
| CCNE1 | 1500009C09RIK | PAOX | 2010204K13RIK | MTHFD1L | RBM3 |
| CYP2D6 | S100A8 | METTL22 | PHF21B | PLA2G2E | TNFSF9 |
| MTL5 | BAG2 | CD6 | GRASP | B9D1 | |
| MSTO1 | RARE | SPATA24 | CHCHD6 | SLC29A2 | |
| RPS6KL1 | ILDR1 | RRM2 | ACHE | ARL6 | |

### Survival Analysis

CRC patient samples (GSE5851, GSE33113, TCGA) were segregated into four subgroups: *PRKCI*^{Low}*PRKCZ*^{Low}, *PRKCI*^{Hi}*PRKCZ*^{Hi}, *PRKCI*^{Low}*PRKCZ*^{Hi}, and *PRKCI*^{Hi}*PRKCZ*^{Hi} based on the expression of *PRKCI* and *PRKCZ.* Median expression value of each gene was used as a cut off. *PRKCI*^{Low}*PRKCZ*^{Low} and *PRKCI*^{Hi}*PRKCZ*^{H} groups were compared by GSEA or survival analyses. To generate a Kaplan-Meier curves for TCGA patients, clinical and gene expression data of 369 patients with complete overall survival (OS) information were processed, and 5-year OS rate was compared between *PRKCI*^{Low}*PRKCZ*^{Low} (n = 88) and *PRKCI*^{Hi}*PRKCZ*^{H} i (n = 87) groups. For GSE5851, disease free survival (DFS) rate was compared between *PRKCI*^{Low}*PRKCZ*^{Low} (n = 27) and *PRKCI*^{Hi}*PRKCZ*^{H} (n = 27) groups.

### Statistical analysis

Data are presented as the mean ± SEM. Statistical analysis was performed using GraphPad Prism 7. Significant differences between groups were determined using a Student's t test (two-tailed unpaired) when the data met the normal distribution tested by D'Agostino test. If the data did not meet this test, a Mann-Whitney test was used. Differences in tumor incidence were analyzed by Chi-square test (Figure 4J). Differences in Kaplan-Meier plots were analyzed by Log-Rank (Mantel-Cox) test (Figure 1C, 7F, S7E). Differences in aPKC staining for human samples (Figure 7B) were analyzed by performing ANOVA multiple comparisons (Kruskal-Wallis test). The p-value indicates the difference between signal intensity (strong/moderate vs weak/negative) in normal, SSA/P, and TA. Differences in proportion of CRC patients in CCS1, CCS2, or CCS3 CRC subtype according to aPKC mRNA expression were analyzed by Fisher's exact test for CCS3 vs CCS1/CCS2 **(****FIG. 7E****,** **FIG. 14C, FIG. 14D****).** The significance level for statistical testing was set at p < 0.05.

### Example 10. Characterizing the Effects of PVHA Hyaluronidase in a Novel Mouse Model of Colorectal Cancer

Epithelial tumor cells in the serrated (DKO^{IEC}) mouse model display increased levels of CD44 and the stroma, that is highly activated, expressed hyaluronan (HA). HA is a ligand for CD44, which is a stem cell receptor. HA contributes to the barrier role of the stroma that makes immunotherapy ineffective for some patients. An experiment is performed to understand the effects of an inhibitor of HA in the DKO^{IEC} mouse model to determine if tumorigenesis is inhibited without further treatment or upon co-treatment with an anti-PD-L1 therapy.

*Prkcz*^{fl/fl}, *Prkci*^{fl/fl}, *Apc*^{fl/fl}, Villin-cre and Lgr5-EGFP-ires-creERT2 (Lgr5-creERT2) mice are acquired. Prkci^{fl/fl} and Prkcz^{fl/fl} mice are crossed with Villin-cre mice to generate a mouse line with deletions in both aPKCs (DKO^{IEC}) in IECs. Genotypes of DKO^{IEC} mice are determined. Exemplary methods for determining a genotype include polymerase chain reaction (PCR).

DKO^{IEC} mice are treated twice per week beginning at 16 weeks of age with pegvorhyaluronidase alfa (PVHA) and/or anti-PD-L1 treatment (e.g., anti-PD-L1 monoclonal antibody) for 4 weeks. Control DKO^{IEC} mice are treated with a vehicle twice per week beginning at 16 weeks of age for 4 weeks. When co-treatment PVHA and anti-PD-L1 treatment is administered, the PVHA is administered to DKO^{IEC} mice 24 hours prior to administration of the anti-PD-L1 treatment. Table 8 shows exemplary treatment regimens for 6 groups of DKO^{IEC} mice.

**Table 8. DKO^{IEC} mice Exemplary Treatment Regimens**

| **Group** | **# Animals** | **Test Article 1 (mg/kg)** | **Test Article 2 (mg/kg)** |
|---|---|---|---|
| 1 | 10 | Vehicle | Vehicle |
| 2 | 10 | PVHA (0.0375) | Vehicle |
| 3 | 10 | PVHA (1.0) | Vehicle |
| 4 | 10 | Vehicle | Anti PD-L1 (10.0) |
| 5 | 10 | PVHA (0.0375) | Anti PD-L1 (10.0) |
| 6 | 10 | PVHA (1.0) | Anti PD-L1 (10.0) |

DKO^{IEC} mice are euthanized to evaluate at various antitumor activity endpoints at a period of time after the final treatment. Suitable periods of time include, but are not limited to, 1 hour, 2 hours, 3 hours, 4 hours, 5 hours, 10 hours, 15 hours, 20 hours, 1 day, 1.5 days, 2 days, or 3 days, after final treatment. Measures of antitumor activity endpoints are analyzed, including tumor scoring (e.g., number, size, pathological characterization, incidence of invasion), H&E, HA staining, CD44 staining, CD8 staining. Examination of microsatellite instability (MSI) by detecting a presence or absence of five microsatellite repeat markers is performed. Suitable assays for detecting the presence or absence of the five microsatellite repeat markers includes PCR. Plasma samples are obtained from the DKO^{IEC} mice and evaluated for a presence or absence of HA/biomarkers.

Throughout this disclosure, various embodiments are presented in a range format. It should be understood that the description in range format is merely for convenience and brevity and should not be construed as an inflexible limitation on the scope of any embodiments. Accordingly, the description of a range should be considered to have specifically disclosed all the possible subranges as well of any dividual numerical values within that range to the tenth of the unit of the lower limit unless the context clearly dictates otherwise. For example, description of a range such as from 1 to 6 should be considered to have specifically disclosed subranges such as from 1 to 3, from 1 to 4, from 1 to 5, from 2 to 4, from 2 to 6, from 3 to 6 etc., as well of any dividual values within that range, for example, 1.1,2, 2.3, 5, and 5.9. This applies regardless of the breadth of the range. The upper and lower limits of these intervening ranges may independently be included in the smaller ranges, to any specifically excluded limit in the stated range. Where the stated range includes one or both of the limits, ranges excluding either or both of those included limits are also included, unless the context clearly dictates otherwise.

The terminology used herein is for the purpose of describing particular embodiments only and is not intended to be limiting. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

While preferred embodiments of the present invention have been shown and described herein, it will be apparent to those skilled in the art that such embodiments are provided by way of example only. Numerous variations, changes, and substitutions will now occur to those skilled in the art.

## Claims

1. An inhibitor of programmed death-ligand 1 (PD-L1) activity or expression for use in a method for treating a subtype of a disease or condition in a subject, the method comprising:
determining a decreased expression level of PKCζ and PKC 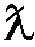 / 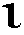 in a biological sample obtained from the subject compared to a level of expression of PKCζ and PKC 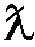 / 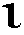 in an individual who does not have the disease or condition; and
administering to the subject a therapeutically effective amount of the programmed death-ligand 1 (PD-L1) inhibitor, wherein the subtype of the disease or condition comprises a disease or condition **characterized by** serrated polyps in an intestine or by serrated adenocarcinoma.

2. The PD-L1 inhibitor for use of claim 1, wherein the method further comprises:
determining an increased expression level of hyaluronan in the biological sample compared to an expression level of hyaluronan in an individual who does not have the disease or condition; or
administering to the subject a therapeutically effective amount of an inhibitor of hyaluronan activity or expression.

3. The PD-L1 inhibitor for use of claim 2, wherein the expression level of PKCζ, PKC 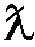 / 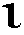 , or hyaluronan is detected by an assay comprising polymerase chain reaction (PCR), reverse transcription PCR (RT-PCR), deoxyribonucleic acid (DNA) sequencing, ribonucleic acid (RNA) sequencing, genotyping array, immunohistochemistry, enzyme-linked immunosorbent assay (ELISA), single-molecule array (Simoa), or a combination thereof.

4. The PD-L1 inhibitor for use of claim 1, wherein the disease or condition comprises colorectal cancer, pancreatic cancer, inflammatory bowel disease, ulcerative colitis, Crohn's disease, fibrosis, fibrostenosis, or a combination thereof.

5. The PD-L1 inhibitor for use of claim 1, wherein the method further comprises determining whether the subject has or will develop a subtype of the disease or condition, wherein the subtype of the disease or condition comprises a disease or condition **characterized by** an increase in hyaluronan in the biological sample obtained from the subject, as compared to the individual who does not have the subtype of the disease or condition; wherein the subtype of the disease or condition comprises a disease or condition **characterized by** serrated colorectal cancer (CLC).

6. The PD-L1 inhibitor for use of claim 1, wherein the subtype of the disease or condition comprises a disease or condition **characterized by** serrated polyps in the intestine of the subj ect.

7. The PD-L1 inhibitor for use of claim 6, wherein the serrated polyps comprise sessile serrated adenomas (SSAs).

8. The PD-L1 inhibitor for use method of claim 1, wherein the subtype of the disease or condition comprises a disease or condition **characterized by** serrated adenocarcinoma.

9. The PD-L1 inhibitor for use of claim 1, wherein the PD-L1 inhibitor is selected from the group consisting of an antagonist of PD-L1, an antagonist of programmed cell death protein 1 (PD-1), an anti-PD-L1 antibody, an anti-PD-1 antibody, and any combination thereof.

10. The PD-L1 inhibitor for use of claim 2, wherein the hyaluronan inhibitor is selected from the group consisting of an antagonist of CD44, an antibody inhibitor of CD44, a small molecule inhibitor of CD44, an agonist of hyaluronidase, pegvorhyaluronidase alfa (PVHA), an exogenous hyaluronidase, a recombinant hyaluronidase.

11. The PD-L1 inhibitor for use of claim 1, the method further comprising administering to the subject having the disease or condition a therapeutically effective amount of an inhibitor of transforming growth factor beta 1 (TGF-β1) activity or expression.

12. The PD-L1 inhibitor for use of claim 11, wherein the inhibitor of TGF- β1 activity or expression comprises an antibody, antigen-binding fragment, a small molecule, or a small molecule inhibitor of a receptor of TGF- β1.

13. The PD-L1 inhibitor for use of claim 11, wherein the inhibitor of TGF- β1 activity or expression comprises galunisertib.

14. The PD-L1 inhibitor for use of claim 1, wherein the biological sample comprises blood, blood plasma, sera, or tissue biopsy.

15. The PD-L1 inhibitor for use of claim 1, wherein the subject is a human.

## Patentansprüche

1. Inhibitor der Aktivität oder Expression eines Programmed Death Ligand 1 (PD-L1) zur Verwendung in einem Verfahren zur Behandlung eines Subtyps einer Erkrankung oder eines Leidens bei einem Individuum, wobei das Verfahren Folgendes umfasst:
Bestimmen eines verringerten Expressionsniveaus von PKCζ und PKC 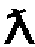 / 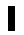 in einer biologischen Probe, die von dem Individuum erhalten wurde, im Vergleich zum Expressionsniveau von PKCζ und PKC 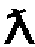 / 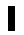 bei einem Individuum, das die Erkrankung oder das Leiden nicht hat; und
Verabreichen einer therapeutisch wirksamen Menge des Inhibitors des Programmed Death Ligand 1 (PD-L1) an das Individuum, wobei der Subtyp der Erkrankung oder des Leidens eine Erkrankung oder ein Leiden umfasst, die/das durch gezackte Polypen im Darm oder durch gezackte Adenokarzinome gekennzeichnet ist.

2. PD-L1-Inhibitor zur Verwendung nach Anspruch 1, wobei das Verfahren weiters Folgendes umfasst:
Bestimmen eines erhöhten Expressionsniveaus von Hyaluronan in der biologischen Probe im Vergleich zum Expressionsniveau von Hyaluronan bei einem Individuum, das die Erkrankung oder das Leiden nicht hat; oder
Verabreichen einer therapeutisch wirksamen Menge eines Inhibitors von Hyaluronan-Aktivität oder-Expression an das Individuum.

3. PD-L1-Inhibitor zur Verwendung nach Anspruch 2, wobei das Expressionsniveau von PKCζ, PKC 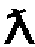 / 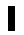 oder Hyaluronan durch einen Test detektiert wird, der eine Polymerasekettenreaktion (PCR), eine Reverse-Transkriptase-PCR (RT-PCR), Desoxyribonucleinsäure-(DNA-) Sequenzierung, Ribonucleinsäure- (RNA-) Sequenzierung, Genotypisierungsarray, Immunhistochemie, enzymgekoppelten Immunabsorptionstest (ELISA), Einzelmolekül-Array (Simoa) oder eine Kombination davon umfasst.

4. PD-L1-Inhibitor zur Verwendung nach Anspruch 1, wobei die Erkrankung oder das Leiden Kolorektalkrebs, Pankreaskrebs, entzündliche Darmerkrankung, Colitis ulcerosa, Morbus Crohn, Fibrose, Fibrostenose oder eine Kombination umfasst.

5. PD-L1-Inhibitor zur Verwendung nach Anspruch 1, wobei das Verfahren weiters das Bestimmen umfasst, ob das Individuum einen Subtyp der Erkrankung oder des Leidens hat oder entwickeln wird, wobei der Subtyp der Erkrankung oder des Leidens eine Erkrankung oder ein Leiden umfasst, die/das durch eine Erhöhung von Hyaluronan in der biologischen Probe vom Individuum im Vergleich zu einem Individuum, das den Subtyp der Erkrankung oder des Leidens nicht hat, gekennzeichnet ist; wobei der Subtyp der Erkrankung oder des Leidens eine Erkrankung oder ein Leiden umfasst, die/das durch gezackten Kolorektalkrebs (CLC) gekennzeichnet ist.

6. PD-L1-Inhibitor zur Verwendung nach Anspruch 1, wobei der Subtyp der Erkrankung oder des Leidens eine Erkrankung oder ein Leiden umfasst, die/das durch gezackte Polypen im Darm des Individuums gekennzeichnet ist.

7. PD-L1-Inhibitor zur Verwendung nach Anspruch 6, wobei die gezackten Polypen sessile gezackte Adenome (SSAs) umfassen.

8. PD-L1-Inhibitor zur Verwendung nach Anspruch 1, wobei der Subtyp der Erkrankung oder des Leidens eine Erkrankung oder ein Leiden umfasst, die/das durch gezackte Adenokarzinome gekennzeichnet ist.

9. PD-L1-Inhibitor zur Verwendung nach Anspruch 1, wobei der PD-L1-Inhibitor aus der aus einem Antagonisten von PD-L1, einem Antagonisten des Programmed Cell Death Protein 1 (PD-1), einem Anti-PD-L1-Antikörper, einem Anti-PD-1-Antikörper und einer beliebigen Kombination davon bestehenden Gruppe ausgewählt ist.

10. PD-L1-Inhibitor zur Verwendung nach Anspruch 2, wobei der Hyaluronan-Inhibitor aus der aus einem Antagonisten von CD44, einem Antikörper-Inhibitor von CD44, einem kleinmolekularen Inhibitor von CD44, einem Agonisten von Hyaluronidase, Pegvorhyaluronidase Alpha (PVHA), einer exogenen Hyaluronidase und einer rekombinanten Hyaluronidase bestehenden Gruppe ausgewählt ist.

11. PD-L1-Inhibitor zur Verwendung nach Anspruch 1, wobei das Verfahren weiters das Verabreichen einer therapeutisch wirksamen Menge eines Inhibitors der Aktivität oder Expression des Transforming Growth Factor Beta 1 (TGF-β1) an das Individuum mit der Erkrankung oder dem Leiden umfasst.

12. PD-L1-Inhibitor zur Verwendung nach Anspruch 11, wobei der Inhibitor der TGF-β1-Aktivität oder -Expression einen Antikörper, ein antigenbindendes Fragment, ein kleines Molekül oder einen kleinmolekularen Inhibitor eines Rezeptors von TGF-β1 umfasst.

13. PD-L1-Inhibitor zur Verwendung nach Anspruch 11, wobei der Inhibitor der TGF-β1-Aktivität oder -Expression Galunisertib umfasst.

14. PD-L1-Inhibitor zur Verwendung nach Anspruch 1, wobei die biologische Probe Blut, Blutplasma, Serum oder eine Gewebebiopsie umfasst.

15. PD-L1-Inhibitor zur Verwendung nach Anspruch 1, wobei das Individuum ein Mensch ist.

## Revendications

1. Inhibiteur de l'activité ou de l'expression du ligand de mort programmée 1 (PD-L1) à utiliser dans un procédé de traitement d'un sous-type d'une maladie ou d'un état chez un sujet, le procédé comprenant les étapes consistant à :
déterminer un niveau d'expression réduit de PKCζ et de PKC 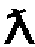 / 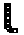 dans un échantillon biologique obtenu auprès du sujet par rapport à un niveau d'expression de PKCζ et de PKC 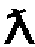 / 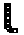 chez un individu qui n'est pas atteint de la maladie ou de l'affection ; et
administrer au sujet une quantité thérapeutiquement efficace de l'inhibiteur de ligand de mort programmée 1 (PD-L1), dans lequel le sous-type de la maladie ou de l'état comprend une maladie ou un état **caractérisé par** des polypes dentelés dans un intestin ou par un adénocarcinome dentelé.

2. Inhibiteur de PD-L1 destiné à être utilisé selon la revendication 1, dans lequel le procédé comprend en outre les étapes consistant à :
déterminer un niveau d'expression accru de hyaluronane dans l'échantillon biologique par rapport à un niveau d'expression de hyaluronane chez un individu qui n'est pas atteint de la maladie ou de l'affection ; ou
administrer au sujet une quantité thérapeutiquement efficace d'un inhibiteur de l'activité ou de l'expression de hyaluronane.

3. Inhibiteur de PD-L1 destiné à être utilisé selon la revendication 2, dans lequel le niveau d'expression de PKCζ, PKC 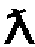 / 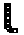 ou hyaluronane est détecté par un dosage comprenant une réaction en chaîne par polymérase (PCR), une PCR de transcription inverse (RT-PCR), un séquençage d'acide désoxyribonucléique (ADN), un séquençage d'acide ribonucléique (ARN), un réseau de génotypage, une immunohistochimie, un dosage immuno-absorbant lié à une enzyme (ELISA), un réseau de molécules uniques (Simoa) ou une combinaison de ceux-ci.

4. Inhibiteur de PD-L1 à utiliser selon la revendication 1, dans lequel la maladie ou l'état comprend le cancer colorectal, le cancer du pancréas, la maladie intestinale inflammatoire, la rectocolite hémorragique, la maladie de Crohn, la fibrose, la fibrosténose, ou une combinaison de ceux-ci.

5. Inhibiteur de PD-L1 à utiliser selon la revendication 1, dans lequel le procédé comprend en outre l'étape consistant à déterminer si le sujet a ou développera un sous-type de la maladie ou de l'état, dans lequel le sous-type de la maladie ou de l'état comprend une maladie ou un état caractérisé(e) par une augmentation de hyaluronane dans l'échantillon biologique obtenu auprès du sujet, par rapport à l'individu qui n'a pas le sous-type de la maladie ou de l'état ; dans lequel le sous-type de la maladie ou de l'état comprend une maladie ou un état caractérisé(e) par un cancer colorectal dentelé (CLC).

6. Inhibiteur de PD-L1 à utiliser selon la revendication 1, dans lequel le sous-type de la maladie ou de l'état comprend une maladie ou un état caractérisé(e) par des polypes dentelés dans l'intestin du sujet.

7. Inhibiteur de PD-L1 à utiliser selon la revendication 6, dans lequel les polypes dentelés comprennent des adénomes dentelés sessiles (ASS).

8. Procédé d'utilisation d'un inhibiteur de PD-L1 selon la revendication 1, dans lequel le sous-type de la maladie ou de l'état comprend une maladie ou un état caractérisé(e) par un adénocarcinome dentelé.

9. Inhibiteur de PD-L1 à utiliser selon la revendication 1, dans lequel l'inhibiteur de PD-I1 est choisi dans le groupe constitué d'un antagoniste de PD-L1, d'un antagoniste de la protéine de mort cellulaire programmée 1 (PD-1), d'un anticorps anti-PD-L1, d'un anticorps anti-PD-1 et de toute combinaison de ceux-ci.

10. Inhibiteur de PD-L1 à utiliser selon la revendication 2, dans lequel l'inhibiteur de hyaluronane est choisi dans le groupe constitué d'un antagoniste de CD44, d'un inhibiteur d'anticorps de CD44, d'un inhibiteur de petites molécules de CD44, d'un agoniste de hyaluronidase, de pegvorhyaluronidase alfa (PVHA), d'une hyaluronidase exogène, d'une hyaluronidase recombinante.

11. Inhibiteur de PD-L1 à utiliser selon la revendication 1, le procédé comprenant en outre l'administration au sujet présentant la maladie ou l'état d'une quantité thérapeutiquement efficace d'un inhibiteur de l'activité ou de l'expression du facteur de croissance transformant bêta 1 (TGF-β1).

12. Inhibiteur de PD-L1 à utiliser selon la revendication 11, dans lequel l'inhibiteur de l'activité ou de l'expression de TGF-β1 comprend un anticorps, un fragment de liaison à un antigène, une petite molécule ou un inhibiteur de petites molécules d'un récepteur de TGF-β1.

13. Inhibiteur de PD-L1 à utiliser selon la revendication 11, dans lequel l'inhibiteur de l'activité ou de l'expression du TGF-β1 comprend le galunisertib.

14. Inhibiteur de PD-L1 à utiliser selon la revendication 1, dans lequel l'échantillon biologique comprend du sang, du plasma sanguin, des sérums ou une biopsie de tissu.

15. Inhibiteur de PD-L1 destiné à être utilisé selon la revendication 1, dans lequel le sujet est un humain.
